# EUROPEAN PATENT SPECIFICATION

(11) **EP 3 911 413 B1**
(45) Date of publication and mention of the grant of the patent: **06.12.2023**
(21) Application number: 20701700.5
(22) Date of filing: 16.01.2020
(51) Int. Cl.: A61P 31/12, C07D 311/30, A61K 31/352

(54) **SUBSTITUTED CHROMEN-4-ONE FOR THE TREATMENT AND PROPHYLAXIS OF HEPATITIS B VIRUS INFECTION**
SUBSTITUIERTES CHROMEN-4-ON ZUR BEHANDLUNG UND PROPHYLAXE VON HEPATITIS-B-VIRUS-INFEKTIONEN
CHROMÈNE-4-ONE SUBSTITUÉE POUR LE TRAITEMENT ET LA PROPHYLAXIE D'UNE INFECTION PAR LE VIRUS DE L'HÉPATITE B

(30) Priority: 17.01.2019 WO PCT/CN2019/072117
(43) Date of publication of application: 24.11.2021
(73) Proprietor: F. Hoffmann-La Roche AG, 4070 Basel (CH)
(72) Inventor: CHEN, Dongdong, Shanghai, 201203 (CN); FENG, Song, Shanghai, 201203 (CN); LIANG, Chungen, Shanghai, 201203 (CN); MIAO, Kun, Shanghai, 201203 (CN); TAN, Xuefei, Shanghai, 201203 (CN); SHEN, Hong, Shanghai, 201203 (CN)
(74) Representative: Jochnowitz, Evan
(86) International application number: PCT/EP2020/050953
(87) International publication number: WO 2020/148354

(56) References cited:
- TIAN Y ET AL: "Anti-HBV active flavone glucosides from Euphorbia humifusa Willd", FITOTERAPIA, IDB HOLDING, MILAN, IT, vol. 81, no. 7, 1 October 2010 (2010-10-01), pages 799-802, XP027275823, ISSN: 0367-326X [retrieved on 2010-09-09]
- ZHANG FAN ET AL: "A review of non-nucleoside anti-hepatitis B virus agents", EUROPEAN JOURNAL OF MEDICINAL CHEMISTRY, ELSEVIER, AMSTERDAM, NL, vol. 75, 30 January 2014 (2014-01-30), pages 267-281, XP028625376, ISSN: 0223-5234, DOI: 10.1016/J.EJMECH.2014.01.046

## Description

The present invention relates to organic compounds useful for therapy and/or prophylaxis of HBV infection in a mammal, and in particular to cccDNA (covalently closed circular DNA) inhibitors useful for treating HBV infection.

### FIELD OF THE INVENTION

The present invention relates to substituted chromen-4-one having pharmaceutical activity, their manufacture, pharmaceutical compositions containing them and their potential use as medicaments.

The present invention relates to compounds of formula (I) 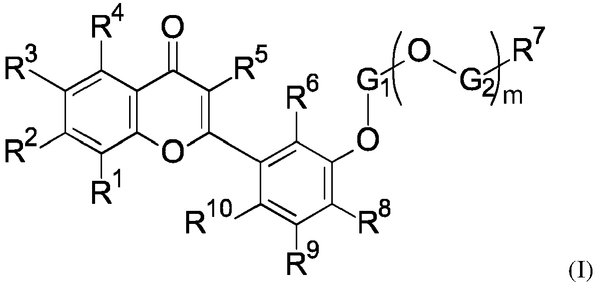 wherein R¹ to R¹⁰, G₁, G₂ and m are as described below, or a pharmaceutically acceptable salt thereof.

Hepatitis B virus (HBV) infection is one of the most prevalent viral infections and is a leading cause of chronic hepatitis. It is estimated that worldwide, around 2 billion people have evidence of past or present infection with HBV. Over 250 million individuals are currently chronically infected with HBV and are therefore at high risk to develop liver fibrosis, cirrhosis and hepatocellular carcinoma (HCC). There are data to indicate ~800,000 deaths per year are directly linked to HBV infection (Lozano, R. et al., Lancet (2012), 380 (9859), 2095-2128; Goldstein, S.T. et al., Int J Epidemiol (2005), 34 (6), 1329-1339).

Many countries in the world administer hepatitis B vaccine starting at birth or in early childhood, which has greatly reduced the incidence and prevalence of hepatitis B in most endemic regions over the past few decades. However, the vaccine has no impact on people who were infected before the widely use of the vaccine in developing end-stage liver disease or HCC (Chen, D.S., J Hepatol (2009), 50 (4), 805-816). Vaccination at birth of infants born to HBV positive mothers is usually not sufficient for protecting vertical transmission and combination with hepatitis B immune globulin is needed (Li, X.M. et al., World J Gastroenterol (2003), 9 (7), 1501-1503).

Currently FDA-approved treatments for chronic hepatitis B include two type 1 interferons (IFN) which are IFNalfa-2b and pegylated IFN alfa-2a and six nucleos(t)ide analogues (NAs) which are lamivudine (3TC), tenofovir disoproxil fumarate (TDF), adefovir (ADV), telbivudine (LdT), entecavir (ETV), and vemlidy (tenofovir alafenamide (TAF)). IFN treatment is finite, but it is known to have severe side effects, and only a small percentage of patients showed a sustained virological response, measured as loss of hepatitis B surface antigen (HBsAg). NAs are inhibitors of the HBV reverse transcriptase, profoundly reduce the viral load in vast majority of treated patients, and lead to improvement of liver function and reduced incidence of liver failure and hepatocellular carcinoma. However, the treatment of NAs is infinite (Ahmed, M. et al., Drug Discov Today (2015), 20 (5), 548-561; Zoulim, F. and Locarnini, S., Gastroenterology (2009), 137 (5), 1593-1608 e1591-1592).

HBV chronic infection is caused by persistence of covalently closed circular (ccc)DNA, which exists as an episomal form in hepatocyte nuclei. cccDNA serves as the template for viral RNA transcription and subsequent viral DNA generation. Only a few copies of cccDNA per liver cell can establish or re-initiate viral replication. Therefore, a complete cure of chronic hepatitis B will require elimination of cccDNA or permanently silencing of cccDNA. However, cccDNA is intrinsically very stable and currently available therapeutics could not eliminate cccDNA or permanently silence cccDNA (Nassal, M., Gut (2015), 64 (12), 1972-1984; Gish, R.G. et al., Antiviral Res (2015), 121, 47-58; Levrero, M. et al., J Hepatol (2009), 51 (3), 581-592.). The current SoC could not eliminate the cccDNA which are already present in the infected cells. There is an urgent need to discover and develop new anti-HBV reagents to eliminate or permanently silence cccDNA, the source of chronicity (Ahmed, M. et al., Drug Discov Today (2015), 20 (5), 548-561; Nassal, M., Gut (2015), 64 (12), 1972-1984)

Tyan et al.; Fitoterapia 2010, vol. 81, no 7, pages 799-802 and Zhang et al.; European Journal of Medicinal Chemistry 2014, vol. 75, pages 267-281 disclose flavone derivatives useful for the treatment of hepatitis B.

### SUMMARY OF THE INVENTION

Objects of the present invention are compounds of formula (I), their manufacture, medicaments based on a compound in accordance with the invention and their production as well as the compounds of formula (I) as cccDNA inhibitors and for the treatment of prophylaxis of HBV infection. The compounds of formula (I) show superior anti-HBV activity. In addition, the compounds of formula (I) also show good PK profiles.

The present invention relates to a compound of formula (I) 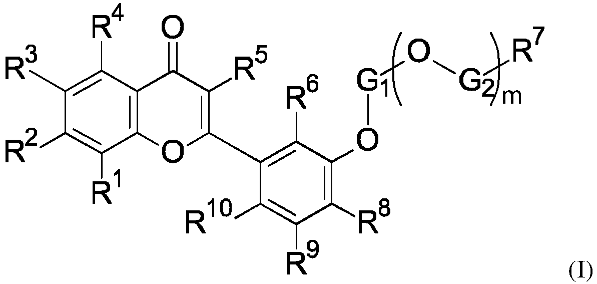 wherein
- R¹: is halogen;
- R²: is selected from H, OH, halogen, C₁₋₆alkyl, haloC₁₋₆alkyl and C₁₋₆alkoxy;
- R³: is selected from H, OH, halogen, C₁₋₆alkyl, haloC₁₋₆alkyl and C₁₋₆alkoxy;
- R⁴: is selected from H, OH, halogen, C₁₋₆alkyl, haloC₁₋₆alkyl and C₁₋₆alkoxy;
- R⁵: is selected from H, OH, halogen, C₁₋₆alkyl, haloC₁₋₆alkyl and C₁₋₆alkoxy;
- R⁶: is selected from H, OH, halogen, C₁₋₆alkyl, haloC₁₋₆alkyl and C₁₋₆alkoxy;
- R⁷: is selected from carboxy, C₁₋₆alkoxycarbonyl, carboxycarbonylamino, C₃₋₇cycloalkylsulfonylaminocarbonyl and C₁₋₆alkoxycarbonylcarbonylamino;
- R⁸: is selected from H, OH, halogen, C₁₋₆alkyl, haloC₁₋₆alkyl and C₁₋₆alkoxy;
- R⁹: is selected from H, OH, halogen, C₁₋₆alkyl, haloC₁₋₆alkyl and C₁₋₆alkoxy;
- R¹⁰: is selected from H, OH, halogen, C₁₋₆alkyl, haloC₁₋₆alkyl, C₁₋₆alkoxy and haloC₁₋₆alkoxy;
- G₁: is selected from C₁₋₆alkyl and C₃₋₇cycloalkyl;
- G₂: is selected from C₁₋₆alkyl and C₃₋₇cycloalkyl;
- m: is selected from 0 and 1;
or a pharmaceutically acceptable salt thereof.

### DETAILED DESCRIPTION OF THE INVENTION

### DEFINITIONS

As used herein, the term "C₁₋₆alkyl" alone or in combination signifies a saturated, linear- or branched chain alkyl group containing 1 to 6, particularly 1 to 4 carbon atoms, for example methyl, ethyl, propyl, isopropyl, butyl, isobutyl, *tert*-butyl and the like. Particular "C₁₋₆alkyl" groups are methyl, ethyl, propyl, isopropyl, isobutyl and *tert-*butyl*.* Most particular "C₁₋₆alkyl" group is methyl.

The term "C₁₋₆alkoxy" alone or in combination signifies a group C₁₋₆alkyl-O-, wherein the "C₁₋₆alkyl" is as defined above; for example, methoxy, ethoxy, propoxy, *iso*-propoxy, *n*-butoxy, *iso*-butoxy, 2-butoxy, *tert*-butoxy, pentoxy, hexyloxy and the like. Particular "C₁₋₆alkoxy" groups are methoxy and ethoxy.

The term "C₃₋₇cycloalkyl" denotes to a saturated carbon ring containing from 3 to 7 carbon atoms, particularly from 3 to 6 carbon atoms, for example, cyclopropyl, cyclobutyl, cyclopentyl, cyclohexyl, cycloheptyl and the like. Particular "C₃₋₇cycloalkyl" groups are cyclopropyl and cyclobutyl.

The term "halogen" and "halo" are used interchangeably herein and denote fluoro, chloro, bromo, or iodo.

The term "haloC₁₋₆alkyl" denotes an alkyl group wherein at least one of the hydrogen atoms of the alkyl group is replaced by same or different halogen atoms, particularly fluoro atoms. Examples of haloC₁₋₆alkyl include monochloro-, difluoro-or trifluoro-methyl, -ethyl or - propyl, for example difluoromethyl and trifluoromethyl.

The term "haloC₁₋₆alkoxy" denotes a C₁₋₆alkoxy group wherein at least one of the hydrogen atoms of the C₁₋₆alkoxy group is replaced by same or different halogen atoms, particularly fluoro atoms. Examples of haloC₁₋₆alkoxy include mono fluoro-, difluoro- or trifluoro-methoxy, -ethoxy or -propoxy, for example difluoromethoxy and trifluoromethoxy.

The term "carbonyl" alone or in combination refers to the group -C(O)-.

The term "sulfonyl" alone or in combination refers to the group -S(O)₂-.

The compounds according to the present invention may exist in the form of their pharmaceutically acceptable salts. The term "pharmaceutically acceptable salt" refers to conventional acid-addition salts or base-addition salts that retain the biological effectiveness and properties of the compounds of formula (I) and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flowability and solubility of compounds. It is for example described in Bastin R.J., et al., Organic Process Research & Development 2000, 4, 427-435. Particular are the sodium salts of the compounds of formula (I).

Compounds of the general formula (I) which contain one or several chiral centers can either be present as racemates, diastereomeric mixtures, or optically active single isomers. The racemates can be separated according to known methods into the enantiomers. Particularly, diastereomeric salts which can be separated by crystallization are formed from the racemic mixtures by reaction with an optically active acid such as e.g. D- or L-tartaric acid, mandelic acid, malic acid, lactic acid or camphorsulfonic acid.

### HBV INHIBITORS

The present invention provides (i) a compound having the general formula (I): 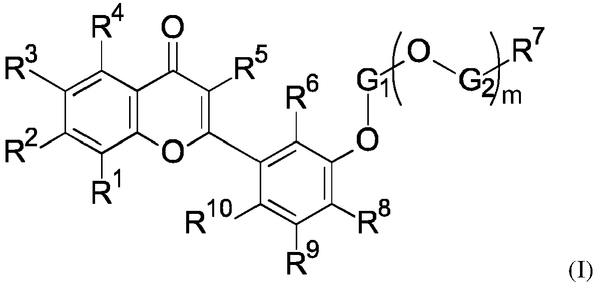 wherein
- R¹: is halogen;
- R²: is selected from H, OH, halogen, C₁₋₆alkyl, haloC₁₋₆alkyl and C₁₋₆alkoxy;
- R³: is selected from H, OH, halogen, C₁₋₆alkyl, haloC₁₋₆alkyl and C₁₋₆alkoxy;
- R⁴: is selected from H, OH, halogen, C₁₋₆alkyl, haloC₁₋₆alkyl and C₁₋₆alkoxy;
- R⁵: is selected from H, OH, halogen, C₁₋₆alkyl, haloC₁₋₆alkyl and C₁₋₆alkoxy;
- R⁶: is selected from H, OH, halogen, C₁₋₆alkyl, haloC₁₋₆alkyl and C₁₋₆alkoxy;
- R⁷: is selected from carboxy, C₁₋₆alkoxycarbonyl, carboxycarbonylamino, C₃₋₇cycloalkylsulfonylaminocarbonyl and C₁₋₆alkoxycarbonylcarbonylamino;
- R⁸: is selected from H, OH, halogen, C₁₋₆alkyl, haloC₁₋₆alkyl and C₁₋₆alkoxy;
- R⁹: is selected from H, OH, halogen, C₁₋₆alkyl, haloC₁₋₆alkyl and C₁₋₆alkoxy;
- R¹⁰: is selected from H, OH, halogen, C₁₋₆alkyl, haloC₁₋₆alkyl, C₁₋₆alkoxy and haloC₁₋₆alkoxy;
- G₁: is selected from C₁₋₆alkyl and C₃₋₇cycloalkyl;
- G₂: is selected from C₁₋₆alkyl and C₃₋₇cycloalkyl;
- m: is selected from 0 and 1;
or a pharmaceutically acceptable salt thereof.

A further embodiment of the present invention is (ii) a compound of formula (I) according to (i), wherein
- R¹: is halogen;
- R²: is H;
- R³: is H;
- R⁴: is H;
- R⁵: is H;
- R⁶: is selected from H and C₁₋₆alkoxy;
- R⁷: is selected from carboxy, carboxycarbonylamino and C₃₋₇cycloalkylsulfonylaminocarbonyl;
- R⁸: is selected from H, halogen, C₁₋₆alkyl, haloC₁₋₆alkyl and C₁₋₆alkoxy;
- R⁹: is selected from H and C₁₋₆alkoxy;
- R¹⁰: is selected from H, halogen, C₁₋₆alkoxy and haloC₁₋₆alkoxy;
- G₁: is selected from C₁₋₆alkyl and C₃₋₇cycloalkyl;
- G₂: is selected from C₁₋₆alkyl and C₃₋₇cycloalkyl;
- m: is selected from 0 and 1;
or a pharmaceutically acceptable salt thereof.

A further embodiment of the present invention is (iii) a compound of formula (I) according to (i), wherein
- R¹: is Cl;
- R²: is H;
- R³: is H;
- R⁴: is H;
- R⁵: is H;
- R⁶: is selected from H and methoxy;
- R⁷: is selected from carboxy, carboxycarbonylamino and cyclopropylsulfonylaminocarbonyl;
- R⁸: is selected from H, Cl, Br, methyl, CF₃ and methoxy;
- R⁹: is selected from H and methoxy;
- R¹⁰: is selected from H, F, Cl, Br, methoxy, ethoxy, difluoromethoxy and trifluoromethoxy;
- G₁: is selected from methyl, ethyl, propyl and cyclobutyl;
- G₂: is selected from methyl and cyclobutyl;
- m: is selected from 0 and 1;
or a pharmaceutically acceptable salt thereof.

A further embodiment of the present invention is (iv) a compound of formula (I) according to (i), or a pharmaceutically acceptable salt thereof, wherein R⁷ is selected from carboxy and C₃₋₇cycloalkylsulfonylaminocarbonyl.

A further embodiment of the present invention is (v) a compound of formula (I) according to (i), or a pharmaceutically acceptable salt thereof, wherein R⁷ is selected from carboxy and cyclopropylsulfonylaminocarbonyl.

A further embodiment of the present invention is (vi) a compound of formula (I) according to (i), or a pharmaceutically acceptable salt thereof, wherein R⁹ is H.

A further embodiment of the present invention is (vii) a compound of formula (I) according to (i), or a pharmaceutically acceptable salt thereof, wherein R¹⁰ is selected from H, halogen and C₁₋₆alkoxy.

A further embodiment of the present invention is (viii) a compound of formula (I) according to (i), or a pharmaceutically acceptable salt thereof, wherein R¹⁰ is selected from H, F, methoxy and ethoxy.

A further embodiment of the present invention is (ix) a compound of formula (II) according to (i), or a pharmaceutically acceptable salt thereof, 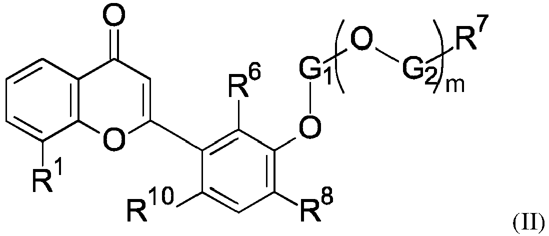 wherein
- R¹: is halogen;
- R⁶: is selected from H and C₁₋₆alkoxy;
- R⁷: is selected from carboxy and C₃₋₇cycloalkylsulfonylaminocarbonyl;
- R⁸: is selected from H, halogen, C₁₋₆alkyl, haloC₁₋₆alkyl and C₁₋₆alkoxy;
- R¹⁰: is selected from H, halogen and C₁₋₆alkoxy;
- G₁: is selected from C₁₋₆alkyl and C₃₋₇cycloalkyl;
- G₂: is selected from C₁₋₆alkyl and C₃₋₇cycloalkyl;
- m: is selected from 0 and 1.

A further embodiment of the present invention is (x) a compound of formula (II) according to (i), or a pharmaceutically acceptable salt thereof, wherein
- R¹: is Cl;
- R⁶: is selected from H and methoxy;
- R⁷: is selected from carboxy and cyclopropylsulfonylaminocarbonyl;
- R⁸: is selected from H, Cl, Br, methyl, CF₃ and methoxy;
- R¹⁰: is selected from H, F, methoxy and ethoxy;
- G₁: is selected from methyl, ethyl, propyl and cyclobutyl;
- G₂: is selected from methyl and cyclobutyl;
- m: is selected from 0 and 1.

In another embodiment (xi) of the present invention, particular compounds of the present invention are selected from:
cis-3-[2-[2-bromo-5-(8-chloro-4-oxo-chromen-2-yl)-4-methoxy-phenoxy]ethoxy]cyclobutanecarboxylic acid;
3-[2-bromo-5-(8-chloro-4-oxo-chromen-2-yl)-4-methoxy-phenoxy]propanoic acid;
3-[2-bromo-5-(8-chloro-4-oxo-chromen-2-yl)-4-methoxy-phenoxy]-N-cyclopropylsulfonyl-propanamide;
2-[2-bromo-5-(8-chloro-4-oxo-chromen-2-yl)-4-methoxy-phenoxy]acetic acid;
3-[2-bromo-5-(8-chloro-4-oxo-chromen-2-yl)-4-methoxy-phenoxy]cyclobutanecarboxylic acid;
2-[3-[2-bromo-5-(8-chloro-4-oxo-chromen-2-yl)-4-methoxy-phenoxy]propoxy]acetic acid;
3-[2-chloro-5-(8-chloro-4-oxo-chromen-2-yl)-4-methoxy-phenoxy]cyclobutanecarboxylic acid;
cis-3-[2-[2-chloro-5-(8-chloro-4-oxo-chromen-2-yl)-4-methoxy-phenoxy]ethoxy]cyclobutanecarboxylic acid;
3-[2-[5-(8-chloro-4-oxo-chromen-2-yl)-4-methoxy-2-methyl-phenoxy]ethoxy]cyclobutanecarboxylic acid;
3-[5-(8-chloro-4-oxo-chromen-2-yl)-4-methoxy-2-methyl-phenoxy]propanoic acid;
2-[3-[5-(8-chloro-4-oxo-chromen-2-yl)-4-methoxy-2-methyl-phenoxy]propoxy]acetic acid;
3-[5-(8-chloro-4-oxo-chromen-2-yl)-2,4-dimethoxy-phenoxy]cyclobutanecarboxylic acid;
3-[2-[5-(8-chloro-4-oxo-chromen-2-yl)-2,4-dimethoxy-phenoxy]ethoxy]cyclobutanecarboxylic acid;
2-[5-(8-chloro-4-oxo-chromen-2-yl)-2-methyl-phenoxy]acetic acid;
cis-3-[5-(8-chloro-4-oxo-chromen-2-yl)-2-methyl-phenoxy]cyclobutanecarboxylic acid;
trans-3-[5-(8-chloro-4-oxo-chromen-2-yl)-2-methyl-phenoxy]cyclobutanecarboxylic acid;
3-[5-(8-chloro-4-oxo-chromen-2-yl)-2-methoxy-phenoxy]cyclobutanecarboxylic acid;
3-[2-[5-(8-chloro-4-oxo-chromen-2-yl)-2-methoxy-phenoxy]ethoxy]cyclobutanecarboxylic acid;
3-[5-(8-chloro-4-oxo-chromen-2-yl)-2-methoxy-phenoxy]propanoic acid;
cis-3-[5-(8-chloro-4-oxo-chromen-2-yl)-2-(trifluoromethyl)phenoxy]cyclobutanecarboxylic acid;
trans-3-[5-(8-chloro-4-oxo-chromen-2-yl)-2-(trifluoromethyl)phenoxy]cyclobutanecarboxylic acid;
2-[5-(8-chloro-4-oxo-chromen-2-yl)-2-(trifluoromethyl)phenoxy] acetic acid;
cis-3-[5-(8-chloro-4-oxo-chromen-2-yl)-2,3-dimethoxy-phenoxy]cyclobutanecarboxylic acid;
trans-3-[2-[5-(8-chloro-4-oxo-chromen-2-yl)-2,3-dimethoxy-phenoxy]ethoxy]cyclobutanecarboxylic acid;
2-[5-(8-chloro-4-oxo-chromen-2-yl)-2,3-dimethoxy-phenoxy] acetic acid;
trans-3-[4-bromo-5-(8-chloro-4-oxo-chromen-2-yl)-2-methoxy-phenoxy]cyclobutanecarboxylic acid;
trans-3-[2-[4-bromo-5-(8-chloro-4-oxo-chromen-2-yl)-2-methoxy-phenoxy]ethoxy]cyclobutanecarboxylic acid;
3-[3-(8-chloro-4-oxo-chromen-2-yl)-2-methoxy-phenoxy]cyclobutanecarboxylic acid;
cis-3-[2-[3-(8-chloro-4-oxo-chromen-2-yl)-2-methoxy-phenoxy]ethoxy]cyclobutanecarboxylic acid;
3-[3-(8-chloro-4-oxo-chromen-2-yl)-4-methoxy-phenoxy]cyclobutanecarboxylic acid;
cis-3-[2-[3-(8-chloro-4-oxo-chromen-2-yl)-4-methoxy-phenoxy]ethoxy]cyclobutanecarboxylic acid;
2-[3-(8-chloro-4-oxo-chromen-2-yl)-4-methoxy-phenoxy]acetic acid;
3-[3-(8-chloro-4-oxo-chromen-2-yl)-4-methoxy-phenoxy]propanoic acid;
cis-3-[2-[6-bromo-3-(8-chloro-4-oxo-chromen-2-yl)-2-methoxy-phenoxy]ethoxy]cyclobutanecarboxylic acid;
3-[6-bromo-3-(8-chloro-4-oxo-chromen-2-yl)-2-methoxy-phenoxy]cyclobutanecarboxylic acid;
3-[3-(8-chloro-4-oxo-chromen-2-yl)-2-methoxy-6-methyl-phenoxy]cyclobutanecarboxylic acid;
2-[6-chloro-3-(8-chloro-4-oxo-chromen-2-yl)-2-methoxy-phenoxy]acetic acid;
2-[3-(8-chloro-4-oxo-chromen-2-yl)-2,6-dimethoxy-phenoxy]acetic acid;
3-[2-bromo-5-(8-chloro-4-oxo-chromen-2-yl)-4-ethoxy-phenoxy]propanoic acid;
3-[2-bromo-5-(8-chloro-4-oxo-chromen-2-yl)-4-(difluoromethoxy)phenoxy]propanoic acid;
3-[2-bromo-5-(8-chloro-4-oxo-chromen-2-yl)-4-fluoro-phenoxy]propanoic acid;
3-[2-bromo-5-(8-chloro-4-oxo-chromen-2-yl)-4-(trifluoromethoxy)phenoxy]propanoic acid;
3-[2-bromo-4-chloro-5-(8-chloro-4-oxo-chromen-2-yl)phenoxy]propanoic acid;
2-[3-[6-bromo-3-(8-chloro-4-oxo-chromen-2-yl)-2-methoxy-phenoxy]propylamino]-2-oxo-acetic acid;
2-[2-[2-bromo-5-(8-chloro-4-oxo-chromen-2-yl)-4-methoxy-phenoxy]ethylamino]-2-oxo-acetic acid; and
2-[3-[2-bromo-5-(8-chloro-4-oxo-chromen-2-yl)-4-methoxy-phenoxy]propylamino]-2-oxo-acetic acid;
or a pharmaceutically acceptable salt thereof.

In another embodiment (xii) of the present invention, particular compounds of the present invention are selected from:
3-[2-bromo-5-(8-chloro-4-oxo-chromen-2-yl)-4-methoxy-phenoxy]propanoic acid;
3-[2-bromo-5-(8-chloro-4-oxo-chromen-2-yl)-4-methoxy-phenoxy]-N-cyclopropylsulfonyl-propanamide;
2-[2-bromo-5-(8-chloro-4-oxo-chromen-2-yl)-4-methoxy-phenoxy]acetic acid;
2-[3-[2-bromo-5-(8-chloro-4-oxo-chromen-2-yl)-4-methoxy-phenoxy]propoxy]acetic acid;
cis-3-[2-[2-chloro-5-(8-chloro-4-oxo-chromen-2-yl)-4-methoxy-phenoxy]ethoxy]cyclobutanecarboxylic acid;
3-[5-(8-chloro-4-oxo-chromen-2-yl)-2,4-dimethoxy-phenoxy]cyclobutanecarboxylic acid;
3-[2-[5-(8-chloro-4-oxo-chromen-2-yl)-2,4-dimethoxy-phenoxy]ethoxy]cyclobutanecarboxylic acid;
cis-3-[5-(8-chloro-4-oxo-chromen-2-yl)-2-methyl-phenoxy]cyclobutanecarboxylic acid;
2-[5-(8-chloro-4-oxo-chromen-2-yl)-2-(trifluoromethyl)phenoxy] acetic acid;
3-[3-(8-chloro-4-oxo-chromen-2-yl)-2-methoxy-phenoxy]cyclobutanecarboxylic acid;
cis-3-[2-[6-bromo-3-(8-chloro-4-oxo-chromen-2-yl)-2-methoxy-phenoxy]ethoxy]cyclobutanecarboxylic acid;
3-[6-bromo-3-(8-chloro-4-oxo-chromen-2-yl)-2-methoxy-phenoxy]cyclobutanecarboxylic acid;
2-[3-(8-chloro-4-oxo-chromen-2-yl)-2,6-dimethoxy-phenoxy] acetic acid;
3-[2-bromo-5-(8-chloro-4-oxo-chromen-2-yl)-4-ethoxy-phenoxy]propanoic acid; and
3-[2-bromo-5-(8-chloro-4-oxo-chromen-2-yl)-4-fluoro-phenoxy]propanoic acid;
or a pharmaceutically acceptable salt thereof.

### SYNTHESIS

The compounds of the present invention can be prepared by any conventional means. Suitable processes for synthesizing these compounds as well as their starting materials are provided in the schemes below and in the examples. All substituents, in particular, R¹ to R¹⁰, Gi, G₂ and m are as defined above unless otherwise indicated. Furthermore, and unless explicitly otherwise stated, all reactions, reaction conditions, abbreviations and symbols have the meanings well known to a person of ordinary skill in organic chemistry. 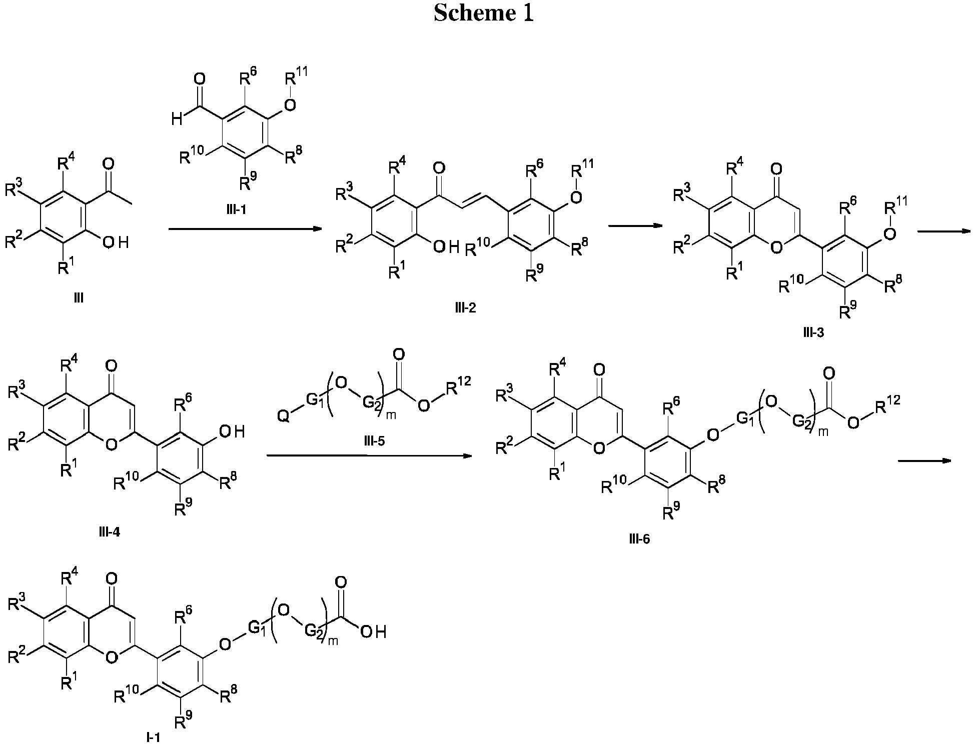 wherein R¹¹ is C₁₋₆alkyl, PMB or MOM; R¹² is C₁₋₆alkyl.

A compound of formula **III-2** can be prepared by condensation of a compound of formula **III** and a compound formula **III-1** in the presence of a base, such as KOH, in a suitable solvent, such as ethanol. Cyclization of a compound of formula **III-2** in the presence of **I**₂ in DMSO gives a compound of formula **III-3.** Deprotection of a compound of formula **III-3** in the presence of TFA or BBr₃ gives a compound of formula **III-4.** Alkylation of a compound of formula **III-4** with a compound of formula **III-5** affords a compound of formula **III-6** in the presence of a base, such as K₂CO₃ or Cs₂CO₃, in a suitable solvent, such as DMF or acetone. A compound of formula **I-1** is obtained by hydrolysis of a compound of formula **III-6** in the presence of a base, such as LiOH, or an acid, such as TFA. 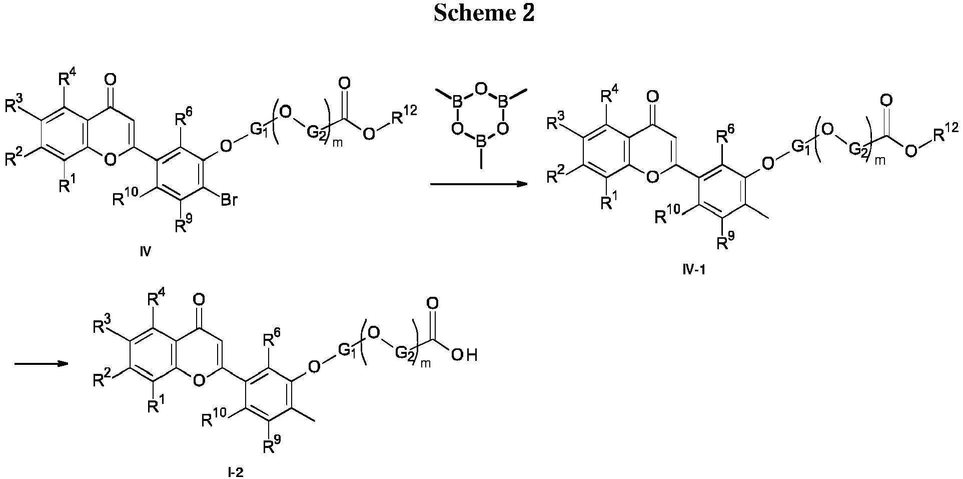 wherein R¹² is C₁₋₆alkyl.

A compound of formula **IV-1** can be prepared by the reaction of a compound of formula **IV** with trimethylboroxine in the presence of a palladium catalyst. A compound of formula **I-2** can be prepared by hydrolysis of a compound of formula **IV-1** in the presence of a base, such as LiOH, or an acid, such as TFA.

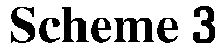

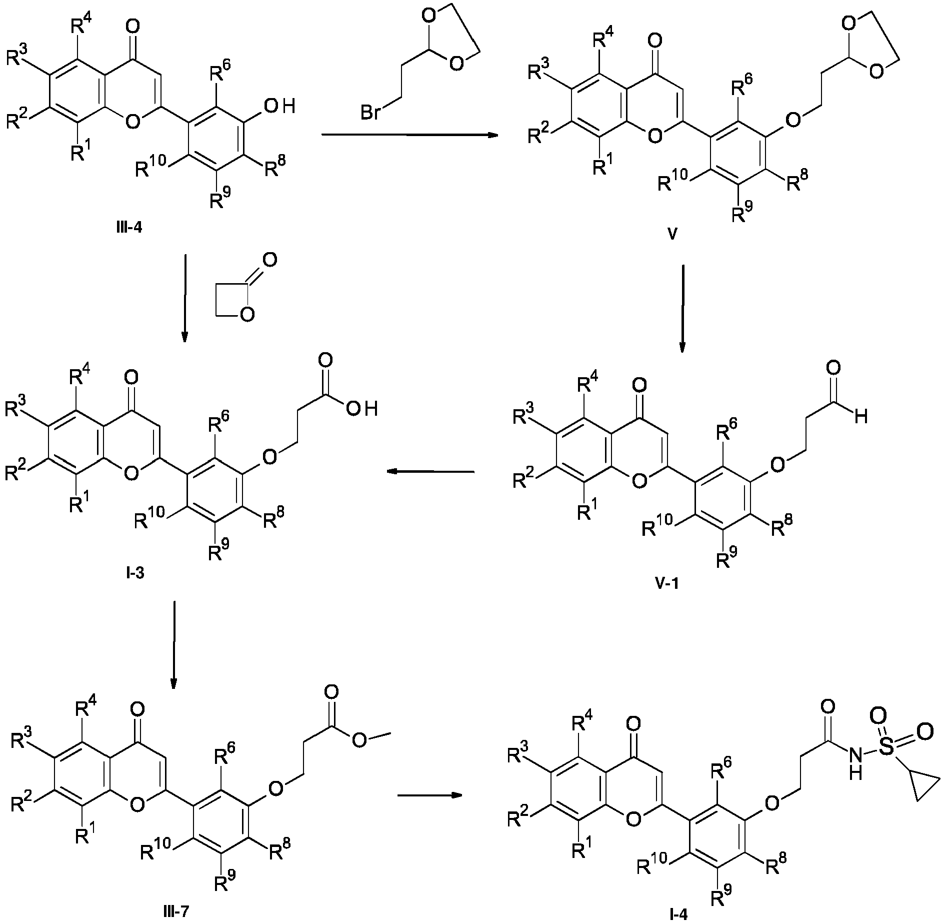

A compound of formula **I-3** can be prepared directly by reaction of a compound of formula **III-4** with oxetan-2-one in the presence of a base, such as sodium hydride, in a suitable solvent, such as DMF. Alternatively, alkylation of a compound of formula **III-4** with 2-(2-bromoethyl)-1,3-dioxolane in the presence of a base, such as K₂CO₃, Cs₂CO₃ in the presence of a suitable solvent, such as DMF or acetone, gives a compound of formula **V.** Deprotection of compound of formula **V** in the presence of TFA affords a compound of formula **V-1.** Oxidation of a compound of formula **V-1** using NaClO₂ gives compound of formula **I-3.** Treatment of a compound of formula **I-3** with thionyl chloride in the presence of methanol affords a compound of formula **III-7.** Reaction of a compound of formula **III-7** with sulfonamide in the presence of a Lewis acid, such as titanium tetrachloride, gives a compound of formula **I-4.** 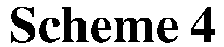 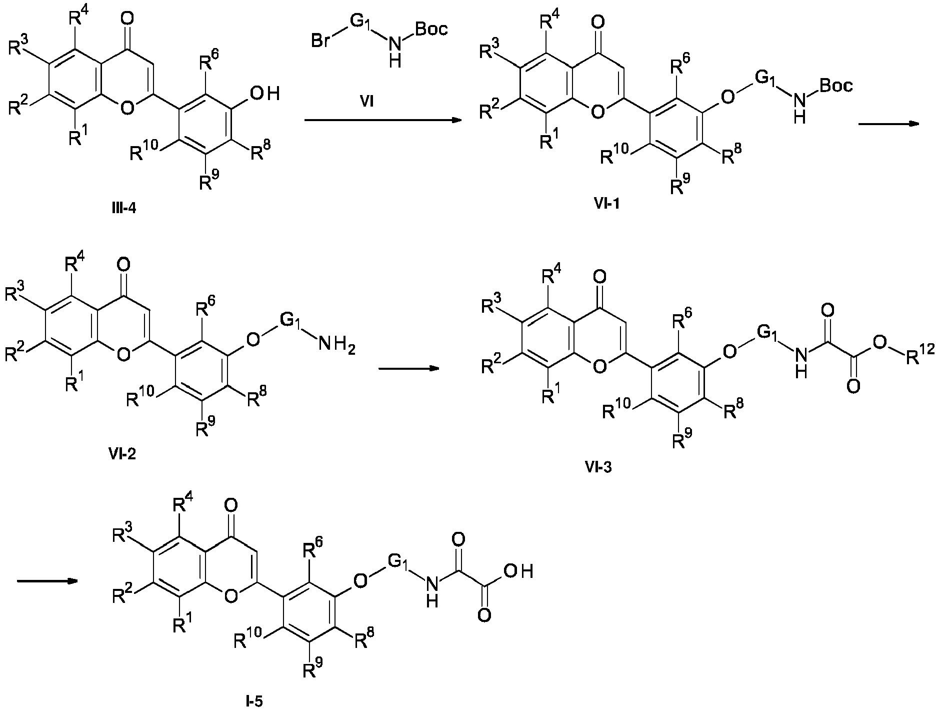 wherein R¹² is C₁₋₆alkyl.

Alkylation of a compound of formula **III-4** with bromide **VI** in the presence of a base, such as t-BuOK, in a suitable solvent, such as DMF, gives a compound of formula **VI-1.** Deprotection of a compound of formula **VI-1** in the presence of an acid, such as TFA, gives a compound of formula **VI-2.** Reaction of a compound of formula **VI-2** with ethyl 2-chloro-2-oxo-acetate in a suitable organic base, such as TEA, affords a compound of formula **VI-3.** Hydrolysis of a compound of formula **VI-3** in the presence of a base, such as LiOH or NaOH, affords a compound for formula **I-5.**

This invention also relates to a process for the preparation of a compound of formula (I) comprising at least one of the following steps:
(a) Hydrolysis of a compound of formula (III-6), 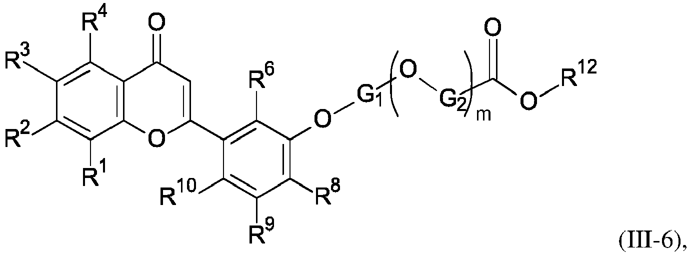 in the presence of a base or an acid;
(b) Hydrolysis of a compound of formula (IV-1), 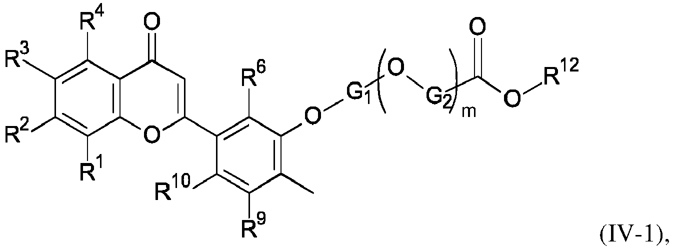 in the presence of a base or an acid;
(c) Reaction of a compound of formula (III-4), 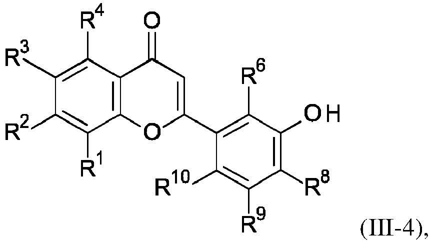 with oxetan-2-one in the presence of a base;
(d) Oxidation of a compound of formula (V-1), 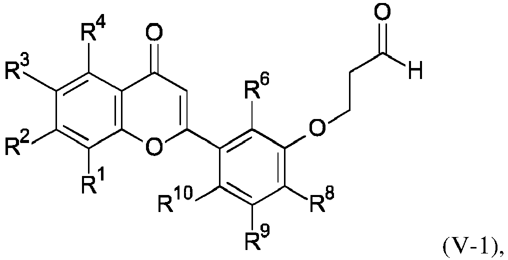 with NaClO₂;
(e) Reaction of a compound of formula (III-7), 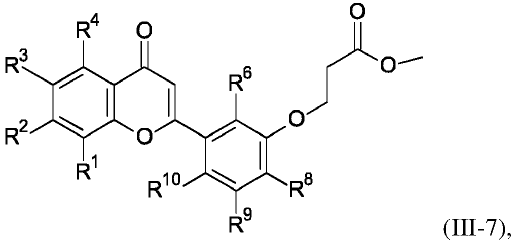 with sulfonamide in the presence of a Lewis acid;
(f) Hydrolysis of a compound of formula (VI-3), 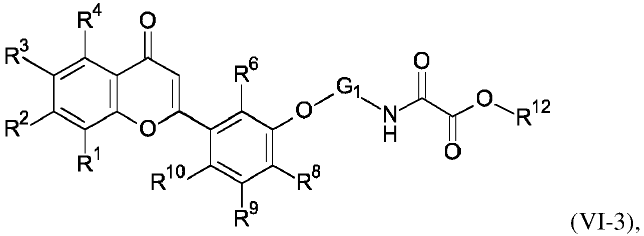 in the presence of a base; wherein R¹ to R⁴, R⁶, R⁸ to R¹⁰, Gi, G₂ and m are defined as any one of claims 1 to 3; R¹² is C₁₋₆alkyl.

The base in step (a) can be for example LiOH;
The acid in step (a) can be for example TFA;
The base in step (b) can be for example LiOH;
The acid in step (b) can be for example TFA;
The base in step (c) can be for example sodium hydride;
The Lewis acid in step (e) can be for example titanium tetrachloride;
The base in step (f) can be for example LiOH.

A compound of formula (I) or (II) when manufactured according to the above process is also an object of the invention.

The compound of this invention also shows good safety and PK profile.

### PHARMACEUTICAL COMPOSITIONS AND ADMINISTRATION

The invention also relates to a compound of formula (I) or (II) for use as therapeutically active substance. Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) or (II) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) or (II) is formulated in an acetate buffer, at pH 5. In another embodiment, the compounds of formula (I) or (II) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to inhibit cccDNA in HBV patients, consequently lead to the reduction of HBsAg and HBeAg (HBV e antigen) in serum. For example, such amount may be below the amount that is toxic to normal cells, or the mammal as a whole.

In one example, the pharmaceutically effective amount of the compound of the invention administered parenterally per dose will be in the range of about 0.1 to 100 mg/kg, alternatively about 0.1 to 50 mg/kg of patient body weight per day, with the typical initial range of compound used being 0.3 to 15 mg/kg/day. In another embodiment, oral unit dosage forms, such as tablets and capsules, preferably contain from about 25 to about 1000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 25 to 500 mg of the compound of the invention compounded with about 90 to 30 mg anhydrous lactose, about 5 to 40 mg sodium croscarmellose, about 5 to 30 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 400 mg, of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of Formula (I) or (II), or pharmaceutically acceptable salt or enantiomer or diastereomer thereof.

In a further embodiment includes a pharmaceutical composition comprising a compound of formula (I) or (II), or pharmaceutically acceptable salt or enantiomer or diastereomer thereof, together with a pharmaceutically acceptable carrier or excipient.

Another embodiment includes a pharmaceutical composition comprising a compound of formula (I) or (II), or pharmaceutically acceptable salt or enantiomer or diastereomer thereof for use in the treatment of HBV infection.

### INDICATIONS AND METHODS OF TREATMENT

The compounds of the invention can inhibit cccDNA and have anti-HBV activity. Accordingly, the compounds of the invention are useful for the treatment or prophylaxis of HBV infection.

The invention relates to the compound of formula (I) or (II) for the inhibition of cccDNA.

The invention also relates to the compound of formula (I) or (II) for the inhibition of HBeAg.

The invention further relates to the compound of formula (I) or (II) for the inhibition of HBsAg.

The invention relates to the compound of formula (I) or (II) for the inhibition of HBV DNA.

The invention relates to the compound of formula (I) or (II) for use in the treatment or prophylaxis of HBV infection.

The use of a compound of formula (I) or (II) for the preparation of medicaments useful in the treatment or prophylaxis diseases that are related to HBV infection is an object of the invention.

The invention relates in particular to the use of a compound of formula (I) or (II) for the preparation of a medicament for the treatment or prophylaxis of HBV infection.

Another embodiment includes a compound of formula (I) or (II) for use in a method for the treatment or prophylaxis of HBV infection, which method comprises administering an effective amount of a compound of Formula (I) or (II), or enantiomers, diastereomers, or pharmaceutically acceptable salts thereof.

### EXAMPLES

The invention will be more fully understood by reference to the following examples. They should not, however, be construed as limiting the scope of the invention.

Abbreviations used herein are as follows:
- ACN:: acetonitrile
- BBr3:: boron tribromide
- DMAP:: 4-dimethylaminopyridine
- DMF:: N,N-dimethylformamide
- IC50:: the molar concentration of an inhibitor, which produces 50% of the maximum possible response for that inhibitor.
- FBS:: fetal bovine serum
- HPLC:: high performance liquid chromatography
- MS (ESI):: mass spectroscopy (electron spray ionization)
- Ms:: methylsulfonyl
- obsd.:: observed
- PE:: petroleum ether
- EtOAc:: ethyl acetate
- AcOH:: acetic acid
- THF:: tetrahydrofuran
- TFA:: trifluoroacetic acid
- TEA:: triethyl amine
- DIPEA:: *N*,*N*-Diisopropylethylamine
- DIAD:: Diisopropyl azodicarboxylate
- Ts:: p-tolylsulfonyl
- δ:: chemical shift
- V/V:: volume ratio
- BOC:: butyloxylcarbonyl
- PMB:: *p*-methoxybenzyl
- MOM:: methoxymethyl
- TMS:: trimethylsilyl
- hr(s):: hour(s)
- min(s):: minute(s)
- TBS:: *tert*-butyldimethylsilane

### GENERAL EXPERIMENTAL CONDITIONS

Intermediates and final compounds were purified by flash chromatography using one of the following instruments: i) Biotage SP1 system and the Quad 12/25 Cartridge module. ii) column chromatography on silica gel combi-flash chromatography instrument. Silica gel Brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using X Bridge^{™} Perp Cis (5 µm, OBD^{™} 30 × 100 mm) column or SunFire^{™} Perp Cis (5 µm, OBD^{™} 30 × 100 mm) column.

LC/MS spectra were obtained using a Waters UPLC-SQD Mass. Standard LC/MS conditions were as follows (running time 3 minutes):
Acidic condition: A: 0.1% formic acid and 1% acetonitrile in H₂O; B: 0.1% formic acid in acetonitrile;
Basic condition: A: 0.05% NH₃·H₂O in H₂O; B: acetonitrile.

Mass spectra (MS): generally only ions which indicate the parent mass are reported, and unless otherwise stated the mass ion quoted is the positive mass ion (M+H)⁺.

NMR spectra were obtained using Bruker Avance 400MHz.

All reactions involving air-sensitive reagents were performed under an argon atmosphere. Reagents were used as received from commercial suppliers without further purification unless otherwise noted.

### PREPARATIVE EXAMPLES

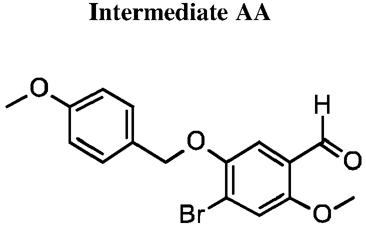

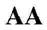

### Step 1. Synthesis of 4-bromo-5-hydroxy-2-methoxy-benzaldehyde

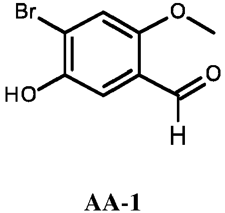

To a solution of 5-hydroxy-2-methoxy-benzaldehyde (1.6 g) in dry THF (20 mL) and tetrachloroethylene (40 mL) at 0 °C was added *N*-bromosuccinimide (1.87 g, 10.5 mmol) in small portions. After being stirred at 50 °C for 16 hrs, the reaction mixture was poured into water (150 mL). The resulting solution was extracted with DCM (100 mL) three times. The combined organics layer was dried over anhydrous Na₂SO₄, filtered and concentrated *in vacuo.* The resulting oil was purified by flash column chromatography (eluting with EtOAc/PE = 1/5) to give 4-bromo-5-hydroxy-2-methoxy-benzaldehyde (1.0 g, compound **AA-1**) as a brown solid. MS obsd. (ESI⁺): 231.0 [(M+H)⁺], 233.0 [(M+2+H)⁺].

### Step 2. Synthesis 4-bromo-2-methoxy-5-[(4-methoxyphenyl)methoxy]benzaldehyde

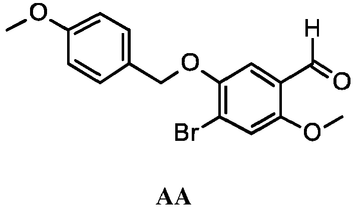

To a solution of 4-bromo-5-hydroxy-2-methoxy-benzaldehyde (1.0 g, **AA-1**) in dry DMF (20 mL) at 0 °C was added sodium hydride (180 mg) in small portions. The reaction mixture was stirred at 0 °C for 0.5 hr. Then 4-methoxybenzylchloride (0.61 mL) was added dropwise. After being stirred at 25 °C for 16 hrs, the reaction mixture was poured into ice/water (150 g), extracted with EtOAc/THF(V/V)= 4/1 (100 mL) three times. The combined organic layer was washed with brine (50 mL), dried over anhydrous sodium sulfate, filtered and concentrated *in vacuo.* The crude product was purified by flash silica gel chromatography (eluting with EtOAc/PE = 1/10) to give 4-bromo-2-methoxy-5-[(4-methoxyphenyl)methoxy]benzaldehyde (800 mg, Intermediate **AA**) as a white solid. MS obsd. (ESI⁺): 373.0 [(M+Na)⁺], 375.0 [(M+2+Na)⁺].

### Intermediae AB

### Step1. Synthesis of 4-chloro-5-hydroxy-2-methoxy-benzaldehyde

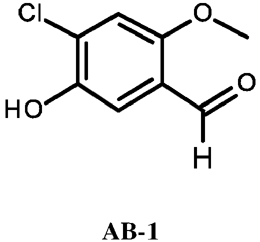

To a solution of 5-hydroxy-2-methoxy-benzaldehyde (0.5 g, 3.29 mmol) in dry THF (20 mL) and tetrachloroethylene (20 mL) was added N-chlorosuccinimide (0.53 g). After being stirred at 25 °C for 16 hrs, the reaction mixture was concentrated *in vacuo.* The resulting reaction mixture was diluted with EtOAc (100 mL), washed with brine (50 mL), dried over anhydrous sodium sulfate, filtered and concentrated *in vacuo.* The residue was purified by flash silica gel chromatography (eluting with EtOAc/PE = 1/4) to give 4-chloro-5-hydroxy-2-methoxybenzaldehyde (450 mg, compound **AB-1**) as a yellow solid. MS obsd. (ESI⁺): 187.1 [(M+H)⁺], 189.1 [(M+2+H)⁺].

### Step 2. Synthesis of 4-chloro-2-methoxy-5-(methoxymethoxy)benzaldehyde

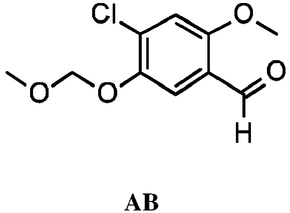

To a solution of 4-chloro-5-hydroxy-2-methoxy-benzaldehyde (0.4 g, **AB-1**) in dry DMF (10 mL) at 0 °C was added sodium hydride (154 mg, 3.86 mmol) in small portions. The reaction mixture was stirred at 0 °C for 0.5 h, bromomethyl methyl ether (482 mg) was added dropwise. After being stirred at 25 °C for 6 hrs, the reaction mixture was poured into ice/water (50 g), extracted with EtOAc (30 mL) three times. The combined organic layer was washed with brine (25 mL), dried over anhydrous sodium sulfate, filtered and concentrated *in vacuo.* The crude product was purified by flash silica gel chromatography (eluting with EtOAc/PE = 1/10) to give 4-chloro-2-methoxy-5-(methoxymethoxy)benzaldehyde (280 mg, Intermediate A**B**) as a white solid. MS obsd. (ESI⁺): 231.1 [(M+H)⁺], 233.1 [(M+2+H)⁺].

### Intermediate AC

### 5-Hydroxy-2,4-dimethoxy-benzaldehyde

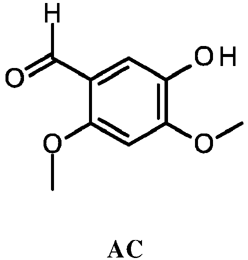

To a solution of sodium methoxide (4.21 g, 77.9 mmol) in DMF (50 ml) was added copper (I) iodide (247 mg, 1.3 mmol) and 2-bromo-5-hydroxy-4-methoxybenzaldehyde (3 g, 13 mmol). The resulting mixture was refluxed for 4 hrs. After it was cooled to room temperature and filtered, the filtrate was transferred to an Erlenmeyer flask containing 30 g of ice and 10 mL of concentrated HCl and extracted with EtOAc (50 mL) three times. The organic layer was dried over Na₂SO₄ and concentrated *in vacuo* to give of 5-hydroxy-2,4-dimethoxy-benzaldehyde (1.8 g, Intermediate **AC**) as a yellow solid. MS obsd. (ESI⁺) [(M+H)⁺]:183.

### Intermediate AD

### 3-[Tert-butyl(dimethyl)silyl]oxy-2-methoxy-benzaldehyde

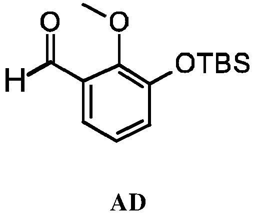

### Step 1. Synthesis of 3-[tert-butyl(dimethyl)silyl]oxy-2-hydroxy-benzaldehyde

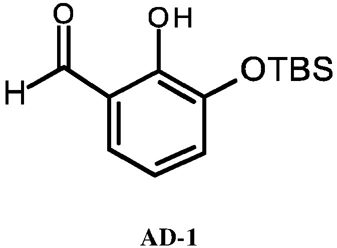

To a solution of *tert*-butyldimethylchlorosilane (7.19 mL) and imidazole (3.75 g) in DCM (100 mL) was added 2,3-dihydroxybenzaldehyde (5.07 g). After being stirred at 25 °C for 16 hrs, the reaction mixture was diluted with DCM (100 mL). The organic phase was washed with water (150 mL), brine (150 mL), dried over anhydrous sodium sulfate, filtered and concentrated *in vacuo.* The residue was purified by flash silica gel chromatography (eluting with EtOAc/PE = 1/20) to give 3-[*tert*-butyl(dimethyl)silyl]oxy-2-hydroxy-benzaldehyde (7.2 g, **AD-1**) as a yellow oil. MS obsd. (ESI⁺): 253.1 [(M+H)⁺], 275.1 [(M+Na)⁺].

### Step 2. Synthesis of 3-[tert-butyl(dimethyl)silyl]oxy-2-methoxy-benzaldehyde

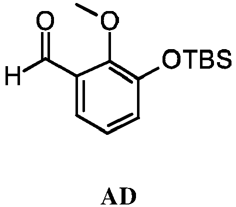

To a solution of 3-[*tert*-butyl(dimethyl)silyl]oxy-2-hydroxy-benzaldehyde (7.2 g) and potassium carbonate (6.3 g) in DMF (70 mL) was added iodomethane (6.56 g). After being stirred at 25 °C for 16 hrs, the reaction mixture was filtered through celite. The filtered cake was washed with EtOAc (100 mL). The combined filtrate was concentrated *in vacuo.* The residue was diluted with EtOAc (200 mL), washed with brine (50 mL), dried over anhydrous sodium sulfate, filtered and concentrated *in vacuo.* The crude product was purified by flash silica gel chromatography (eluting with EtOAc/PE = 1/10) to give 3-[*tert*-butyl(dimethyl)silyl]oxy-2-methoxy-benzaldehyde (6 g, Intermediate **AD**) as a colorless oil. MS obsd. (ESI⁺): 267.1 [(M+H)⁺], 289.1 [(M+Na)⁺].

### Intermediate AE

### 5-Hydroxy-2-methoxy-benzaldehyde

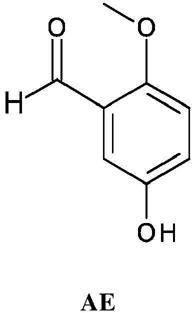

### Step 1. Synthesis of 2,5-dimethoxybenzaldehyde

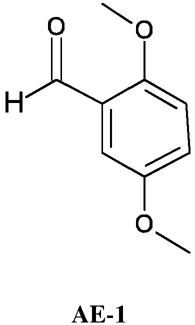

To a solution of 2,5-dihydroxybenzaldehyde (2.76 g) and potassium carbonate (11.06 g) in dry DMF (40 mL) was added iodomethane (4.98 mL). After being stirred at 25 °C for 16 hrs, the reaction mixture was filtered through celite. The filtered cake was washed with DCM (100 mL), the combined filtrate was concentrated *in vacuo.* The residue was diluted with EtOAc (200 mL), washed with brine (50 mL), dried over anhydrous sodium sulfate, filtered and concentrated *in vacuo.* The crude product was purified by flash silica gel chromatography (eluting with EtOAc/PE = 1/10) to give 2,5-dimethoxybenzaldehyde (2.4 g. compound **AE-1**) as a white solid. MS obsd. (ESI⁺): 167.1 [(M+H)⁺], 189.1 [(M+Na)⁺].

### Step 2. Synthesis of 5-hydroxy-2-methoxy-benzaldehyde

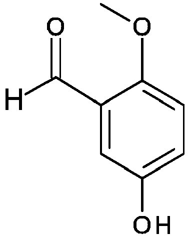

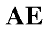

To 2,5-dimethoxybenzaldehyde (1.2 g, compound **AE-1**) at 0 °C was added sulfuric acid (6.5 mL). After being stirred at 60 °C for 72 hrs, the reaction mixture was cooled to room temperature and poured into ice (250 g). The precipitated oil was extracted with diethyl ether (50 mL) three times. The diethyl ether solution was washed with 20 mL of 5% sodium hydroxide, the water solution was acidified to pH = 5 with 2 *M* HCl at 0 °C, extracted with diethyl ether (50 mL) three times. The combined organics were washed with brine (50 mL), dried over anhydrous Na₂SO₄, filtered and concentrated *in vacuo.* The crude product was purified by flash column chromatography (eluting with EtOAc/PE = 2/3) to give 5-hydroxy-2-methoxy-benzaldehyde (300 mg, Intermediate **AE**) as a yellow solid. MS obsd. (ESI⁺): 153.1 [(M+H)⁺], 175.1 [(M+Na)⁺].

### Intermediate AF

### 4-Bromo-3-hydroxy-2-methoxy-benzaldehyde

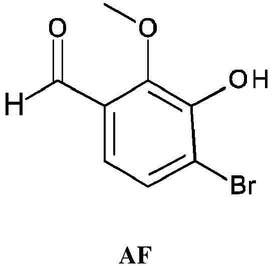

### Step 1. Synthesis of 3-hydroxy-2-methoxy-benzaldehyde

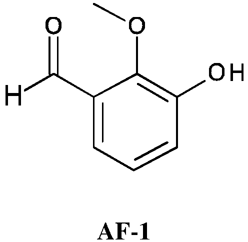

To a solution of 3-[tert-butyl(dimethyl)silyl]oxy-2-methoxy-benzaldehyde (1.1 g) in methanol (10 mL) was added a solution of hydrochloric acid (10.0 mL) in methanol (4 *M*)*.* After being stirred at 25 °C for 16 hrs, the reaction mixture was concentrated *in vacuo.* The crude product was diluted with EtOAc (100 mL), washed with brine (100 mL), dried over anhydrous Na₂SO₄, filtered and concentrated *in vacuo* to give 3-hydroxy-2-methoxy-benzaldehyde (550 mg, compound **AF-1**) as a brown solid which was used for next step without purification. MS obsd. (ESI⁺): 153.1 [(M+H)⁺], 175.1 [(M+Na)⁺].

### Step 2. Synthesis of 4-bromo-3-hydroxy-2-methoxy-benzaldehyde

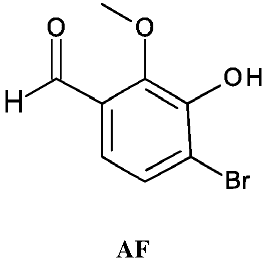

To a solution of 3-hydroxy-2-methoxy-benzaldehyde (0.32 g, compound **AF-1**) in dry DCM (25 mL) was added *N*-bromosuccinimide (0.45 g). After being stirred at 25 °C for 16 hrs, the reaction mixture was diluted with DCM (25 mL), washed with brine (25 mL), dried over anhydrous sodium sulfate, filtered and concentrated *in vacuo.* The crude product was purified by flash silica gel chromatography (eluting with EtOAc/PE = 1/4) to give 4-bromo-3-hydroxy-2-methoxy-benzaldehyde (420 mg, Intermediate **AF**) as a white solid. MS obsd. (ESI⁺): 231.0 [(M+H)⁺], 233.0 [(M+2+H)⁺].

### Intermediate AG

### 2-Methoxy-3-(methoxymethoxy)-4-methyl-benzaldehyde

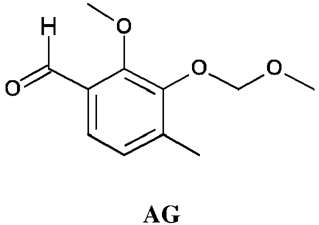

### Step 1. Synthesis of 3-hydroxy-2-methoxy-4-methyl-benzaldehyde

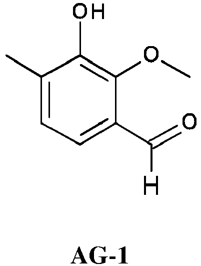

To a solution of 4-bromo-3-hydroxy-2-methoxy-benzaldehyde (420.0 mg) in 1,4-dioxane (20 mL) were added [1,1'-bis(diphenylphosphino)ferrocene]dichloropalladium(II) (110.4 mg), cesium carbonate (983 mg). The mixture was degassed with N₂ for 3 times. Then trimethylboroxine (378.81 mg) was added. After being stirred at 100 °C for 16 hrs, the reaction mixture was diluted with EtOAc (30 mL), filtered through celite, acidified to pH = 3 with 4 *M* HCl, extracted with EtOAc (30 mL) three times. The combined organics were dried over anhydrous Na₂SO₄, filtered and concentrated *in vacuo.* The crude product was purified by flash silica gel chromatography (eluting with EtOAc/PE = 1/5) to give 3-hydroxy-2-methoxy-4-methyl-benzaldehyde (200 mg, compound **AG-1**) as a yellow solid. MS obsd. (ESI⁺): 167.1 [(M+H)⁺].

### Step 2. Synthesis of 2-methoxy-3-(methoxymethoxy)-4-methyl-benzaldehyde

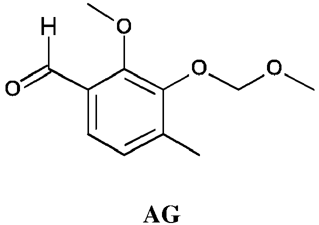

To a solution of 3-hydroxy-2-methoxy-4-methyl-benzaldehyde (180.0 mg) in dry DMF (10 mL) was added sodium hydride (58 mg) in small portions at 0 °C. After the mixture was stirred for 0.5 h, then bromomethyl methyl ether (180.71 mg) was added dropwise. After being stirred at 25 °C for 16 hrs, the reaction mixture was poured into ice/water (50 g), extracted with EtOAc (30 mL) three times. The combined organic layer was washed with brine (25 mL), dried over anhydrous sodium sulfate, filtered and concentrated *in vacuo.* The crude product was purified by flash silica gel chromatography (eluting with EtOAc/PE = 1/10) to give 2-methoxy-3-(methoxymethoxy)-4-methyl-benzaldehyde (200 mg, Intermediate **AG**) as a white solid. MS obsd. (ESI⁺): 211.1 [(M+H)⁺].

### Intermediate AH

### Step 1. Synthesis of 4-chloro-3-hydroxy-2-methoxy-benzaldehyde

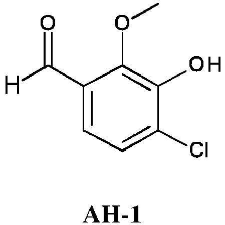

To a solution of 3-hydroxy-2-methoxy-benzaldehyde (2.0 g) in dry THF (37.5 mL) and tetrachloroethylene (75 mL) was added *N*-chlorosuccinimide (1.93 g) in small portions at 0 °C. After addition, the mixture was degassed with N₂ for 3 times. After being stirred at 50 °C for 48 hrs, the reaction mixture was poured into water (150 mL), and the resulting biphasic mixture was extracted with DCM (100 mL) three times. The combined organics were dried over anhydrous Na₂SO₄, filtered and concentrated *in vacuo.* The resulting oil was purified by flash silica gel column chromatography (eluting with EtOAc/PE = 1/5) to give 4-chloro-3-hydroxy-2-methoxybenzaldehyde (1.7 g, compound **AH-1**) as a white solid. MS obsd. (ESI⁺): 187.1 [(M+H)⁺], 189.1 [(M+2+H)⁺].

### Step 2. Synthesis of 4-chloro-2-methoxy-3-[(4-methoxyphenyl)methoxy]benzaldehyde

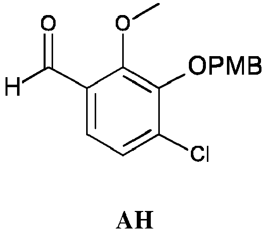

To a solution of 4-chloro-3-hydroxy-2-methoxy-benzaldehyde (1.7 g, compound **AH-1**) in dry DMF (30 mL) was added sodium hydride (398 mg) in small portions at 0 °C. After being stirred at 0 °C for 0.5 h, a solution of 4-methoxybenzylchloride (1.35 mL) in dry DMF (10 mL) was added dropwise. After being stirred at 25 °C for 6 hrs, the reaction mixture was poured into ice/water (150 g) and extracted with EtOAc/THF = 4/1 (100 mL) three times. The combined organic layer was washed with brine (150 mL), dried over anhydrous sodium sulfate, filtered and concentrated *in vacuo.* The crude product was purified by flash silica gel chromatography (eluting with EtOAc/PE = 1/10) to give 4-chloro-2-methoxy-3-[(4-methoxyphenyl)methoxy]benzaldehyde (1.1 g, Intermediate **AH** ) as a white solid. MS obsd. (ESI⁺): 329.0 [(M+Na)⁺], 331.1 [(M+2+Na)⁺].

### Intermediate AI

### 2,4-Dimethoxy-3-[(4-methoxyphenyl)methoxy]benzaldehyde

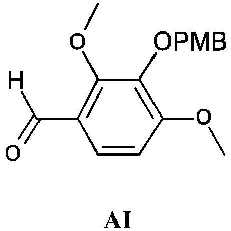

### Step 1. Synthesis of 3-hydroxy-2,4-dimethoxy-benzaldehyde

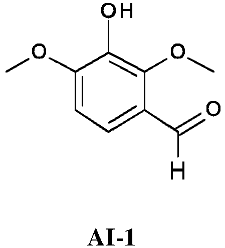

To a solution of 3-[tert-butyl(dimethyl)silyl]oxy-2,4-dimethoxy-benzaldehyde (3.8 g) in DCM (39 mL) at 0 °C, was added a solution of hydrochloric acid (32 mL) in MeOH (4 *M*). After addition, the mixture was stirred 25 °C for 16 hrs. Then the reaction mixture was concentrated *in vacuo,* diluted with ethyl acetate (200 mL), washed with brine (50 mL) two times, dried over anhydrous sodium sulfate, filtered and concentrated *in vacuo* to give 3-hydroxy-2,4-dimethoxybenzaldehyde (2.2 g, compound **AI-1**) as a yellow oil. MS obsd. (ESI⁺): 183.1 [(M+H)⁺].

### Step 2. Synthesis of 2,4-dimethoxy-3-[(4-methoxyphenyl)methoxy]benzaldehyde

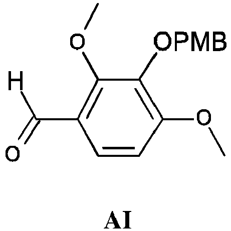

To a solution of 3-hydroxy-2,4-dimethoxy-benzaldehyde (2.2 g, compound **AI-1**) in DMF (50 mL) at 25 °C were added potassium iodide (0.13 mL), dipotassium carbonate (2.5 g) and 4-methoxybenzylchloride (2.46 mL). After being stirred at 60 °C for 16 hrs, the reaction mixture was poured into ice/water (250 mL), extracted with EtOAc (100 mL) three times. The combined organic layer was washed with brine (100 mL) four times. The organic phase was dried over anhydrous sodium sulfate, filtered and concentrated *in vacuo.* The crude product was purified by flash silica gel chromatography (eluting with EtOAc/PE = 3/7) to give 2,4-dimethoxy-3-[(4-methoxyphenyl)methoxy]benzaldehyde (3 g, Intermediate A**I**) as a yellow solid.MS obsd. (ESI⁺): 325.1 [(M+Na)⁺].

### Intermediate AJ

### Synthesis of 4-bromo-2-hydroxy-5-(methoxymethoxy)benzaldehyde

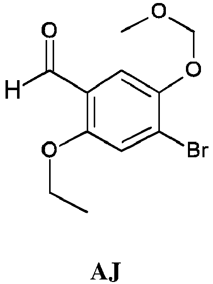

### Step 1. Synthesis of 4-bromo-2-hydroxy-5-(methoxymethoxy)benzaldehyde

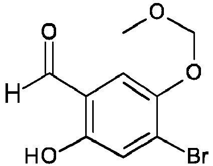

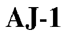

To a solution of 4-bromo-2,5-dihydroxy-benzaldehyde (1.1 g, 5.07 mmol) in THF (6.29 mL) was added *N*,*N*-diisopropylethylamine (1.77 mL, 10.14 mmol). The reaction was stirred at 0 °C for 10 mins. Then bromomethyl methyl ether (0.41 mL, 5.07 mmol) was added. After being stirred at 0 °C for 1 hr, the reaction mixture was diluted with water (20 mL) and extracted with EtOAc (30 mL) three times. The combined organic layer was washed with brine (30 mL) two times, then the organic layer was dried with MgSO₄, filtered and concentrated. The crude was purified by flash column chromatography (eluting with EtOAc/PE = 1/10) to afford 4-bromo-2-hydroxy-5-(methoxymethoxy)benzaldehyde (200 mg, 15% yield, compound **AJ-1**) as a white solid. MS obsd. (ESI⁺): 261.0 [(M+H)⁺], 263.0 [(M+2+H)⁺].

### Step 2. Synthesis 4-bromo-2-ethoxy-5-(methoxymethoxy)benzaldehyde

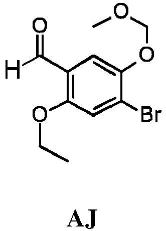

To a mixture of 4-bromo-2-hydroxy-5-(methoxymethoxy)benzaldehyde (100.0 mg), potassium carbonate (158 mg) in ACN (3 mL) was added iodoethane (0.06 mL). The mixture was stirred at 25 °C for 1.5 h. The reaction mixture was added water (20 mL) and extracted with EtOAc (30 mL) three times. The combined organic layer was washed with brine, dried over anhydrous sodium sulfate and concentrated. The residue was purified by flash column chromatography (eluting with EtOAc/PE = 1/10) to give 4-bromo-2-ethoxy-5-(methoxymethoxy)benzaldehyde (90 mg, 81%, Intermediate **AJ**) as a yellow solid. ¹H NMR (400 MHz, DMSO-*d₆*): δ ppm 10.11-10.45 (m, 1H), 7.49-7.67 (m, 1H), 7.44 (s, 1H), 5.14-5.38 (m, 2H), 4.05-4.32 (m, 2H), 3.36-3.54 (m, 3H), 1.25-1.46 (m, 3H). MS obsd. (ESI⁺): 289.0 [(M+H)⁺], 291.0 [(M+2+H)⁺].

### Intermediate AK

### 4-Bromo-2-(difluoromethoxy)-5-(methoxymethoxy)benzaldehyde

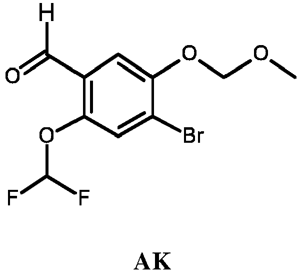

### Step 1. Synthesis of 4-bromo-2-hydroxy-5-(methoxymethoxy)benzaldehyde

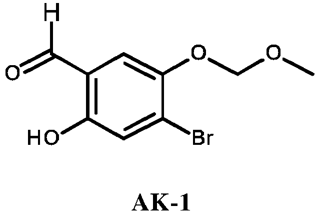

To a solution of 4-bromo-2,5-dihydroxy-benzaldehyde (3.45 g) in THF (40 mL) was added *N*,*N*-diisopropylethylamine (5.54 mL). The reaction was stirred at 25 °C for 10 min, then bromomethyl methyl ether (1.03 mL) was added. After being stirred at 25 °C for 1.5 h, the reaction mixture was diluted with water (20 mL). The solution was extracted with EtOAc (30 mL) three times. The combined organic layer was washed with brine (30 mL) two times, then the organic layer was dried with MgSO₄, filtered and concentrated. The crude was purified by flash column chromatography (eluting with EtOAc/PE = 1/10) to afford 4-bromo-2-hydroxy-5-(methoxymethoxy)benzaldehyde (1.8 g, 43% yield, compound **AK-1**) as a white solid. MS obsd. (ESI⁺): 261.0 [(M+H)⁺], 263.0 [(M+2+H)⁺].

### Step 2. Synthesis of 5-bromo-2-(hydroxymethyl)-4-(methoxymethoxy)phenol

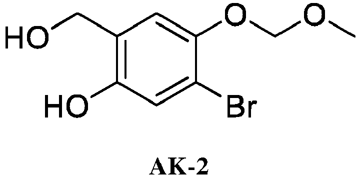

To the solution of 4-bromo-2-hydroxy-5-(methoxymethoxy)benzaldehyde (300.0 mg, compound **AK-1**) in THF (15 mL) at 0 °C was added sodium borohydride (65 mg). Then the mixture was stirred at 25 °C for 1 hr. The reaction mixture was poured into water (30 mL) and extracted with EtOAc (30 mL) three times. The combined organic layer was washed with brine, dried over anhydrous sodium sulfate and concentrated. The residue was purified by flash silica column chromatography (eluting with EtOAc/PE = 1/10) to give 5-bromo-2-(hydroxymethyl)-4-(methoxymethoxy)phenol (200 mg, 66% yield, compound **AD-2**) as a yellow solid. MS obsd. (ESI⁺): 283.0 [(M+Na)⁺], 285.0 [(M+2+Na)⁺].

### Step 3. Synthesis of 4-bromo-2-(difluoromethoxy)-5-(methoxymethoxy)benzaldehyde

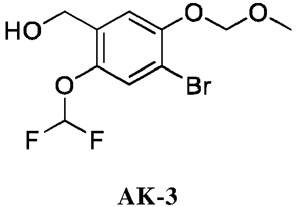

To a solution of 5-bromo-2-(hydroxymethyl)-4-(methoxymethoxy)phenol (200.0 mg, compound **AK-2**) in DMF (3.3 mL) was added (2-chloro-2,2-difluoro-acetyl)oxysodium (127 mg) and cesium carbonate (743 mg). After being stirred at 80 °C for 3 hrs, the reaction mixture was added water (50 mL) and extracted with EtOAc (50 mL) three times. The combined organic layer was washed with brine, dried over anhydrous sodium sulfate and concentrated. The residue was purified by flash silica column chromatography (eluting with EtOAc/PE = 1/4) to give 4-bromo-2-(difluoromethoxy)-5-(methoxymethoxy)benzaldehyde (160 mg, 68% yield, compound **AK-3**) as a yellow solid. MS obsd. (ESI⁺): 295.0 [(M-18+H)⁺], 297.0 [(M-18+2+H)⁺].

### Step 4. Synthesis of 4-bromo-2-(difluoromethoxy)-5-(methoxymethoxy)benzaldehyde (A4)

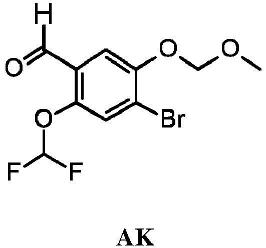

A mixture of [4-bromo-2-(difluoromethoxy)-5-(methoxymethoxy)phenyl]methanol (150.0 mg, compound **AK-3**) and manganese dioxide (416.53 mg) in DCM (2.5 mL) was stirred at 25 °C for 10 hrs. The mixture was filtered and concentrated. The residue was purified by flash column chromatography (eluting with EtOAc/PE = 1/10) to give 4-bromo-2-(difluoromethoxy)-5-(methoxymethoxy)benzaldehyde (90 mg, 60% yield, Intermediate **AK**) as a yellow solid. ¹H NMR (400 MHz, DMSO-d₆): δ ppm 10.18 (s, 1H), 7.76 (s, 1H), 7.57 (s, 1H), 7.51 (s, 0.25H), 7.33 (s,0.5H), 7.14 (s,0.25H), 5.37 (s, 2H), 3.40 (d, *J* = 5.5 Hz, 4H). MS obsd. (ESI⁺): 291.0 [(M-18+H)⁺], 293.0 [(M-18+2+H)⁺].

### Intermediate AL

### 4-Bromo-2-fluoro-5-(methoxymethoxy)benzaldehyde

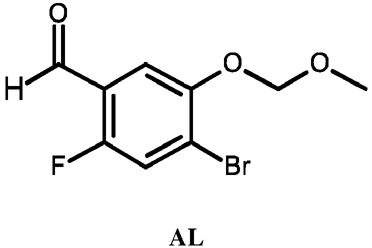

### Step 1. Synthesis of 4-bromo-2-fluoro-5-methoxy-benzaldehyde

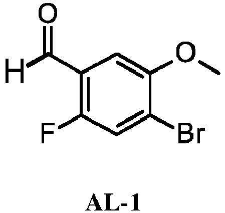

To a 25 mL round-bottom flask was added titanium tetrachloride (8.42 mL), followed by 2-bromo-4-fluoroanisole (3.15 g) in N₂. The stirred mixture was cooled in an ice water bath and treated dropwise with 1,1-dichlorodimethyl ether (6.95 mL). After being stirred for 1 h, water (20 mL) was added. The solution was extracted with EtOAc (50 mL) three times. The combined organic layer was washed with brine (50 mL) two times, dried with MgSO₄, filtered and concentrated. The crude was purified by flash column chromatography (eluting with EtOAc/PE = 1/20) to afford 4-bromo-2-fluoro-5-methoxy-benzaldehyde (1.6 g, 44% yield, **AL-1**) as a white solid. MS obsd. (ESI⁺): 233.0 [(M+H)⁺], 234.9 [(M+H+2)⁺].

### Step 2. Synthesis of 4-bromo-2-fluoro-5-hydroxy-benzaldehyde

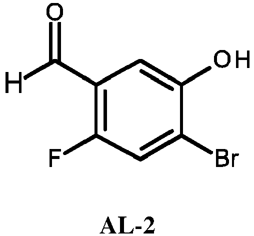

To a solution of 4-bromo-2-fluoro-5-methoxy-benzaldehyde (1.6 g, **AL-1**) in DCM (30 mL) at -78 °C was added boron tribromide (3.31 mL). After being stirred at -78 °C for 1 hr, water was added. The solution was extracted with DCM (50 mL) three times. The combined organic layer was washed with brine (50 mL) three times, then the organic layer was dried with MgSO₄, filtered and concentrated. The crude was purified by flash column chromatography (eluting with EtOAc/PE = 1/4) to afford 4-bromo-2-fluoro-5-hydroxy-benzaldehyde (1.35 g, 89% yield, compound **AL-2**) as a white solid. MS obsd. (ESI⁺): 219.0 [(M+H)⁺], 220.9 [(M+H+2)⁺].

### Step 2. Synthesis of 4-bromo-2-fluoro-5-(methoxymethoxy)benzaldehyde

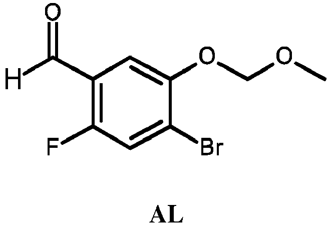

To a solution of 4-bromo-2-fluoro-5-hydroxy-benzaldehyde (300.0 mg, **AL-2**) in THF (6 mL) was added sodium hydride (82 mg), the reaction was stirred at 0 °C for 10 min. Then bromomethyl methyl ether (205.4 mg) was added. After being stirred at 0 °C for 1 h, the reaction mixture was diluted with water (20 mL) and extracted with EtOAc (30 mL) three times. The combined organic layer was washed with brine (30 mL) two times, then the organic layer was dried with MgSO₄, filtered and concentrated. The crude was purified by flash column chromatography (eluting with EtOAc/PE = 1/10) to afford 4-bromo-2-fluoro-5-(methoxymethoxy)benzaldehyde (260 mg, 72% yield, Intermediate **AE**) as a white solid. MS obsd. (ESI⁺): 263.0 [(M+H)⁺], 265.0 [(M+H+2)⁺].

### Intermediate AM

### 4-Bromo-2-chloro-5-(methoxymethoxy)benzaldehyde

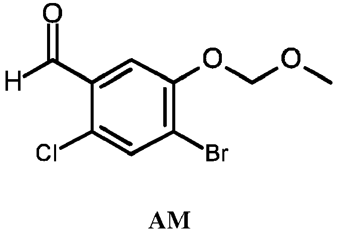

### Step 1. Synthesis of 4-bromo-2-chloro-5-hydroxy-benzaldehyde

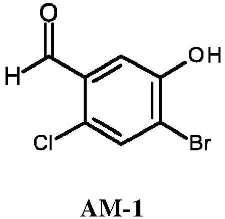

To a solution of 2-chloro-5-hydroxy-benzaldehyde (2.0 g) in chloroform (20 mL) was added bromine (2.0 g) and the mixture was stirred at 25 °C for 1 hrs. The reaction was concentrated to dryness and the residue was taken up in EtOAc (50 mL) and washed with water (50 mL) three times. The organic phase was dried over MgSO₄ and concentrated. The crude product was then purified by flash column chromatography (eluting with EtOAc/isohexane = 1/4. The desired fractions were concentrated *in vacuo* to give 4-bromo-2-chloro-5-hydroxybenzaldehyde (1.50 g, compound **AM-1**) as a white solid MS obsd. (ESI⁺): 249.0 [(M+Me)⁺], 251.0 [(M+2+Me)⁺].

### Step 2. Synthesis of 4-bromo-2-chloro-5-(methoxymethoxy)benzaldehyde

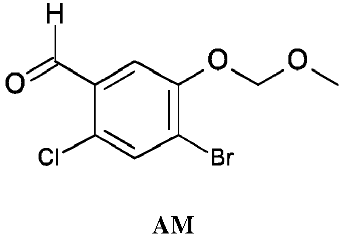

To a solution of 4-bromo-2-chloro-5-hydroxy-benzaldehyde (300.0 mg, **AG-1**) in THF (3 mL) was added sodium hydride (76 mg) and stirred for 20 min, followed by addition of bromomethyl methyl ether (238 mg). After being stirred for 0.5 h, the reaction mixture was quenched with water and concentrated *in vacuo.* The residue was purified by flash chromatography (eluting with EtOAc/PE = 1/10) to give 4-bromo-2-chloro-5-(methoxymethoxy)benzaldehyde (325 mg, Intermediate **AM**) as colorless oil.

### Intermediate AN

### 3-[2-Bromo-5-formyl-4-(trifluoromethoxy)phenoxy]propanoic acid

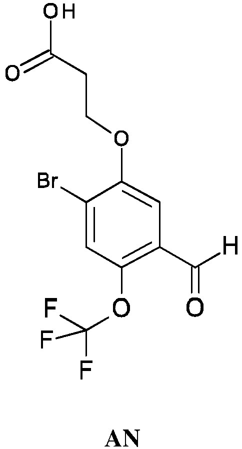

### Step 1. Synthesis of 2-bromo-4-methoxy-1-(trifluoromethoxy)benzene

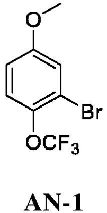

To a solution of 3-bromo-4-(trifluoromethoxy)phenol (4.0 g) in acetone (20 mL) were added potassium carbonate (8.6 g) and iodomethane (6.6 g) at r.t and then heated at 80 °C for 4 hrs. The mixture was quenched with water and extracted with EtOAc (50 mL) three times. The combined organic layer was concentrated and then purified by flash chromatography (eluting with EtOAc/PE = 1/10) to give 2-bromo-4-methoxy-1-(trifluoromethoxy)benzene (3.5 g, compound **AN-1**) as a colorless oil.

### Step 2. Synthesis of 5-methoxy-2-(trifluoromethoxy)benzaldehyde

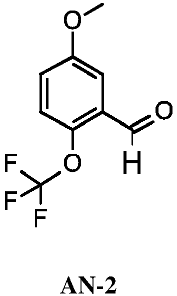

To a solution of 2-bromo-4-methoxy-1-(trifluoromethoxy)benzene (2.0 g, compound **AN-1**) in THF (33 mL) was added dropwise butyllithium solution (0.66 g) at -60 °C. The mixture was added dimethylformamide (1.71 mL). After being warmed to r.t for 1 h, the reaction mixture was quenched by NH₄Cl (2 mL), diluted with water and extracted with EtOAc (80 mL) three times. The combined organic layer was washed by brine and dried over Na₂SO₄, concentrated and purified by flash chromatography (eluting with EtOAc/PE = 1/10) to give **5**-methoxy-2-(trifluoromethoxy)benzaldehyde (1.4 g, compound **AN-2**) as a colorless oil.

### Step 3. Synthesis of 4-bromo-5-methoxy-2-(trifluoromethoxy)benzaldehyde

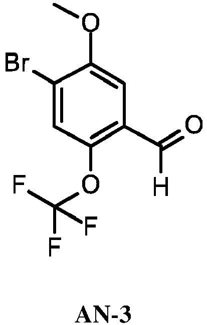

To a solution of 5-methoxy-2-(trifluoromethoxy)benzaldehyde (3.8 g, compound **AN-2**) in ACN (100 mL) was added N-bromosuccinimide (3.1 g) and benzoyl peroxide (0.21 g) at r.t. After being heated at 90 °C for 2 hrs, the reaction mixture was cooled with ice-water, poured into water and extracted with EtOAc (120 mL) three times. The organic phase was washed with water, dried over Na₂SO₄, concentrated. The residue was purified by flash chromatography (eluting with EtOAc/PE = 1/10) to give 4-bromo-5-methoxy-2-(trifluoromethoxy)benzaldehyde (2.6 g, compound **AN-3**) as a light yellow solid. MS obsd. (ESI⁺): 299.0 [(M+H)⁺], 301.0 [(M+2+H)⁺].

### Step 4. Synthesis of 4-bromo-5-hydroxy-2-(trifluoromethoxy)benzaldehyde

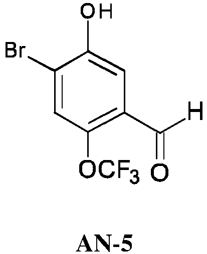

To a solution of 4-bromo-5-methoxy-2-(trifluoromethoxy)benzaldehyde (400.0 mg, compound **AN-4**) in DCM (8 mL) were added boron tribromide (335.1 mg) at -65 °C for 0.5 h and warmed up to r.t for 3 hrs. The mixture dropwise added to ice water and extracted with DCM (20 mL) three times. The combined organic layer was dried over Na₂SO₄, concentrated *in vacuo.* The residue was purified by flash chromatography (eluting with EtOAc/PE = 1/10) to give 4-bromo-5-hydroxy-2-(trifluoromethoxy)benzaldehyde (300 mg, **AN-5**) as a brown oil. MS obsd. (ESI⁺): 299.0 [(M+14+H)⁺], 301.0 [(M+14+2+H)⁺].

### Step 5. Synthesis of 3-[2-bromo-5-formyl-4-(trifluoromethoxy)phenoxy]propanoic acid

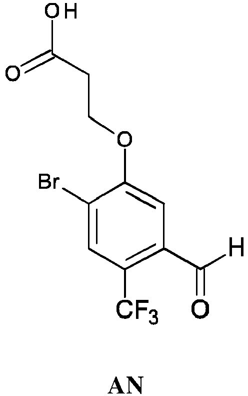

To a solution of 4-bromo-5-hydroxy-2-(trifluoromethoxy)benzaldehyde (2.6 g, compound **AN-5**) in NaOH (9.1 mL, 1 *M*) were added 3-bromopropionic acid (1.4 g) with NaOH (9.1 mL, 1*M*). The resulting mixture was stirred at 110 °C for 16 hrs. The reaction mixture was acidified to pH = 6 with 1 *M* HCl, extracted with EtOAc (80 mL) three times. The combined organic layer was washed by brine, dried over anhydrous sodium sulfate, concentrated *in vacuo.* The residue was purified by flash column chromatography (eluting with EtOAc/PE = 1:8~1:1) to give 3-[2-bromo-5-formyl-4-(trifluoromethoxy)phenoxy]propanoic acid (1.0 g, Intermediate **AN**) as a light brown solid. MS obsd. (ESI⁺): 357.0 [(M+H)⁺], 359.0 [(M+2+H)⁺].

### Intermediate AO

### Methyl 3-[2-(p-tolylsulfonyloxy)ethoxy]cyclobutanecarboxylate

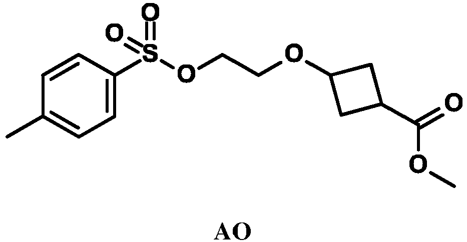

### Step 1: Preparation of 2-benzyloxyethoxy(trimethyl)silane

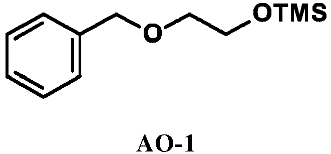

To a solution of 2-benzyloxyethanol (20.0 g, 131.4 mmol) and TEA (20.0 g, 197.1 mmol) in dichloromethane (200 mL) cooled at 0 °C was added trimethylsilyl chloride (17.1 g, 157.7 mmol) and the mixture was then stirred at 25 °C for 16 hrs. After the reaction was completed, the mixture was concentrated *in vacuo* and the residue was purified by column chromatography on silica gel (eluting with PE:EtOAc=50:1 to 10:1) to give the 2-benzyloxyethoxy(trimethyl)silane (25.0 g, 84.9%, compound **AO-1**) as a colorless oil.

### Step 2: Preparation of methyl 3-(2-benzyloxyethoxy)cyclobutanecarboxylate

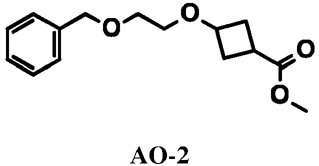

To a solution of 2-benzyloxyethoxy(trimethyl)silane (25.0 g, 111.4 mmol) and methyl 3-oxocyclobutanecarboxylate (CAS #: 4934-99-0, Cat.#: PB01390, from PharmaBlock (NanJing) R&D Co. Ltd,15.0 g, 117.0 mmol) in dichloromethane (200 mL) was added trimethylsilyl trifluoromethanesulfonate (12.4 g, 55.7 mmol) dropwise at -78 °C. After addition, the mixture was stirred at -78 °C for additional 1 hr, then to the resulting mixture was added triethylsilane (14.25 g, 122.57 mmol). After addition, the resulting mixture was warmed to room temperature and stirred for additional 1 hr. After the reaction was completed, the mixture was washed with saturated NH₄Cl solution, brine, dried over anhydrous sodium sulfate, and concentrated *in vacuo.* The residue was purified by column chromatography on silica gel (eluting with PE/EtOAc=100: 1-50: 1) to give methyl 3-(2-benzyloxyethoxy)cyclobutanecarboxylate (28 g, 95.1%, compound **AO-2**) as a colorless oil. MS obsd. (ESI⁺) [(M+H)⁺]: 265.1.

### Step 3: Preparation of methyl 3-(2-hydroxyethoxy)cyclobutanecarboxylate

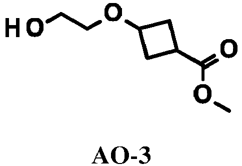

To a solution of methyl 3-(2-benzyloxyethoxy)cyclobutanecarboxylate (28.0 g, 105.9 mmol, compound **AO-2**) in MeOH (300.0 mL) was added Pd(OH)₂(wet) (1.48 g, 10.6 mmol) at room temperature and the mixture was then hydrogenated under H₂ atmosphere at room temperature overnight. After the reaction was completed, the reaction was filtered through silica gel pad and the filtrate was concentrated *in vacuo* to give 18 g crude methyl 3-(2-hydroxyethoxy)cyclobutanecarboxylate (18 g, 97.6%, compound **AO-3**) as a colorless oil.

### Step 4: Preparation of methyl 3-[2-(p-tolylsulfonyloxy)ethoxy]cyclobutanecarboxylate

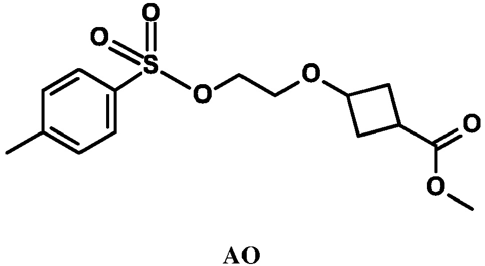

To a solution of methyl 3-(2-hydroxyethoxy)cyclobutanecarboxylate (5 g, 28.7 mmol) and DMAP (5.26 g, 43.1 mmol) in dichloromethane (80 mL) was added 4-methylbenzene-1-sulfonyl chloride (6.02 g, 31.6 mmol) at room temperature and the mixture was then stirred at room temperature overnight. After the reaction was completed, the mixture was washed with 1*N* HCl (25 mL), water (15mL), saturated NaHCO₃ solution, brine and concentrated *in vacuo* to give the crude methyl 3-[2-(*p*-tolylsulfonyloxy)ethoxy]cyclobutanecarboxylate (8.1 g, 85.6%, Intermediate **AO**) as a colorless oil, which was used in the next step directly without further purification. MS obsd. (ESI⁺) [(M+H)⁺]: 329.2.

### Intermediate AP

### Cis-tert-butyl 3-[2-(p-tolylsulfonyloxy)ethoxy]cyclobutanecarboxylate

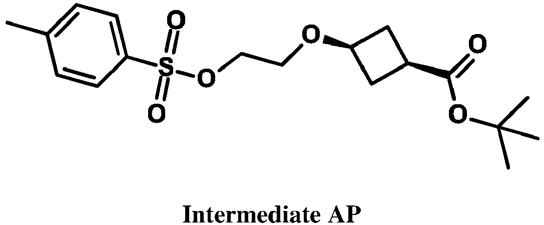

### Step 1: preparation of cis-tert-butyl 3-(2-benzyloxyethoxy)cyclobutanecarboxylate

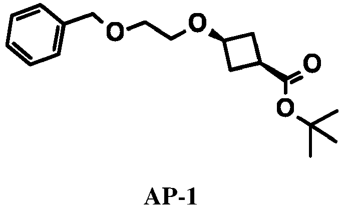

To a solution of trifluoromethanesulfonic anhydride (27.8 g, 98.56 mmol) and 2,6-lutidine (11.48 mL, 98.56 mmol) in DCM (100 mL) cooled at -30 °C was added 2-(benzyloxy)ethanol (10.0 g, 65.71 mmol) and the reaction mixture was stirred at -30 °C for 1 hr. The reaction mixture was washed with brine (30ml) twice and the organic layer was concentrated *in vacuo* to give the crude 2-(benzyloxy)ethyltrifluoromethanesulfonate (18.7 g) as a yellow oil. To a solution of *cis-tert*-butyl 3-hydroxycyclobutanecarboxylate (CAS #: 939768-64-6, Cat.#: B253665, from BePharm Ltd., 11.3g, 65.71 mmol) in THF (150 mL) cooled at 0 °C was added NaH (3.95g, 98.56 mmol) and the mixture was stirred at room temperature for 1 hr. To the resulting solution was added 2-(benzyloxy)ethyltrifluoromethanesulfonate (18.7 g, previously prepared) and the mixture was stirred at room temperature for 2 hrs. The reaction was then quenched with ice water (100 mL) and extracted with EtOAc (200 mL) twice. The combined organic layer was dried over Na₂SO₄ and concentrated *in vacuo.* The residue was purified by column chromatography on silica gel (eluting with PE:EtOAc 100:1 to 2:1) to give *cis*-*tert*-butyl 3-(2-benzyloxyethoxy)cyclobutanecarboxylate (10.0 g, 49.67% yield) as a yellow oil. ¹H NMR (CDCl₃, 400MHz): δ ppm 1.44 (s, 9H), 2.17 (m, 2H), 2.54-2.42 (m, 3H), 3.55-3.50 (m, 2H), 3.62-3.57 (m, 2H), 3.99-3.83 (m, 1H), 4.57 (s, 2H), 7.30-7.27 (m, 1H), 7.34 (d, *J* = 4.3 Hz, 4H). MS obsd. (ESI⁺) [(M+Na)⁺]: 329.1.

### Step 2: preparation of cis-tert-butyl 3-[2-(p-tolylsulfonyloxy)ethoxy]cyclobutanecarboxylate

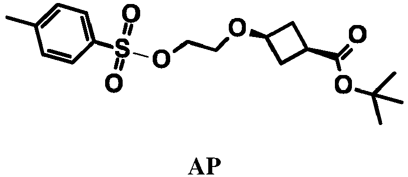

**Intermediate AP** was prepared in analogy to the procedure described for the preparation of compound **AN** by using *cis*-*tert*-butyl 3-(2-benzyloxyethoxy)cyclobutanecarboxylate as the starting material instead of methyl 3-(2-benzyloxyethoxy)cyclobutanecarboxylate in **Step 3**. MS obsd. (ESI⁺) [(M+H)⁺]: 371.2.

### Intermediate AQ

### Tert-butyl 2- [3-(p-tolylsulfonyloxy)propoxy]acetate

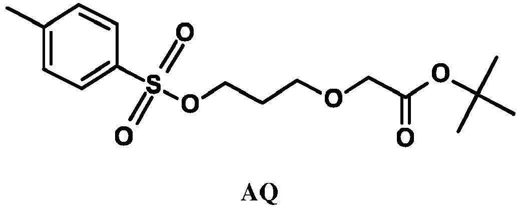

### Step 1: Preparation of tert-butyl 2-(3-(benzyloxy)propoxy)acetate

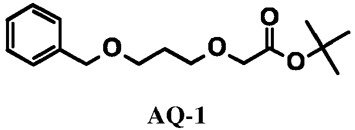

To a mixture of NaOH (10M, 300.0 mL), *tert*-butyl 2-bromoacetate (23.5 g, 120.3 mmol) and tetrabutylammonium iodide (8.8 g, 24.06 mmol) in DCM (300 mL) was added 3-benzyloxypropan-1-ol (12.99 mL, 120.32 mmol) at 30 °C and the mixture was stirred at 30 °C for 72 hrs. After the reaction was completed, the organic phase was separated out and the aquatic phase was extracted with DCM (150 mL) twice. The combined organic layer was washed with brine, dried over MgSO₄ and concentrated *in vacuo.* The residue was purified by column chromatography on silica gel (eluting with PE:EtOAc = 3:1) to give *tert*-butyl 2-(3-(benzyloxy)propoxy)acetate (21.3g, 63.3% yield, compound **AQ-1**) as a colorless liquid. MS obsd. (ESI⁺) [(M+Na)⁺]: 303.2.

### Step 2: Preparation of tert-butyl 2-[3-(p-tolylsulfonyloxy)propoxy]acetate

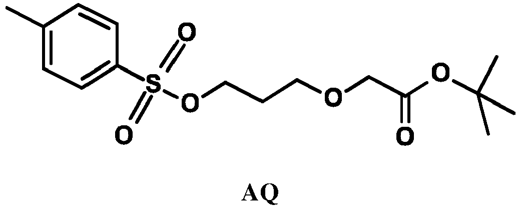

**AQ** was prepared in analogy to the procedure described for the preparation of compound **AN** by using *tert*-butyl 2-(3-(benzyloxy)propoxy)acetate as the starting material instead of methyl 3-(2-benzyloxyethoxy)cyclobutanecarboxylate in **Step 3**. MS obsd. (ESI⁺) [(M+H)⁺]: 345.0.

### Example 1

### Cis-3-[2-[2-bromo-5-(8-chloro-4-oxo-chromen-2-yl)-4-methoxy-phenoxy]ethoxy]cyclobutanecarboxylic acid

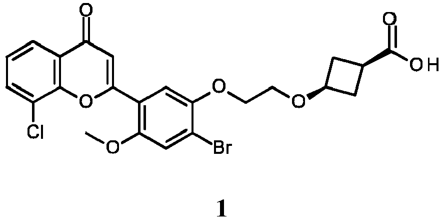

### Step 1: Synthesis of (E)-3-[4-bromo-2-methoxy-5-[(4-methoxyphenyl)methoxy]phenyl]-1-(3-chloro-2-hydroxy-phenyl)prop-2-en-1-one

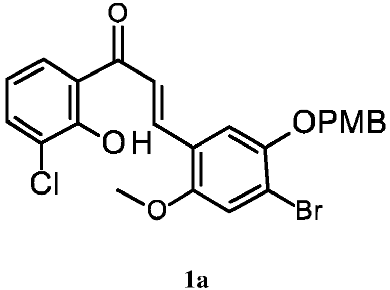

The mixture of 1-(3-chloro-2-hydroxy-phenyl)ethanone (0.39 g, 2.28 mmol), potassium hydroxide (0.51 g, 9.13 mmol) and 4-bromo-2-methoxy-5-[(4-methoxyphenyl)methoxy]benzaldehyde (0.8 g, Intermediate **AA**) in ethanol (40 mL) was stirred at 60 °C for 16 hrs. The reaction mixture was cooled to 0 °C and acidified to pH = 3 with 4 *M* HCl solution. The precipitate was collected by filtration, washed with H₂O (50 mL), PE/EtOAc = 50/1 (50 mL), dried *in vacuo* to give (*E*)-3-[4-bromo-2-methoxy-5-[(4-methoxyphenyl)methoxy]phenyl]-1-(3-chloro-2-hydroxy-phenyl)prop-2-en-1-one (800 mg, compound **1a**) as a yellow solid which was used for next step without further purification.

### Step 2. Synthesis of 2-[4-bromo-2-methoxy-5-[(4-methoxyphenyl)methoxy]phenyl]-8-chloro-chromen-4-one

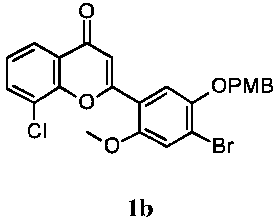

To a solution of (*E*)-3-[4-bromo-2-methoxy-5-[(4-methoxyphenyl)methoxy]phenyl]-1-(3-chloro-2-hydroxy-phenyl)prop-2-en-1-one (0.8 g, compound **1a**) in DMSO (30 mL) was added iodine (48.37 mg). After being stirred at 140 °C for 4 hrs, the reaction mixture was cooled to 25 °C and poured into a mixture of Na₂SO₃ (18 mL) and ice/water (100 g). The precipitate was collected by filtration, washed with water (100 mL), dried *in vacuo* to give a crude yellow solid, which was purified by recrystallization with methanol/EtOAc (V/V) = 1/40 (5 mL) to afford 2-[4-bromo-2-methoxy-5-[(4-methoxyphenyl)methoxy]phenyl]-8-chloro-chromen-4-one (900 mg, 90% yield, compound **1b**) as a yellow solid.

### Step 3. Synthesis of 2-(4-bromo-5-hydroxy-2-methoxy-phenyl)-8-chloro-chromen-4-one

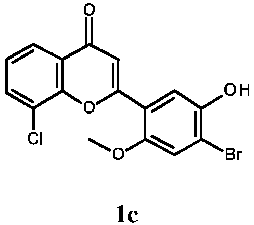

To a solution of trifluoroacetic acid (7.5 mL) at 0 °C was added 2-[4-bromo-2-methoxy-5-[(4-methoxyphenyl)methoxy]phenyl]-8-chloro-chromen-4-one (425.0 mg, compound **1b**). The reaction mixture was degassed with N₂ for 3 times. After being stirred at 0 °C for 4 hrs, the reaction mixture was concentrated *in vacuo.* The residue was purified by recrystallization with PE/EtOAc(V/V) = 1/10 (30 mL) to afford 2-(4-bromo-5-hydroxy-2-methoxy-phenyl)-8-chloro-chromen-4-one (270 mg, 83% yield, compound **1c**) as a brown solid. MS obsd. (ESI⁺): 381.0 [(M+H)⁺], 383.0 [(M+2+H)⁺].

### Step 4: Synthesis of tert-butyl 3-[2-[2-bromo-5-(8-chloro-4-oxo-chromen-2-yl)-4-methoxy-phenoxy]ethoxy]cyclobutanecarboxylate

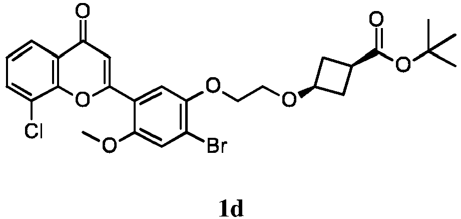

To a solution of 2-(4-bromo-5-hydroxy-2-methoxy-phenyl)-8-chloro-chromen-4-one (100.0 mg, 0.260 mmol, compound **1c**) and *cis*-*tert*-butyl 3-[2-(*p-*tolylsulfonyloxy)ethoxy]cyclobutanecarboxylate (0.45 mL, Intermediate **AP**) in dry DMF (10 mL) was added potassium carbonate (72.44 mg). The reaction mixture was degassed with N₂ for 3 times. After being stirred at 100 °C for 16 hrs, the reaction mixture was concentrated *in vacuo.* The residue was diluted with EtOAc (50 mL), washed with brine (20 mL), dried over anhydrous sodium sulfate, filtered and concentrated *in vacuo.* The crude product was purified by flash silica gel column chromatography (eluting with EtOAc/PE = 1/4) to give *tert*-butyl 3-[2-[2-bromo-5-(8-chloro-4-*oxo*-chromen-2-yl)-4-methoxy-phenoxy]ethoxy]cyclobutanecarboxylate (180 mg, 85% yield, compound **1d**) as a yellow solid. MS obsd. (ESI⁺): 579.1 [(M+H)⁺], 581.1 [(M+2+H)⁺].

### Step 5: Synthesis of cis-3-[2-[2-bromo-5-(8-chloro-4-oxo-chromen-2-yl)-4-methoxy-phenoxy]ethoxy]-cyclobutanecarboxylic acid

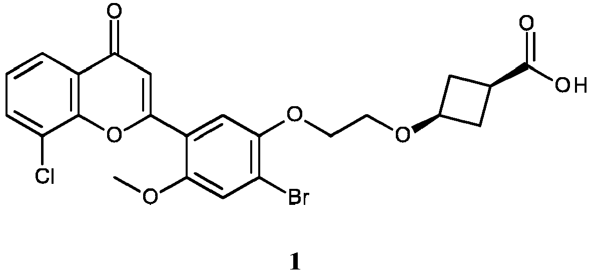

To a solution of *cis-tert-*butyl 3-[2-[2-bromo-5-(8-chloro-4-*oxo*-chromen-2-yl)-4-methoxy-phenoxy]ethoxy]cyclobutanecarboxylate (180.0 mg, compound **1d**) in dry DCM (18 mL) was added trifluoroacetic acid (0.81 mL). The reaction mixture was degassed with N₂ for 3 times. After being stirred at 25 °C for 16 hrs, the reaction mixture was concentrated *in vacuo.* The residue was purified by re-crystallization with PE/EtOAc (V/V) = 1/1 (30 mL) to give cis-3-[2-[2-bromo-5-(8-chloro-4-*oxo*-chromen-2-yl)-4-methoxy-phenoxy]ethoxy]cyclobutanecarboxylic acid (50 mg, **Example 1**) as a yellow solid. ¹H NMR (400 MHz, MeOD) *δ* ppm 8.04 (dd, *J* = 8.0, 1.5 Hz, 1H), 7.89 (dd, *J* = 7.8, 1.5 Hz, 1H), 7.74 (s, 1H), 7.40-7.48 (m, 2H), 7.27 (s, 1H), 4.17-4.24 (m, 2H), 4.04-4.12 (m, 1H), 3.96 (s, 3H), 3.77-3.83 (m, 2H), 2.60-2.68 (m, 1H), 2.50-2.58 (m, 2H), 2.13-2.2 (m, 2H). MS obsd. (ESI⁺): 523.0 [(M+H)⁺], 525.0 [(M+2+H)⁺].

### Example 2

### 3-[2-Bromo-5-(8-chloro-4-oxo-chromen-2-yl)-4-methoxy-phenoxy]propanoic acid

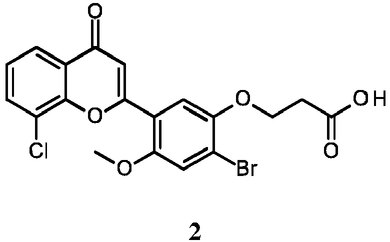

To a solution of 2-(4-bromo-5-hydroxy-2-methoxy-phenyl)-8-chloro-chromen-4-one (100.0 mg, compound **1c**) and beta-propiolactone (22.66 mg) in dry DMF (2 mL) was added, sodium hydride (7.55 mg) under N₂. After being stirred at 50 °C for 16 hrs, the reaction mixture was cooled to room temperature, and acidified to pH = 6 with 2 *M* HCl at 0 °C. The reaction mixture was concentrated *in vacuo.* The crude product was purified by *prep-HPLC* (eluting with ACN in water from 0% to 40%, 0.1% FA in water) to give 3-[2-bromo-5-(8-chloro-4-oxo-chromen-2-yl)-4-methoxy-phenoxy]propanoic acid (12 mg, **Example 2**) as a yellow solid. ¹H NMR (400 MHz, DMSO-*d₆*) δ ppm 7.95-8.03 (m, 2H), 7.68 (s, 1H), 7.45-7.49 (d, *J* = 8.2 Hz, 2H), 7.08 (s, 1H), 4.26 (t, *J* = 6.6 Hz, 2H), 3.93 (s, 3H), 2.58 (t, *J* = 6.6 Hz, 2H). MS obsd. (ESI⁺): 453.0 [(M+H)⁺], 455.0 [(M+2+H)⁺].

### Example 3

### 3-[2-Bromo-5-(8-chloro-4-oxo-chromen-2-yl)-4-methoxy-phenoxy]-N-cyclopropylsulfonyl-propanamide

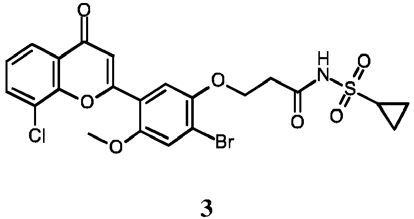

### Step 1. Synthesis of methyl 3-[2-bromo-5-(8-chloro-4-oxo-chromen-2-yl)-4-methoxy-phenoxy]propanoate

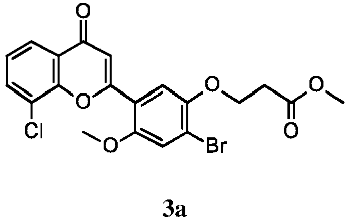

To a solution of 3-[2-bromo-5-(8-chloro-4-*oxo*-chromen-2-yl)-4-methoxy-phenoxy]propanoic acid (300.0 mg, example **2**) in methanol (100 mL) at 0 °C was added thionyl chloride (236 mg) dropwise. After being stirred at 65 °C for 16 h the reaction mixture was concentrated *in vacuo.* The residue was diluted with water (50 mL), extracted with EtOAc (50 mL) three times. The combined organics were washed with brine (30 mL) two times, dried over anhydrous Na₂SO₄, filtered and concentrated *in vacuo.* The resulting oil was purified by flash silica gel column chromatography (eluting with EtOAc/PE =3/7) to give methyl 3-[2-bromo-5-(8-chloro-4-oxo-chromen-2-yl)-4-methoxy-phenoxy]propanoate (250 mg, 80% yield, compound **3a**) as a yellow solid. MS obsd. (ESI⁺): 467.0 [(M+H)⁺], 469.0 [(M+2+H)⁺].

### Step 2. Synthesis of 3-[2-bromo-5-(8-chloro-4-oxo-chromen-2-yl)-4-methoxy-phenoxy]-N-cyclopropylsulfonyl-propanamide

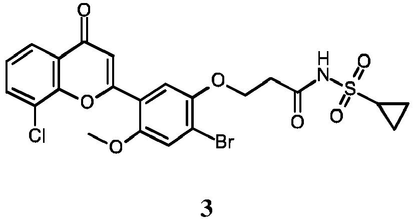

To a solution of methyl 3-[2-bromo-5-(8-chloro-4-*oxo*-chromen-2-yl)-4-methoxy-phenoxy]propanoate (98.0 mg, 0.210 mmol, compound **3a**) in 1,2-dichloroethane (10 mL) was added cyclopropanesulfonamide (0.05 g, 0.42 mmol) and titanium tetrachloride (0.12 g, 0.630 mmol) and stirred at 110 °C for 16 hrs. The reaction mixture was poured into ice/water (20 mL), extracted with DCM (15 mL) three times. The combined organic layer was dried over anhydrous sodium sulfate, filtered and concentrated *in vacuo.* The residue was purified by prep-HPLC (eluting with ACN in water 0% to 40%, 0.1% FA in water) to give 3-[2-bromo-5-(8-chloro-4-oxo-chromen-2-yl)-4-methoxy-phenoxy]-*N*-cyclopropylsulfonyl-propanamide (5 mg, 4.3% yield, Example **3**) as a yellow solid. ¹H NMR (400 MHz, DMSO-*d₆*) δ ppm 8.31 (s, H), 8.00 (t, *J* = 8.1 Hz, 2H), 7.71 (s, 1H), 7.48-7.53 (m, 2H), 7.10 (s, 1H), 4.27 (t, *J* = 6.9 Hz, 2H), 3.94 (s, 3H), 2.69-2.82 (m, 2H), 2.50-2.55 (m, 1H), 0.63-0.83 (m, 4H). MS obsd. (ESI⁺): 556.0 [(M+H)⁺], 558.0 [(M+2+H)⁺].

### Example 4

### 2-[2-Bromo-5-(8-chloro-4-oxo-chromen-2-yl)-4-methoxy-phenoxy]acetic acid

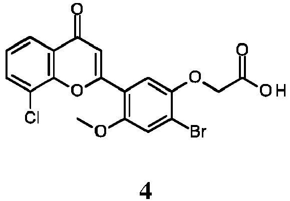

### Step 1. Synthesis of tert-butyl 2-[2-bromo-5-(8-chloro-4-oxo-chromen-2-yl)-4-methoxy-phenoxy]acetate

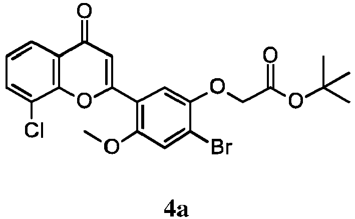

Compound **4a** was prepared in analogy to the procedure described for the preparation of example **1** by using *tert*-butyl bromoacetate as the starting material instead of *cis*-*tert*-butyl 3-[2-(*p*-tolylsulfonyloxy)ethoxy]cyclobutanecarboxylate (Intermediate **AO**) in **Step 4**. *Tert-*butyl *2-*[2-bromo-5-(8-chloro-4-*oxo*-chromen-2-yl)-4-methoxy-phenoxy]acetate (70 mg, compound **4a**) was obtained as a yellow solid. MS obsd. (ESI⁺): 495.0 [(M+H)⁺], 497.0 [(M+2+H)⁺].

### Step 2: Synthesis of 2-[2-bromo-5-(8-chloro-4-oxo-chromen-2-yl)-4-methoxy-phenoxy]acetic acid

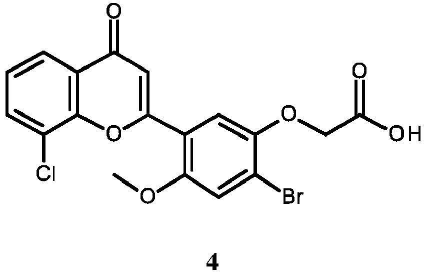

Example **4** was prepared in analogy to the procedure described for the preparation of example **1** by using *tert*-butyl 2-[2-bromo-5-(8-chloro-4-*oxo*-chromen-2-yl)-4-methoxy-phenoxy]acetate ( compound *4a*) as the starting material instead of tert-butyl 3-[2-[2-bromo-5-(8-chloro-4-oxo-chromen-2-yl)-4-methoxy-phenoxy]ethoxy]cyclobutanecarboxylate (compound **1d**) in **Step 5**. 2-[2-Bromo-5-(8-chloro-4-oxo-chromen-2-yl)-4-methoxy-phenoxy]acetic acid (40 mg, 64% yield, Example **4**) was obtained as a yellow solid. ¹H NMR (400 MHz, DMSO-*d₆*) δ ppm 13.15 (br s, 1H), 7.99 (t, *J* = 8.0 Hz, 2H), 7.56 (s, 2H), 7.50 (t, *J* = 7.9 Hz, 1H), 7.11 (s, 1H), 4.85 (s, 2H), 3.96 (s, 3H). MS obsd. (ESI⁺): 439.0 [(M+H)⁺], 441.0 [(M+2+H)⁺].

### Example 5

### 3-[2-Bromo-5-(8-chloro-4-oxo-chromen-2-yl)-4-methoxy-phenoxy]cyclobutanecarboxylic acid

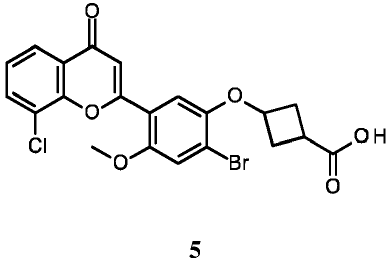

### Step 1: Synthesis of tert-butyl 3-[2-bromo-5-(8-chloro-4-oxo-chromen-2-yl)-4-methoxy-phenoxy]cyclobutanecarboxylate

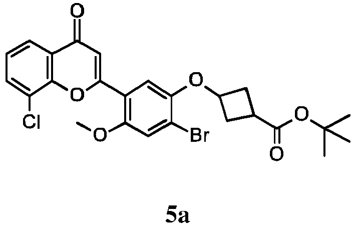

Compound **5a** was prepared in analogy to the procedure described for the preparation of example **1** by using as tert-butyl 3-(*p*-tolylsulfonyloxy)cyclobutanecarboxylate the starting material instead of *cis-tert-*butyl 3-[2-(*p*-tolylsulfonyloxy)ethoxy]cyclobutanecarboxylate (Intermediate **AO**) in **Step 4.** *Tert*-butyl 3-[2-bromo-5-(8-chloro-4-oxo-chromen-2-yl)-4-methoxy-phenoxy]cyclobutanecarboxylate (80 mg,74% yield, compound **5a**) was obtained as a yellow solid. MS obsd. (ESI⁺): 535.0 [(M+H)⁺], 537.1 [(M+2+H)⁺],

### Step 2. Synthesis of 3-[2-bromo-5-(8-chloro-4-oxo-chromen-2-yl)-4-methoxy-phenoxy]cyclobutanecarboxylic acid

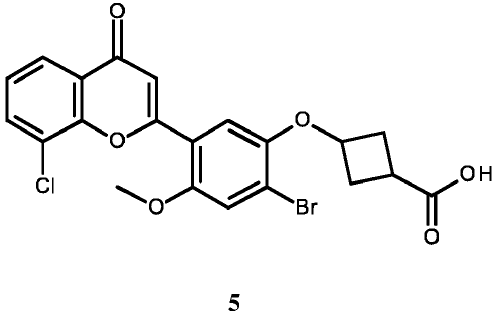

Example **5** was prepared in analogy to the procedure described for the preparation of example **1** by using *tert*-butyl 3-[2-bromo-5-(8-chloro-4-*oxo*-chromen-2-yl)-4-methoxy-phenoxy]cyclobutanecarboxylate (compound **5a**) as the starting material instead of *tert*-butyl 3-[2-[2-bromo-5-(8-chloro-4-*oxo*-chromen-2-yl)-4-methoxy-phenoxy]ethoxy]cyclobutanecarboxylate (compound **1d)** in **Step 5.** 3-[2-Bromo-5-(8-chloro-4-*oxo*-chromen-2-yl)-4-methoxy-phenoxy]cyclobutanecarboxylic acid (23 mg, 35% yield, Example **5**) was obtained as a yellow solid. ¹H NMR (400 MHz, DMSO-*d₆*) δ ppm 12.35 (br s, 1H), 7.97-8.03 (m, 2H), 7.54 (s, 2H), 7.49 (t, *J* = 7.9 Hz, 1H), 7.17 (s, 1H), 4.91-4.98 (m, 1H), 3.95 (s, 3H), 3.063.13 (m, 1H), 2.67-2.76 (m, 2H), 2.33-2.46 (m, 2H). MS obsd. (ESI⁺): 479.0 [(M+H)⁺], 481.0 [(M+2+H)⁺].

### Example 6

### 2-[3-[2-Bromo-5-(8-chloro-4-oxo-chromen-2-yl)-4-methoxy-phenoxy]propoxy]acetic acid

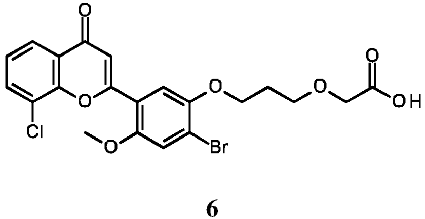

### Step 1. Synthesis of tert-butyl 2-[3-[2-bromo-5-(8-chloro-4-oxo-chromen-2-yl)-4-methoxy-phenoxy]propoxy]acetate

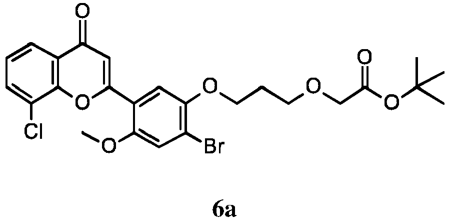

Compound **6a** was prepared in analogy to the procedure described for the preparation of example **1** by using as *tert*-butyl2-[3-(*p*-tolylsulfonyloxy)propoxy]acetate (Intermediate **AQ**) the starting material instead of *cis-tert*-butyl 3-[2-(*p-*tolylsulfonyloxy)ethoxy]cyclobutanecarboxylate (Intermediate **AP**) in **Step 4.** *Tert*-butyl 2-[3-[2-bromo-5-(8-chloro-4-*oxo*-chromen-2-yl)-4-methoxy-phenoxy]propoxy]acetate (180 mg, 59% yield, compound **6a**) was obtained as a yellow solid. MS obsd. (ESI⁺): 553.0 [(M+H)⁺], 555.1 [(M+2+H)⁺],

### Step 2. Synthesis of 2-[3-[2-bromo-5-(8-chloro-4-oxo-chromen-2-yl)-4-methoxy-phenoxy]propoxy]acetic acid

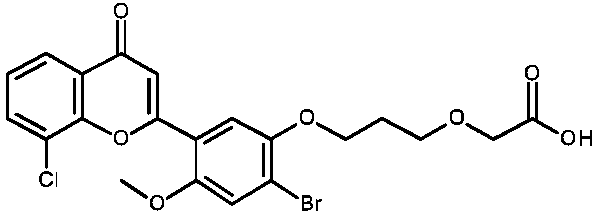

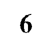

Example **6** was prepared in analogy to the procedure described for the preparation of example **1** by using *tert*-butyl 2-[3-[2-bromo-5-(8-chloro-4-*oxo*-chromen-2-yl)-4-methoxy-phenoxy]propoxy]acetate (compound **6a**) as the starting material instead of *tert*-butyl 3-[2-[2-bromo-5-(8-chloro-4-*oxo*-chromen-2-yl)-4-methoxy-phenoxy]ethoxy]cyclobutanecarboxylate (compound **1d)** in **Step 5.** 2-[3-[2-Bromo-5-(8-chloro-4-*oxo*-chromen-2-yl)-4-methoxy-phenoxy]propoxy]acetic acid (40 mg, 56% yield, Example **6**) was obtained as a white solid. ¹H NMR (400 MHz, DMSO-*d₆*) δ ppm 12.51 (s, 1H), 7.98-8.06 (m, 2H), 7.65 (d, *J* = 8.6 Hz, 1H), 7.58 (d, *J* = 8.6 Hz, 1H), 7.52 (t, *J* = 7.9 Hz, 1H), 6.93 (s, 1H), 4.11 (t, *J* = 6.4 Hz, 2H), 4.03 (s, 2H), 3.93 (s, 3H), 3.70 (t, *J* = 6.3 Hz, 2H), 2.05 (dd, *J* = 13.0, 6.6 Hz, 2H). MS obsd. (ESI⁺): 497.0 [(M+H)⁺], 499.0 [(M+2+H)⁺].

### Example 7

### 3-[2-Chloro-5-(8-chloro-4-oxo-chromen-2-yl)-4-methoxy-phenoxy]cyclobutanecarboxylic acid

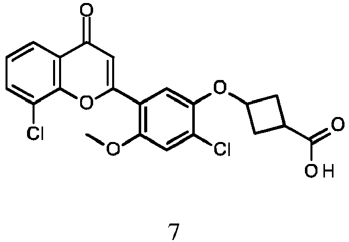

### Step 1. Synthesis of (E)-1-(3-chloro-2-hydroxy-phenyl)-3-[4-chloro-2-methoxy-5-(methoxymethoxy)phenyl]prop-2-en-1-one

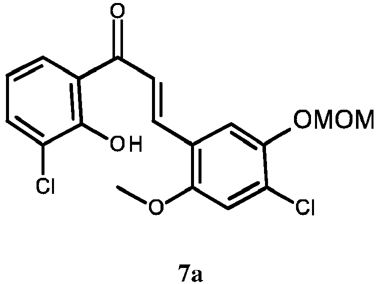

Compound **7a** was prepared in analogy to the procedure described for the preparation of example **1** by using 4-chloro-2-methoxy-5-(methoxymethoxy)benzaldehyde (Intermediate **AB**) as the starting material instead of 4-bromo-2-methoxy-5-[(4-methoxyphenyl)methoxy]benzaldehyde (Intermediate **AA**) in **Step 1.** (*E*)-1-(3-chloro-2-hydroxy-phenyl)-3-[4-chloro-2-methoxy-5-(methoxymethoxy)phenyl]prop-2-en-1-one (280 mg, 70% yield, compound **7a**) was obtained as a yellow solid which was used for next step without purification. MS obsd. (ESI⁺): 405.0 [(M+H)⁺], 407.0 [(M+2+H)⁺],

### Step 2. Synthesis of 8-chloro-2-(4-chloro-5-hydroxy-2-methoxy-phenyl)chromen-4-one

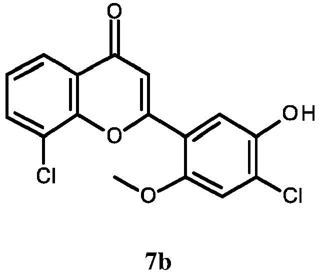

Compound **7b** was prepared in analogy to the procedure described for the preparation of example **1** by using (*E*)-1-(3-chloro-2-hydroxy-phenyl)-3-[4-chloro-2-methoxy-5-(methoxymethoxy)phenyl]prop-2-en-1-one (280 mg, compound **7a**) as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2.** 8-Chloro-2-(4-chloro-5-hydroxy-2-methoxy-phenyl)chromen-4-one (300 mg, 63% yield, compound **7b**) was obtained as a brown solid. MS obsd. (ESI⁺): 337.0 [(M+H)⁺], 339.0 [(M+2+H)⁺],

### Step 3. Synthesis of methyl 3-[2-chloro-5-(8-chloro-4-oxo-chromen-2-yl)-4-methoxy-phenoxy]cyclobutanecarboxylate (7c)

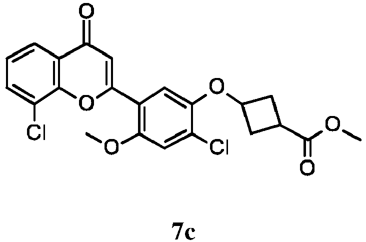

Compound **7c** was prepared in analogy to the procedure described for the preparation of example **1** by using 8-chloro-2-(4-chloro-5-hydroxy-2-methoxy-phenyl)chromen-4-one (compound **7b**) and methyl 3-(*p*-tolylsulfonyloxy)cyclobutanecarboxylate as the starting material instead of 2-(4-bromo-5-hydroxy-2-methoxy-phenyl)-8-chloro-chromen-4-one (compound **1c**) and *cis-tert*-butyl 3-[2-(*p*-tolylsulfonyloxy)ethoxy]cyclobutanecarboxylate (Intermediate **AP**) in **Step 4.** Methyl 3-[2-chloro-5-(8-chloro-4-*oxo*-chromen-2-yl)-4-methoxy-phenoxy]cyclobutanecarboxylate (35 mg, 33% yield, compound **7c**) was obtained as a white solid. MS obsd. (ESI⁺): 449.1 [(M+H)⁺], 451.1 [(M+2+H)⁺].

### Step 4. Synthesis of 3-[2-chloro-5-(8-chloro-4-oxo-chromen-2-yl)-4-methoxy-phenoxy]cyclobutanecarboxylic acid

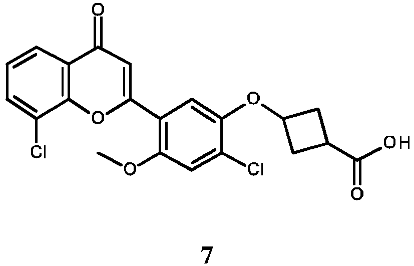

To a solution of methyl 3-[2-chloro-5-(8-chloro-4-*oxo*-chromen-2-yl)-4-methoxy-phenoxy]cyclobutanecarboxylate (35.0 mg, 0.080 mmol) in THF (10 mL) was added lithium hydroxide (0.01 mL, 0.780 mmol). The reaction mixture was degassed with N₂ for 3 times. After being stirred at 25 °C for 3 hrs, the reaction mixture was cooled to room temperature, and concentrated *in vacuo.* The residue was diluted with H₂O (5 mL), acidified to pH = 6 with 2 *M* HCl at 0 °C. The precipitate was collected, and washed with H₂O (5 mL), dried *in vacuo.* The crude product was purified by prep-HPLC (eluting with ACN in water 0% to 20%, 0.1% FA in water) to give 3-[2-chloro-5-(8-chloro-4-*oxo*-chromen-2-yl)-4-methoxy-phenoxy]cyclobutanecarboxylic acid (8 mg, 0.020 mmol, 23.59% yield, Example **7**) as an off-white solid. ¹H NMR (400 MHz, DMSO-*d₆*) δ ppm 12.32 (br s, 1H), 7.97-8.02 (m, 2H), 7.59 (s, 1H), 7.50 (t, *J* = 7.9 Hz, 1H), 7.43 (s, 1H), 7.17 (s, 1H), 4.92-4.99 (m, 1H), 3.95 (s, 1H), 3.10 (t, *J* = 10.1 Hz, 1H), 2.70-2.80 (m, 2H), 2.40-2.47 (m, 2H). MS obsd. (ESI⁺): 435.1 [(M+H)⁺], 437.1 [(M+2+H)⁺].

### Example 8

### Cis-3-[2-[2-chloro-5-(8-chloro-4-oxo-chromen-2-yl)-4-methoxy-phenoxy]ethoxy]cyclobutanecarboxylic acid

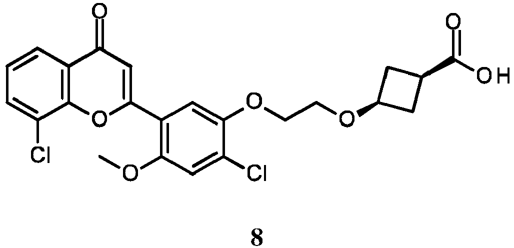

### Step 1. Synthesis of cis-tert-butyl 3-[2-[2-chloro-5-(8-chloro-4-oxo-chromen-2-yl)-4-methoxy-phenoxy]ethoxy]cyclobutanecarboxylate

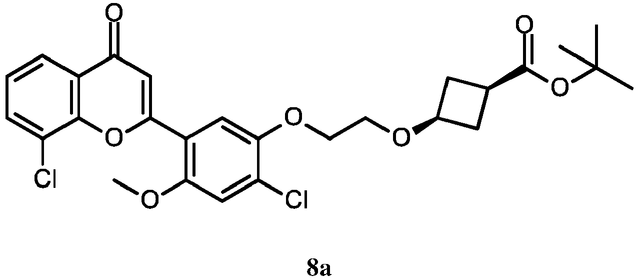

Compound **8a** was prepared in analogy to the procedure described for the preparation of example **1** by using 8-chloro-2-(4-chloro-5-hydroxy-2-methoxy-phenyl)chromen-4-one (compound **7b**) as the starting material instead of 2-(4-bromo-5-hydroxy-2-methoxy-phenyl)-8-chloro-chromen-4-one (compound **1c**) in **Step 4.** *Cis-tert*-butyl3-[2-[2-chloro-5-(8-chloro-4-oxo-chromen-2-yl)-4-methoxy-phenoxy]ethoxy]cyclobutanecarboxylate (40 mg, 62% yield, compound **8a**) was obtained as a white solid. MS obsd. (ESI⁺): 535.1 [(M+H)⁺], 537.2 [(M+2+H)⁺],

### Step 2. Synthesis of cis-3-[2-[2-chloro-5-(8-chloro-4-oxo-chromen-2-yl)-4-methoxy-phenoxy]ethoxy]cyclobutanecarboxylic acid

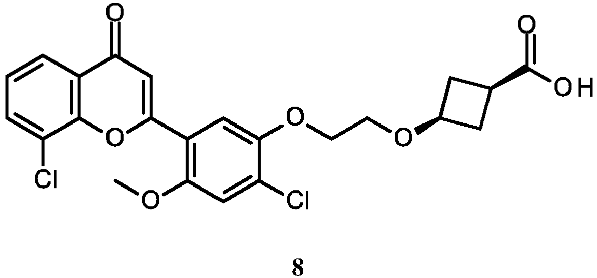

Example **8** was prepared in analogy to the procedure described for the preparation of example **1** by using *cis-tert*-Butyl 3-[2-[2-chloro-5-(8-chloro-4-*oxo*-chromen-2-yl)-4-methoxy-phenoxy]ethoxy]cyclobutanecarboxylate (compound **8a**) as the starting material instead of *tert-*butyl 3-[2-[2-bromo-5-(8-chloro-4-*oxo*-chromen-2-yl)-4-methoxy-phenoxy]ethoxy]cyclobutanecarboxylate (compound **1d)** in **Step 5.** Cis-3-[2-[2-chloro-5-(8-chloro-4-*oxo*-chromen-2-yl)-4-methoxy-phenoxy]ethoxy]cyclobutanecarboxylic acid (26 mg, 72% yield, example **8**) was obtained as a yellow solid. ¹H NMR (400 MHz, DMSO-*d*₆) δ ppm 7.89-7.97 (m, 2H), 7.62 (s, 1H) 7.42 (t, *J* = 7.9 Hz, 1H), 7.34 (s, 1H), 7.00 (s, 1H), 4.15-4.09 (m, 2H), 3.89-3.96 (m, 1H), 3.88 (s, 3H), 3.61-3.66 (m, 2H), 2.49-2.54 (m, 1H), 2.32-2.40 (m, 2H), 1.88-1.97 (m, 2H). MS obsd. (ESI⁺): 479.1 [(M+H)⁺], 481.1 [(M+2+H)⁺].

### Example 9

### 3-[2-[5-(8-Chloro-4-oxo-chromen-2-yl)-4-methoxy-2-methyl-phenoxy]ethoxy]cyclobutanecarboxylic acid

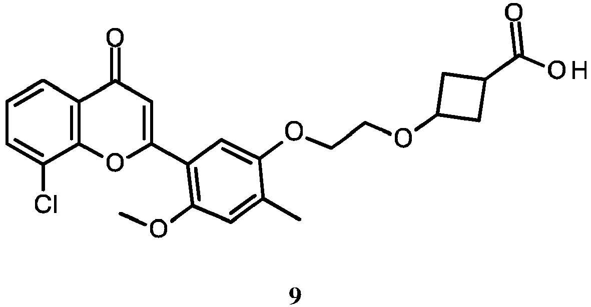

### Step 1. Synthesis of tert-butyl 3-[2-[2-bromo-5-(8-chloro-4-oxo-chromen-2-yl)-4-methoxy-phenoxy]ethoxy]cyclobutanecarboxylate

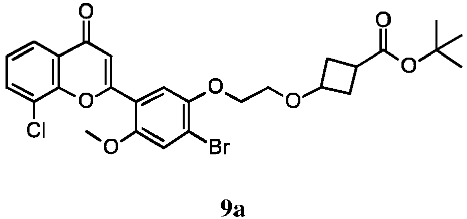

Compound **9a** was prepared in analogy to the procedure described for the preparation of example **1** by using *tert*-butyl 3-[2-(*p*-tolylsulfonyloxy)ethoxy]cyclobutanecarboxylate as the starting material instead of *cis*-*tert*-butyl 3-[2-(*p-*tolylsulfonyloxy)ethoxy]cyclobutanecarboxylate (Intermediate **AP**) in **Step 4.** *Tert*-butyl-3-[2-[2-bromo-5-(8-chloro-4-*oxo*-chromen-2-yl)-4-methoxy-phenoxy]ethoxy]cyclobutanecarboxylate (180 mg, 79% yield, compound **9a**) as a yellow solid. MS obsd. (ESI⁺): 579.1 [(M+H)⁺], 581.1 [(M+2+H)⁺],

### Step 2. Synthesis of tert-butyl 3-[2-[5-(8-chloro-4-oxo-chromen-2-yl)-4-methoxy-2-methyl-phenoxy]ethoxy]cyclobutanecarboxylate

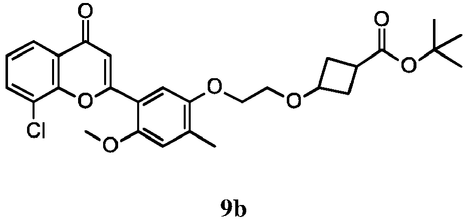

To a solution of *tert*-butyl 3-[2-[2-bromo-5-(8-chloro-4-*oxo*-chromen-2-yl)-4-methoxy-phenoxy]ethoxy]cyclobutanecarboxylate (180.0 mg, compound **9a**) in 1,4-dioxane (15 mL) were added cesium carbonate (202.28 mg) and [1,1'-bis(diphenylphosphino)ferrocene]dichloropalladium(II) (22 mg). After addition, the mixture was degassed with N₂ for 3 times. Then trimethylboroxine (39 mg) was added. After being stirred at 100 °C for 8 hrs, the reaction mixture was concentrated *in vacuo.* The residue was diluted with ethyl acetate (50 mL), washed with brine (20 mL), dried over anhydrous sodium sulfate, filtered and concentrated *in vacuo.* The residue was purified by flash silica gel chromatography (eluting with EtOAc/PE = 1/3) to give *tert*-butyl 3-[2-[5-(8-chloro-4-oxo-chromen-2-yl)-4-methoxy-2-methyl-phenoxy]ethoxy]cyclobutanecarboxylate (130 mg, 74% yield, compound **9b**) as a yellow solid. MS obsd. (ESI⁺): 515.2 [(M+H)⁺], 517.2 [(M+2+H)⁺],

### Step 3. Synthesis of 3-[2-[5-(8-chloro-4-oxo-chromen-2-yl)-4-methoxy-2-methyl-phenoxy]ethoxy]cyclobutanecarboxylic acid

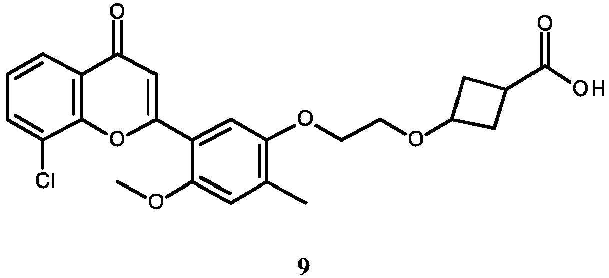

Example **9** was prepared in analogy to the procedure described for the preparation of example **1** by using *tert*-butyl 3-[2-[5-(8-chloro-4-*oxo*-chromen-2-yl)-4-methoxy-2-methyl-phenoxy]ethoxy]cyclobutanecarboxylate (compound **9a**) as the starting material instead of 2-(4-bromo-5-hydroxy-2-methoxy-phenyl)-8-chloro-chromen-4-one (compound **1c**) in **Step 4. 3-[2-**[5-(8-Chloro-4-oxo-chromen-2-y1)-4-methoxy-2-methyl-phenoxy]ethoxy]cyclobutanecarboxylic acid (50 mg, 47% yield, **example 9**) was obtained as a yellow solid. ¹H NMR (400 MHz, DMSO-*d₆*) δ ppm 12.16 (s, 1H), 7.95 - 8.03 (m, 2H), 7.53 (d, *J* = 2.8 Hz, 1H), 7.48 (td, *J* = 7.9, 2.1 Hz, 1H), 7.15 (d, *J* = 3.6 Hz, 1H), 7.09 (d, *J* = 1.2 Hz, 1H), 4.19-4.22 (m, 0.25H), 4.10-4.14 (m, 2H), 3.95-4.00 (m, 0.75H), 3.92 (s, 3H), 3.64-3.73 (m, 2H), 2.85-2.95 (m, 0.25 H), 2.55-2.63 (m, 0.75H), 2.38-2.48 (m, 2H), 2.28 (s, 3H), 2.15-2.20 (m, 0.5 H), 1.95- 2.04 (m, 1.5H). MS obsd. (ESI⁺): 459.1 [(M+H)⁺], 461.1 [(M+2+H)⁺].

### Example 10

### 3-[5-(8-Chloro-4-oxo-chromen-2-yl)-4-methoxy-2-methyl-phenoxy]propanoic acid

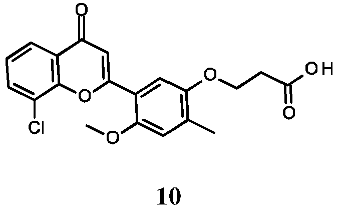

### Step 1. Synthesis of methyl 3-[5-(8-chloro-4-oxo-chromen-2-yl)-4-methoxy-2-methyl-phenoxy]propanoate

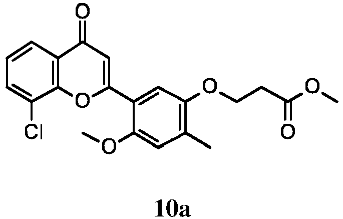

Compound **10a** was prepared in analogy to the procedure described for the preparation of **example 9** by using methyl 3-[2-bromo-5-(8-chloro-4-*oxo*-chromen-2-yl)-4-methoxy-phenoxy]propanoate (compound **3a**) as the starting material instead of *tert*-butyl 3-[2-[2-bromo-5-(8-chloro-4-*oxo*-chromen-2-yl)-4-methoxy-phenoxy]ethoxy]cyclobutanecarboxylate (compound **9a**) in **Step 2**. Methyl 3-[5-(8-chloro-4-*oxo*-chromen-2-yl)-4-methoxy-2-methyl-phenoxy]propanoate (80 mg, 79% yield, compound **10a**) was obtained as a yellow solid, which was used for next step without further purification. MS obsd. (ESI⁺): 403.0 [(M+H)⁺], 405.1 [(M+2+H)⁺],

### Step 2. Synthesis of 3-[5-(8-chloro-4-oxo-chromen-2-yl)-4-methoxy-2-methyl-phenoxy]propanoic acid

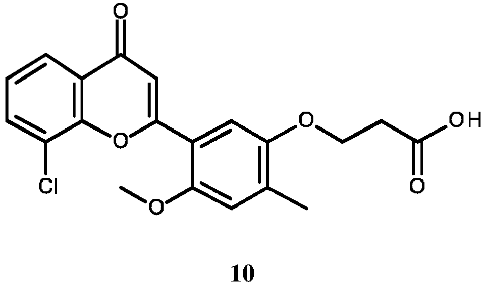

Example **10** was prepared in analogy to the procedure described for the preparation of example **7** by using methyl 3-[5-(8-chloro-4-*oxo*-chromen-2-yl)-4-methoxy-2-methyl-phenoxy]propanoate (80 mg, compound **10a**) as the starting material instead of methyl 3-[2-chloro-5-(8-chloro-4-*oxo*-chromen-2-yl)-4-methoxy-phenoxy]cyclobutanecarboxylate (compound **7c**) in **Step 4.** 3-[5-(8-Chloro-4-*oxo*-chromen-2-yl)-4-methoxy-2-methyl-phenoxy]propanoic acid (3.5 mg, 5.3% yield, **Example 10**) was obtained as a yellow solid. ¹H NMR (400 MHz, DMSO-d₆) δ ppm 7.96 (d, *J* = 7.6 Hz, 2H), 7.55 (s, 1H), 7.46 (t, *J* = 7.7 Hz, 1H), 7.09 (s, 2H), 4.21 (t, *J* = 6.0 Hz, 2H), 3.90 (s, 3H), 2.61 (t, *J* = 6.0 Hz, 2H), 2.21 (s, 3H). MS obsd. (ESI⁺): 389.0 [(M+H)⁺], 391.1 [(M+2+H)⁺].

### Example 11

### 2-[3-[5-(8-Chloro-4-oxo-chromen-2-yl)-4-methoxy-2-methyl-phenoxy]propoxy]acetic acid

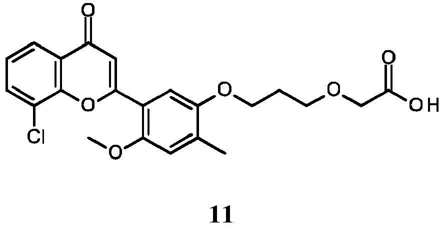

### Step 1. Synthesis of tert-butyl 2-[3-[5-(8-chloro-4-oxo-chromen-2-yl)-4-methoxy-2-methyl-phenoxy]propoxy]acetate

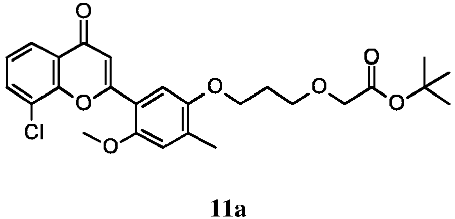

Compound **11a** was prepared in analogy to the procedure described for the preparation of **example 9** by using *tert*-butyl 2-[3-[2-bromo-5-(8-chloro-4-*oxo*-chromen-2-yl)-4-methoxy-phenoxy]propoxy]acetate (compound **6a**) as the starting material instead of *tert*-butyl 3-[2-[2-bromo-5-(8-chloro-4-*oxo*-chromen-2-yl)-4-methoxy-phenoxy]ethoxy]cyclobutanecarboxylate (compound **9a**) in **Step 2.** *Tert*-butyl2-[3-[5-(8-chloro-4-*oxo*-chromen-2-yl)-4-methoxy-2-methyl-phenoxy]propoxy]acetate (80 mg, 81% yield, Example **11**) was obtained as a yellow solid. MS obsd. (ESI⁺): 489.2 [(M+H)⁺], 491.2 [(M+2+H)⁺],

### Step 2. Synthesis of 2-[3-[5-(8-chloro-4-oxo-chromen-2-yl)-4-methoxy-2-methyl-phenoxy]propoxy]acetic acid

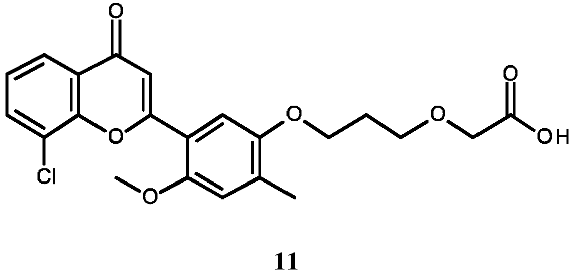

Example **11** was prepared in analogy to the procedure described for the preparation of example **1** by using *tert*-butyl 2-[3-[5-(8-chloro-4-*oxo*-chromen-2-yl)-4-methoxy-2-methyl-phenoxy]propoxy]acetate (compound **11a**) as the starting material instead of *tert*-butyl 3-[2-[2-bromo-5-(8-chloro-4-*oxo*-chromen-2-yl)-4-methoxy-phenoxy]ethoxy]cyclobutanecarboxylate (compound **1d)** in **Step 5.** 2-[3-[5-(8-Chloro-4-*oxo*-chromen-2-yl)-4-methoxy-2-methyl-phenoxy]propoxy]acetic acid (20 mg, 35% yield, Example **11**) was obtained as a white solid. ¹H NMR (400 MHz, DMSO-*d₆*) δ ppm 12.59 (s, 1H), 7.95-8.03 (m, 2H), 7.55 (s, 1H), 7.48 (t, *J* = 7.9 Hz, 1H), 7.14 (s, 1H), 7.10 (s, 1H), 4.11 (t, *J* = 5.9 Hz, 2H), 4.02 (s, 2H), 3.93 (d, *J* = 10.8 Hz, 2H), 3.66 (s, 1H), 2.26 (s, 3H), 1.98-2.07 (m, 2H). MS obsd. (ESI⁺): 433.1 [(M+H)⁺], 435.1 [(M+2+H)⁺],

### Example 12

### 3-[5-(8-Chloro-4-oxo-chromen-2-yl)-2,4-dimethoxy-phenoxy]cyclobutanecarboxylic acid

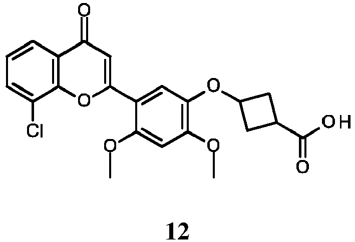

### Step 1. Synthesis of (E)-1-(3-chloro-2-hydroxy-phenyl)-3-(5-hydroxy-2,4-dimethoxyphenyl)prop-2-en-1-one

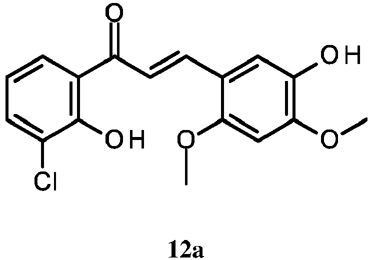

Compound **12a** was prepared in analogy to the procedure described for the preparation of example **1** by using 5-Hydroxy-2,4-dimethoxy-benzaldehyde (Intermediate **AC**) as the starting material instead of 4-bromo-2-methoxy-5-[(4-methoxyphenyl)methoxy]benzaldehyde (Intermediate **AA**) in **Step 1.** (*E*)-1-(3-chloro-2-hydroxy-phenyl)-3-(5-hydroxy-2,4-dimethoxyphenyl)prop-2-en-1-one (4 g, 87%, yield, compound **12a**) was obtained as a yellow solid, which was used in the next step directly without further purification. MS obsd. (ESI+) [(M+H)+]: 335.

### Step 2. Synthesis of 8-chloro-2-(5-hydroxy-2,4-dimethoxy-phenyl)chromen-4-one

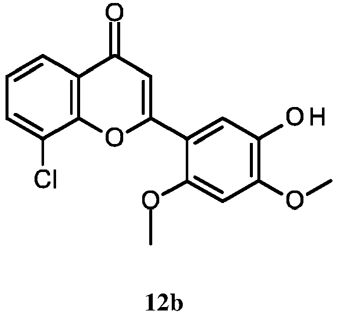

Compound **12b** was prepared in analogy to the procedure described for the preparation of example **1** by using (*E*)-1-(3-chloro-2-hydroxyphenyl)-3-(5-hydroxy-2,4-dimethoxyphenyl)prop-2-en-1-one (500 mg, compound **12a**) as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2.** 8-Chloro-2-(5-hydroxy-2,4-dimethoxy-phenyl)chromen-4-one (400 mg, yield 85%, compound **12b**) was obtained as light yellow solid. MS obsd. (ESI+) [(M+H)+]: 333.

### Step 3. Synthesis of methyl 3-[5-(8-chloro-4-oxo-chromen-2-yl)-2,4-dimethoxy-phenoxy]cyclobutanecarboxylate (12c)

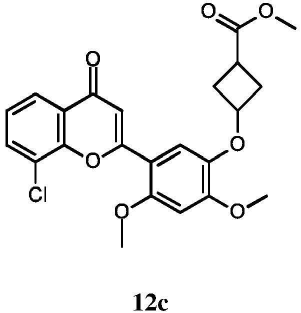

Compound **12c** was prepared in analogy to the procedure described for the preparation of example **1** by using 8-chloro-2-(5-hydroxy-2,4-dimethoxy-phenyl)chromen-4-one (compound **12b**) and methyl 3-(*p*-tolylsulfonyloxy)cyclobutanecarboxylate as the starting material instead of 2-(4-bromo-5-hydroxy-2-methoxy-phenyl)-8-chloro-chromen-4-one (compound **1c**) and cis*-tert-*butyl 3-[2-(*p*-tolylsulfonyloxy)ethoxy]cyclobutanecarboxylate (Intermediate **AP**) in **Step 4.** Methyl 3-[5-(8-chloro-4-*oxo*-chromen-2-yl)-2,4-dimethoxy-phenoxy]cyclobutanecarboxylate (200 mg, 74.8% yield, compound **12c**) was obtained as a yellow oil and was used in the next step directly. MS obsd. (ESI+) [(M+H)+]: 445.

### Step 4. Synthesis of 3-[5-(8-Chloro-4-oxo-chromen-2-yl)-2,4-dimethoxy-phenoxy]cyclobutanecarboxylic acid

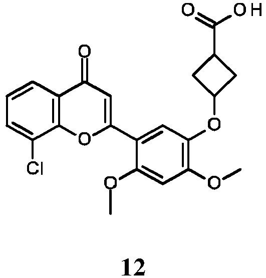

Example **12** was prepared in analogy to the procedure described for the preparation of example **7** by using methyl 3-[5-(8-chloro-4-*oxo*-chromen-2-yl)-2,4-dimethoxy-phenoxy]cyclobutanecarboxylate (compound **12c**) as the starting material instead of methyl 3-[2-chloro-5-(8-chloro-4-*oxo*-chromen-2-yl)-4-methoxy-phenoxy]cyclobutanecarboxylate (compound **7c**) in **Step 4.** 3-[5-(8-Chloro-4-*oxo*-chromen-2-yl)-2,4-dimethoxy-phenoxy]cyclobutanecarboxylic acid (40 mg, 41% yield, example **12**) was obtained as a yellow powder. ¹H NMR (400 MHz, DMSO-*d₆*) δ ppm 12.2-12.4 (m, 1H), 7.9-8.0 (m, 2H), 7.4-7.5 (m, 2H), 7.1-7.2 (m, 1H), 6.9-6.9 (m, 1H), 4.9-4.6 (m, 1H), 4.0-4.0 (m, 3H), 3.93 (s, 3H), 3.06-2.8 (m, 1H), 2.7-2.8 (m, 2H), 2.3-2.5 (m, 1H), 2.2-2.3 (m, 1H). MS obsd. (ESI+) [(M+H)⁺]: 431.

### Example 13

### 3-[2-[5-(8-Chloro-4-oxo-chromen-2-yl)-2,4-dimethoxy-phenoxy]ethoxy]cyclobutanecarboxylic acid

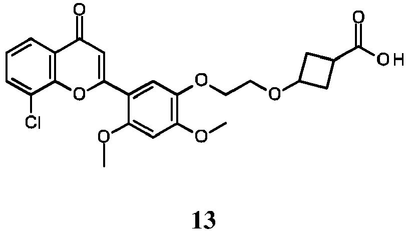

### Step 1. Synthesis of methyl 3-[2-[5-(8-chloro-4-oxo-chromen-2-yl)-2,4-dimethoxy-phenoxy]ethoxy]cyclobutanecarboxylate

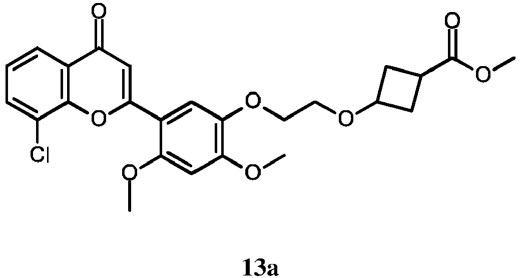

Compound **13a** was prepared in analogy to the procedure described for the preparation of example **1** by using 8-chloro-2-(5-hydroxy-2,4-dimethoxy-phenyl)chromen-4-one (compound **12b**) and methyl 3-[2-(*p*-tolylsulfonyloxy)ethoxy]cyclobutanecarboxylate (Intermediate **AO**) as the starting material instead of 2-(4-bromo-5-hydroxy-2-methoxy-phenyl)-8-chloro-chromen-4-one (compound **1c**) and *cis-tert*-butyl3-[2-(*p*-tolylsulfonyloxy)ethoxy]cyclobutanecarboxylate in **Step 4.** Methyl 3-[2-[5-(8-chloro-4-*oxo*-chromen-2-yl)-2,4-dimethoxy-phenoxy]ethoxy]cyclobutanecarboxylate(200 mg, 74.8% yield, compound **13a**) was obtained as a yellow oil and the residue was used in the next step directly. MS obsd. (ESI⁺) [(M+H)⁺]: 445.

### Step 2. Synthesis of 3-[2-[5-(8-chloro-4-oxo-chromen-2-yl)-2,4-dimethoxy-phenoxy]ethoxy]cyclobutanecarboxylic acid

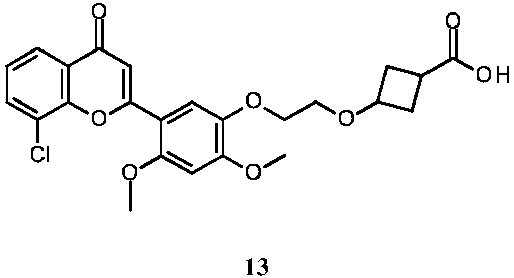

Example **13** was prepared in analogy to the procedure described for the preparation of example **7** by using methyl 3-[2-[5-(8-chloro-4-*oxo*-chromen-2-yl)-2,4-dimethoxy-phenoxy]ethoxy]cyclobutanecarboxylate (compound **13a**) as the starting material instead of methyl 3-[2-chloro-5-(8-chloro-4-*oxo*-chromen-2-yl)-4-methoxy-phenoxy]cyclobutanecarboxylate (compound **7c**) in **Step 4.** 3-[2-[5-(8-Chloro-4-*oxo*-chromen-2-yl)-2,4-dimethoxy-phenoxy]ethoxy]cyclobutanecarboxylic acid (80 mg, 39.9% yield, Example **13**) was obtained as a yellow solid. ¹H NMR (400 MHz, DMSO-d₆) δ ppm 12.0-12.3 (m, 1H), 7.9-8.0 (m, 2H), 7.6-7.6 (m, 1H), 7.4-7.5 (m, 1H), 7.08 (s, 1H), 6.9-6.9 (m, 1H), 4.1-4.2 (m, 1H), 4.09 (br s, 2H), 3.99 (s, 3H), 3.94 (s, 3H), 3.65 (*br* d, 2H, *J* = 1.1 Hz), 2.8-3.0 (m, 1H), 2.3-2.5 (m, 2H), 2.1-2.2 (m, 1H), 1.9-2.0 (m, 1H). MS obsd. (ESI⁺) [(M+H)⁺]: 475.

### Example 14

### 2-[5-(8-Chloro-4-oxo-chromen-2-yl)-2-methyl-phenoxy]acetic acid

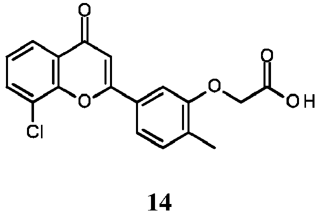

### Step 1. Synthesis of ethyl 2-[5-(8-chloro-4-oxo-chromen-2-yl)-2-methyl-phenoxy]acetate

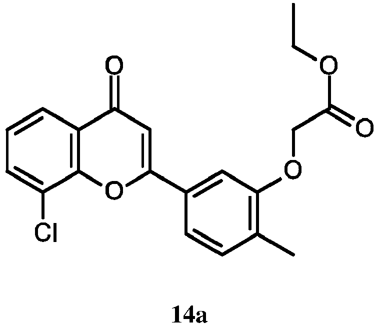

Compound **14a** was prepared in analogy to the procedure described for the preparation of example **1** by using 8-chloro-2-(3-hydroxy-4-methyl-phenyl)chromen-4-one and ethyl 2-bromoacetate as the starting material instead of 2-(4-bromo-5-hydroxy-2-methoxy-phenyl)-8-chloro-chromen-4-one (compound **1c**) and *cis-tert*-butyl 3-[2-(*p-*tolylsulfonyloxy)ethoxy]cyclobutanecarboxylate in **Step 4.** Ethyl 2-(5-(8-chloro-4-*oxo*-chromen-2-yl)-2-methylphenoxy)acetate (240 mg, 92.3 % yield, compound **14a**) was obtained as a yellow solid, which was used in the next step directly. MS obsd. (ESI+) [(M+H)+]: 373.

### Step 2. Synthesis of 2-[5-(8-chloro-4-oxo-chromen-2-yl)-2-methyl-phenoxy]acetic acid

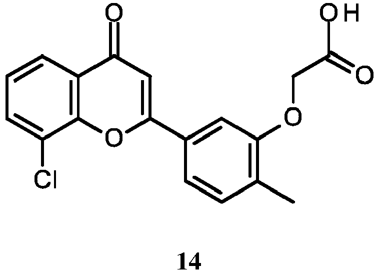

Example **14** was prepared in analogy to the procedure described for the preparation of example **7** by using ethyl 2-(5-(8-chloro-4-*oxo*-chromen-2-yl)-2-methylphenoxy)acetate (compound **14a**) as the starting material instead of methyl 3-[2-chloro-5-(8-chloro-4-*oxo-*chromen-2-yl)-4-methoxy-phenoxy]cyclobutanecarboxylate (compound **7c**) in **Step 4.** 2-[5-(8-Chloro-4-*oxo*-chromen-2-yl)-2-methyl-phenoxy] acetic acid (23 mg, 11.9 % yield, example **14**) was obtained as a yellow foam. ¹H NMR (400 MHz, DMSO-*d₆*) δ ppm 13.07 (*br* s, 1H), 8.0-8.0 (m, 2H), 7.67 (*dd,* 1H, *J* = 1.5, 7.8 Hz), 7.5-7.6 (m, 2H), 7.41 (d, 1H, *J* = 8.3 Hz), 7.22 (s, 1H), 4.89 (s, 2H), 2.29 (s, 3H). MS obsd. (ESI⁺) [(M+H)⁺]: 345.

### Example 15 and Example 16

### Cis-3-[5-(8-chloro-4-oxo-chromen-2-yl)-2-methyl-phenoxy]cyclobutanecarboxylic acid (Example 15) and trans-3-[5-(8-chloro-4-oxo-chromen-2-yl)-2-methyl-phenoxy]cyclobutanecarboxylic acid (Exmaple 16)

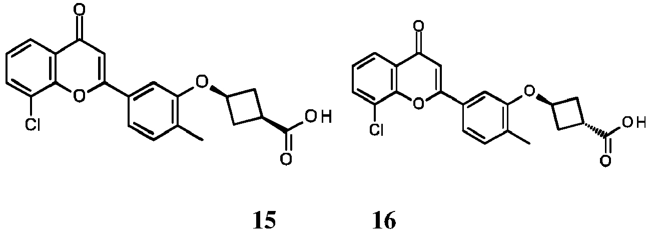

### Step 1. Synthesis of methyl 3-[5-(8-chloro-4-oxo-chromen-2-yl)-2-methyl-phenoxy]cyclobutanecarboxylate

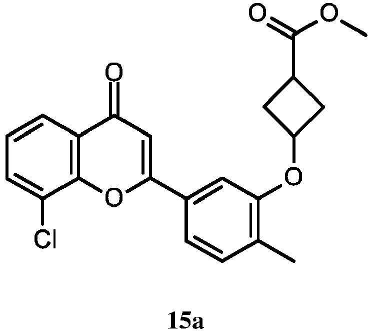

Compound **15a** was prepared in analogy to the procedure described for the preparation of example **1** by using 8-chloro-2-(3-hydroxy-4-methyl-phenyl)chromen-4-one and methyl 3-chlorocyclobutane-1-carboxylate as the starting material instead of 2-(4-bromo-5-hydroxy-2-methoxy-phenyl)-8-chloro-chromen-4-one (compound **1c**) and *tert*-butyl 3-[2-(*p-*tolylsulfonyloxy)ethoxy]cyclobutanecarboxylate in **Step 4.** Methyl 3-[5-(8-chloro-4-*oxo-*chromen-2-yl)-2-methyl-phenoxy]cyclobutanecarboxylate (250 mg, 89% yield, compound **15a**) was obtained as a yellow oil, which was used in the next step directly. MS obsd. (ESI+) [(M+H)+]: 399.

### Step 2. Synthesis of 3-[5-(8-chloro-4-oxo-chromen-2-yl)-2-methyl-phenoxy]cyclobutanecarboxylic acid

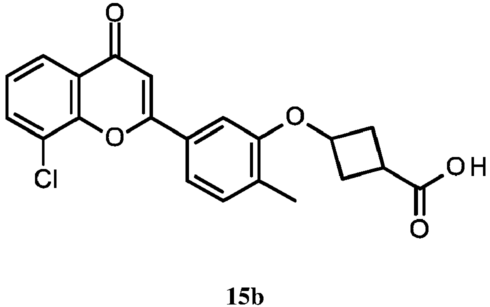

Compound **15b** was prepared in analogy to the procedure described for the preparation of example **7** by using methyl 3-[5-(8-chloro-4-*oxo*-chromen-2-yl)-2-methyl-phenoxy]cyclobutanecarboxylate (compound **15a**) as the starting material instead of methyl 3-[2-chloro-5-(8-chloro-4-*oxo*-chromen-2-yl)-4-methoxy-phenoxy]cyclobutanecarboxylate (compound **7c**) in **Step 4.** 3-[5-(8-Chloro-4-*oxo-*chromen-2-yl)-2-methyl-phenoxy]cyclobutanecarboxylic acid (compound **15b**) was obtained as a yellow solid. MS obsd. (ESI⁺) [(M+H)⁺]: 385.

Compound **15b** was further purified by supercritical fluid chromatography (SFC) to give *cis*-3-[5-(8-chloro-4-*oxo*-chromen-2-yl)-2-methyl-phenoxy]cyclobutanecarboxylic acid (Example **15**) and *trans*-3-[5-(8-chloro-4-*oxo*-chromen-2-yl)-2-methyl-phenoxy]cyclobutanecarboxylic acid (Example **16**). The configuration of Example **15** and Example **16** were determined by NOESY.

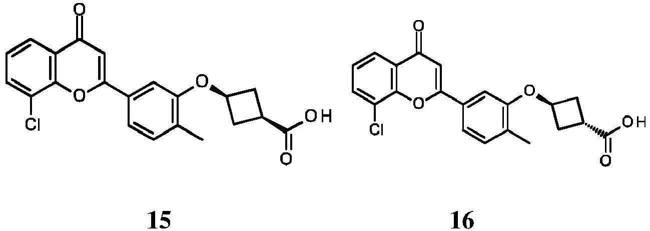

### Example 15

24 mg, 24.1% yield, light yellow solid. ¹H NMR (400 MHz, DMSO-*d₆*) δ ppm 11.9-12.8 (m, 1H), 7.9-8.1 (m, 2H), 7.6-7.7 (m, 1H), 7.5-7.5 (m, 1H), 7.4-7.5 (m, 1H), 7.3-7.4 (m, 1H), 7.1-7.2 (m, 1H), 4.7-4.9 (m, 1H), 2.7-2.9 (m, 3H), 2.1-2.4 (m, 5H). MS obsd. (ESI⁺) [(M+H)⁺]: 385.

### Example 16

15 mg, 14.8% yield, light yellow solid. ¹H NMR (400 MHz, DMSO-*d₆*) δ ppm 12.0-12.7 (m, 1H), 7.9-8.1 (m, 2H), 7.6-7.7 (m, 1H), 7.5-7.5 (m, 1H), 7.4-7.5 (m, 1H), 7.3-7.4 (m, 1H), 7.17 (s, 1H), 4.9-5.1 (m, 1H), 3.1-3.2 (m, 1H), 2.7-2.8 (m, 2H), 2.3-2.5 (m, 2H), 2.25 (s, 3H). MS obsd. (ESI⁺) [(M+H)⁺]: 385.

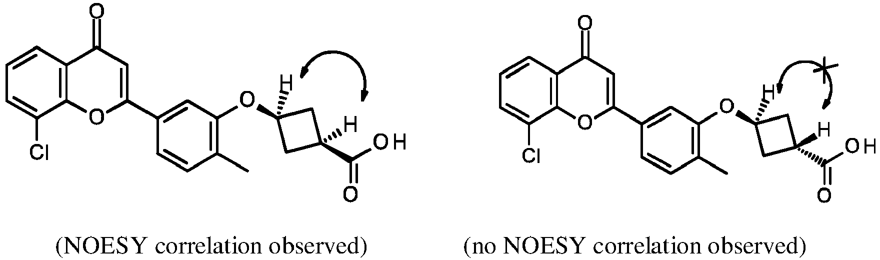

### Example 17

### 3-[5-(8-Chloro-4-oxo-chromen-2-yl)-2-methoxy-phenoxy]cyclobutanecarboxylic acid

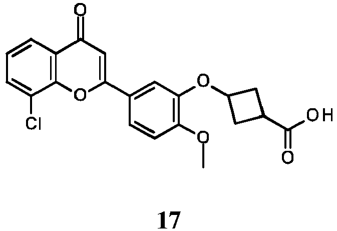

### Step 1. Synthesis of (E)-1-(3-chloro-2-hydroxy-phenyl)-3-(3-hydroxy-4-methoxyphenyl)prop-2-en-1-one

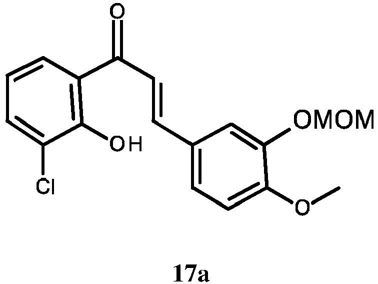

Compound **17a** was prepared in analogy to the procedure described for the preparation of example **1** by using 3-hydroxy-4-methoxybenzaldehyde as the starting material instead of 4-bromo-2-methoxy-5-[(4-methoxyphenyl)methoxy]benzaldehyde (Intermediate **AA**) in **Step 1.** (*E*)-1-(3-chloro-2-hydroxy-phenyl)-3-(3-hydroxy-4-methoxy-phenyl)prop-2-en-1-one (4.3 g, 85.9% yield, compound **17a**) was obtained as a yellow solid, which was used in the next step directly without further purification. MS obsd. (ESI⁺) [(M+H)⁺]: 305.

### Step 2. Synthesis of 8-chloro-2-(3-hydroxy-4-methoxy-phenyl)chromen-4-one

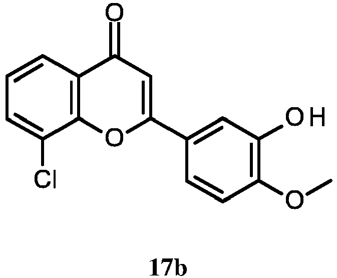

Compound **17b** was prepared in analogy to the procedure described for the preparation of example **1** by using (*E*)-1-(3-chloro-2-hydroxyphenyl)-3-(3-hydroxy-4-methoxyphenyl)prop-2-en-1-one as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2.** 8-Chloro-2-(3-hydroxy-4-methoxy-phenyl)chromen-4-one (1.56 g, 78.5% yield, compound **17b**) was obtained as a light yellow solid. MS obsd. (ESI⁺) [(M+H)⁺]: 303.

### Step 3. Synthesis of methyl 3-[5-(8-chloro-4-oxo-chromen-2-yl)-2-methoxy-phenoxy]cyclobutanecarboxylate

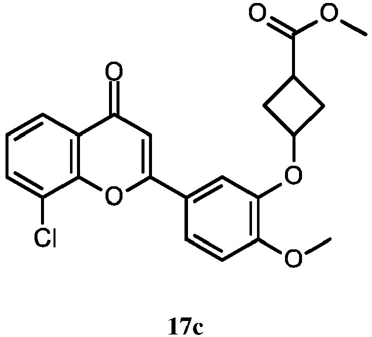

Compound **17c** was prepared in analogy to the procedure described for the preparation of example **1** by using 8-chloro-2-(3-hydroxy-4-methoxyphenyl)-chromen-4-one (compound **17b**) and methyl 3-(*p*-tolylsulfonyloxy)cyclobutanecarboxylate as the starting material instead of 2-(4-bromo-5-hydroxy-2-methoxy-phenyl)-8-chloro-chromen-4-one (compound **1c**) and *cis-tert-butyl* 3-[2-(*p*-tolylsulfonyloxy)ethoxy]cyclobutanecarboxylate (Intermediate **AP**) in **Step 4**. Methyl3-[5-(8-chloro-4-*oxo*-chromen-2-yl)-2-methoxy-phenoxy]cyclobutanecarboxylate (220 mg, 80% yield, compound **17c**) was obtained as a yellow solid and the residue was used in the next step directly. MS obsd. (ESI+) [(M+H)+]: 415.

### Step 4. Synthesis of [5-(8-chloro-4-oxo-chromen-2-yl)-2-methoxy-phenoxy]cyclobutanecarboxylic acid

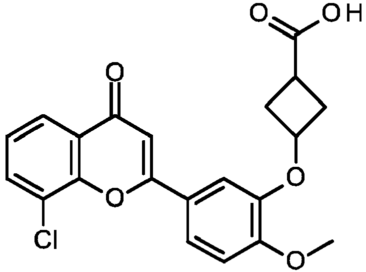

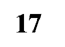

Example **17** was prepared in analogy to the procedure described for the preparation of example **7** by using Methyl 3-[5-(8-chloro-4-*oxo*-chromen-2-yl)-2-methoxy-phenoxy]cyclobutanecarboxylate (220 mg, compound **17c**) as the starting material instead of methyl 3-[2-chloro-5-(8-chloro-4-*oxo*-chromen-2-yl)-4-methoxy-phenoxy]cyclobutanecarboxylate (compound 7c) in **Step 4.** 3-[5-(8-Chloro-4-*oxo*-chromen-2-yl)-2-methoxy-phenoxy]cyclobutanecarboxylic acid (105 mg, 53.3% yield, example **17**) was obtained as a yellow foam. ¹H NMR (400 MHz, DMSO-*d₆*) δ ppm 12.3-12.4 (m, 1H), 8.0-8.0 (m, 2H), 7.7-7.8 (m, 1H), 7.5-7.5 (m, 2H), 7.2-7.2 (m, 1H), 7.1-7.1 (m, 1H), 4.96 (s, 1H), 3.8-3.9 (m, 3H), 2.7-3.1 (m, 3H), 2.4-2.5 (m, 1H), 2.23 (br d, 1H, *J* = 7.5 Hz). MS obsd. (ESI⁺) [(M+H)⁺]: 401.

### Example 18

### 3-[2-[5-(8-Chloro-4-oxo-chromen-2-yl)-2-methoxy-phenoxy]ethoxy]cyclobutanecarboxylic acid

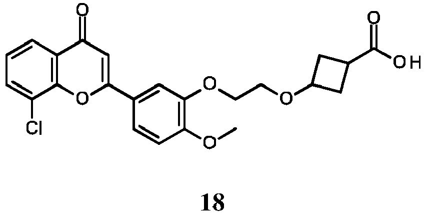

### Step 1. Synthesis of methyl 3-[2-[5-(8-chloro-4-oxo-chromen-2-yl)-2-methoxy-phenoxy]ethoxy]cyclobutanecarboxylate

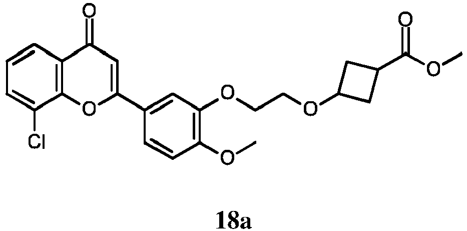

Compound **18a** was prepared in analogy to the procedure described for the preparation of example **1** by using 8-chloro-2-(3-hydroxy-4-methoxyphenyl)-chromen-4-one and methyl 3-(2-(tosyloxy)ethoxy)cyclobutane-1-carboxylate as the starting material (Intermediate **AO**) instead of 2-(4-bromo-5-hydroxy-2-methoxy-phenyl)-8-chloro-chromen-4-one (compound **1c**) and *cis-tert*-butyl 3-[2-(*p*-tolylsulfonyloxy)ethoxy]cyclobutanecarboxylate (Intermediate **AP**) in **Step 4**. Methyl 3-[2-[5-(8-chloro-4-*oxo*-chromen-2-yl)-2-methoxy-phenoxy]ethoxy]cyclobutanecarboxylate (250 mg, 82.5% yield, compound **18a**) was obtained as a yellow oil and the residue was used in the next step directly. MS obsd. (ESI+) [(M+H)+]: 459.

### Step 2. Synthesis of 3-[2-[5-(8-chloro-4-oxo-chromen-2-yl)-2-methoxy-phenoxy]ethoxy]cyclobutanecarboxylic acid

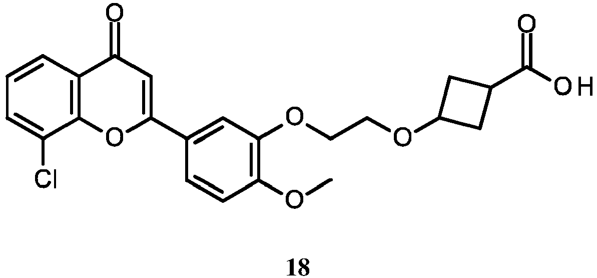

Example **18** was prepared in analogy to the procedure described for the preparation of example **7** by using methyl 3-[2-[5-(8-chloro-4-*oxo*-chromen-2-yl)-2-methoxy-phenoxy]ethoxy]cyclobutanecarboxylate (250 mg, compound **18a**) as the starting material instead of methyl 3-[2-chloro-5-(8-chloro-4-*oxo*-chromen-2-yl)-4-methoxy-phenoxy]cyclobutanecarboxylate (compound **7c**) in **Step 4.** 3-[2-[5-(8-chloro-4-oxo-chromen-2-yl)-2-methoxy-phenoxy]ethoxy]cyclobutanecarboxylic acid (100 mg, 50% yield, example **18**) was obtained as a light yellow powder. ¹H NMR (400 MHz, DMSO-*d₆*) δ ppm 12.1-12.2 (m, 1H), 7.99 (d, *J* = 8.1 Hz, 2H), 7.7-7.8 (m, 1H), 7.6-7.7 (m, 1H), 7.4-7.5 (m, 1H), 7.17 (s, 2H), 4.1-4.3 (m, 2.5H), 3.97 (t, 0.5H, *J* = 6.9 Hz), 3.88 (s, 3H), 3.9-4.3 (m, 3H), 2.5-3.0 (m, 1H), 2.4-2.5 (m, 2H), 2.1-2.2 (m, 1H), 2.0-2.1 (m, 1H). MS obsd. (ESI⁺) [(M+H)⁺]: 445.

### Example 19

### 3-[5-(8-Chloro-4-oxo-chromen-2-yl)-2-methoxy-phenoxy]propanoic acid

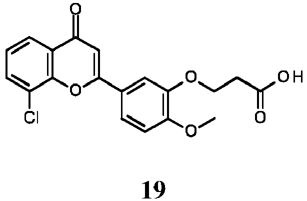

Example **19** was prepared in analogy to the procedure described for the preparation of example **2** by using 8-chloro-2-(3-hydroxy-4-methoxyphenyl)-chromen-4-one as the starting material instead of 2-(4-bromo-5-hydroxy-2-methoxy-phenyl)-8-chloro-chromen-4-one (compound **1c**). 3-[5-(8-Chloro-4-*oxo*-chromen-2-yl)-2-methoxy-phenoxy]propanoic acid (33.8 mg, 13% yield, example **19**) was obtained as an off-white powder. ¹H NMR (400 MHz, DMSO-*d₆)* δ ppm 12.41 (br s, 1H), 8.00 (dd, *J* = 1.2, 7.9 Hz, 2H), 7.7-7.8 (m, 1H), 7.6-7.7 (m, 1H), 7.4-7.5 (m, 1H), 7.19 (s, 2H), 4.31 (t, *J* = 6.1 Hz, 2H), 3.86 (s, 3H), 2.75 (t, *J* = 6.0 Hz, 2H). MS obsd. (ESI⁺) [(M+H)⁺]: 375.

### Example 20 and Example 21

### Cis-3-[5-(8-chloro-4-oxo-chromen-2-yl)-2-(trifluoromethyl)phenoxy]cyclobutanecarboxylic acid and trans-3-[5-(8-chloro-4-oxo-chromen-2-yl)-2-(trifluoromethyl)phenoxy] cyclobutanecarboxylic acid

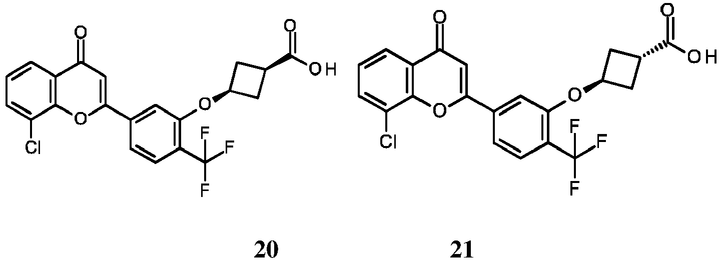

### Step 1. Synthesis of (E)-1-(3-chloro-2-hydroxy-phenyl)-3-[3-methoxy-4-(trifluoromethyl)phenyl]prop-2-en-1-one

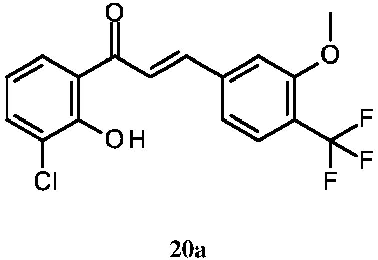

Compound **20a** was prepared in analogy to the procedure described for the preparation of example **1** by using 3-methoxy-4-(trifluoromethyl)benzaldehyde as the starting material instead of 4-bromo-2-methoxy-5-[(4-methoxyphenyl)methoxy]benzaldehyde (Intermediate **AA**) in **Step 1.** (*E*)-1-(3-chloro-2-hydroxy-phenyl)-3-[3-methoxy-4-(trifluoromethyl)phenyl]prop-2-en-1-one (1.5g, 85.8% yield, compound **20a**) was obtained as yellow solid, which was used in the next step directly without further purification. (ESI⁺) [(M+H)⁺]: 357.

### Step 2. Synthesis of 8-chloro-2-(3-methoxy-4-(trifluoromethyl)phenyl)-chromen-4-one

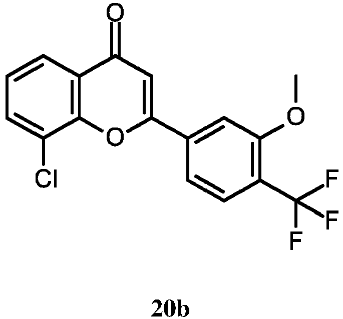

Compound **20b** was prepared in analogy to the procedure described for the preparation of example **1** by using (*E*)-1-(3-chloro-2-hydroxy-phenyl)-3-[3-methoxy-4-(trifluoromethyl)phenyl]prop-2-en-1-one (1.5g, compound **20a**) as the starting material instead of (*E*)-3-[4-bromo-2-methoxy-5-[(4-methoxyphenyl)methoxy]phenyl]-1-(3-chloro-2-hydroxyphenyl)prop-2-en-1-one (compound **1a**) in **Step 2.** 8-chloro-2-(3-methoxy-4-(trifluoromethyl)phenyl)-chromen-4-one (900 mg, 90.5% yield, compound **20b**) was obtained as light yellow solid. MS obsd. (ESI⁺) [(M+H)⁺]: 355.

### Step 3. Synthesis of 8-chloro-2-(3-hydroxy-4-(trifluoromethyl)phenyl)-chromen-4-one

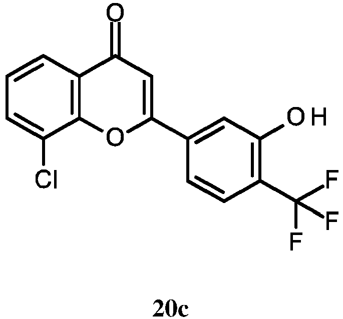

The solution of 8-chloro-2-(3-methoxy-4-(trifluoromethyl)phenyl)-chromen-4-one (300 mg) in DCM (20 mL) was stirred at 25 °C for 14 hrs. EtOAc and water were poured into the reaction mixture. The organic layer was washed with brine, dried by Na₂SO₄, concentrated to give 8-chloro-2-(3-hydroxy-4-(trifluoromethyl)phenyl)-chromen-4-one (270 mg, 93.7% yield, compound **20c)** as a yellow solid, which was used in the next step directly without further purification. MS obsd. (ESI⁺) [(M+H)⁺]: 341.

### Step 4. Synthesis of methyl 3-(5-(8-chloro-4-oxo-chromen-2-yl)-2-(trifluoromethyl)phenoxy)cyclobutane-1-carboxylate

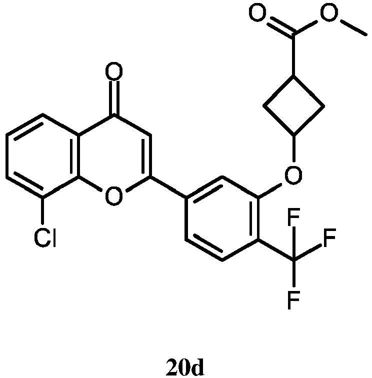

Compound **20d** was prepared in analogy to the procedure described for the preparation of example **1** by using 8-chloro-2-(3-hydroxy-4-(trifluoromethyl)phenyl)-chromen-4-one (compound **20c**) and methyl 3-chlorocyclobutane-1-carboxylate as the starting material instead of 2-(4-bromo-5-hydroxy-2-methoxy-phenyl)-8-chloro-chromen-4-one (compound **1c**) and *cis-tert*-butyl 3-[2-(*p*-tolylsulfonyloxy)ethoxy]cyclobutanecarboxylate (Intermediate **AP**) in **Step 4.** Methyl 3-(5-(8-chloro-4-*oxo*-chromen-2-yl)-2-(trifluoromethyl)phenoxy)cyclobutane-1-carboxylate (100 mg, 75.2% yield, compound **20d**) was obtained as a yellow oil and the residue was used in the next step directly. MS obsd. (ESI⁺) [(M+H)⁺]: 453.

### Step 5. Synthesis of 3-[5-(8-chloro-4-oxo-chromen-2-yl)-2-(trifluoromethyl)phenoxy]cyclobutanecarboxylic acid

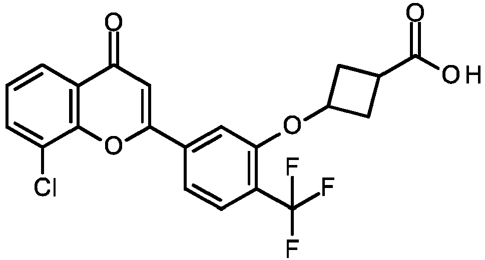

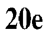

Compound **20e** was prepared in analogy to the procedure described for the preparation of example **7** by using methyl 3-(5-(8-chloro-4-*oxo*-chromen-2-yl)-2-(trifluoromethyl)phenoxy)cyclobutane-1-carboxylate (compound **20d**) as the starting material instead of methyl 3-[2-chloro-5-(8-chloro-4-*oxo*-chromen-2-yl)-4-methoxy-phenoxy]cyclobutanecarboxylate (compound 7c) in Step 4. 3-[5-(8-chloro-4-oxo-chromen-2-yl)-2-(trifluoromethyl)phenoxy]cyclobutanecarboxylic acid (compound **20e**) was obtained as a yellow solid. MS obsd. (ESI⁺) [(M+H)⁺]: 439.

Separation of **20e** by Pre-HPLC give 3-[5-(8-chloro-4-*oxo*-chromen-2-yl)-2-(trifluoromethyl)phenoxy]cyclobutanecarboxylic acid with *cis-* and *trans-* configuration, one of which is characterized as *cis*-configuration (Example **20**, 3.2 mg, 3%) and the other is characterized as *trans*-configuration (Example **21,** 3.0mg, 2.8%). (Separation condition: H₂O (0.1% FA) and ACN (flow rate: 25 ml/min) on waters sun fire C18 Column. The configuration of Example **20** and Example **21** were determined by NOESY

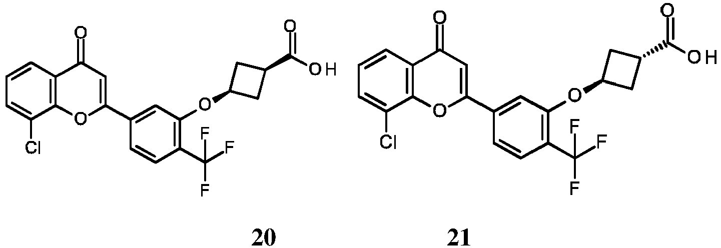

### Example 20

¹H NMR (DMSO-*d₆*) δ ppm 12.12-12.53 (m, 1 H), 8.04 (ddd, *J* = 9.48, 7.95, 1.53 Hz, 2 H), 7.81 - 7.89 (m, 2H), 7.71(s, 1 H), 7.37 (s, 1 H), 7.53 (t, *J* = 7.89 Hz,1H), 6.51 (s, 1 H), 4.92 - 5.08 (m, 1 H), 2.75-2.90 (m, 3 H), 2.17-2.30 (m, 2 H). MS obsd. (ESI⁺) [(M+H)⁺]: 439.

### Example 21

¹H NMR (400 MHz, DMSO-*d₆*) δ ppm 12.23-12.61 (m, 1 H), 8.04 (ddd, *J* = 10.21, 7.95, 1.53 Hz, 2H), 7.86 (s, 2 H), 7.69 (s, 1 H), 7.53 (t, *J* = 7.89 Hz, 1H), 7.38 (s, 1H), 5.19 (t, *J* = 6.66 Hz, 1 H), 3.08-3.18 (m, 1 H), 2.80 (ddd, *J* = 13.51, 7.09, 3.73 Hz, 2 H), 2.34-2.45 (m, 3 H). MS obsd. (ESI⁺) [(M+H)⁺]: 439.

### Example 22

### 2-[5-(8-Chloro-4-oxo-chromen-2-yl)-2-(trifluoromethyl)phenoxy]acetic acid

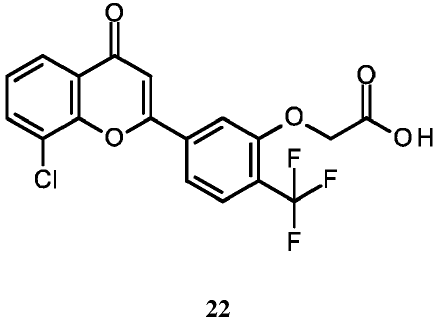

### Step 1. Synthesis of ethyl 2-(5-(8-chloro-4-oxo-chromen-2-yl)-2-(trifluoromethyl)phenoxy)acetate

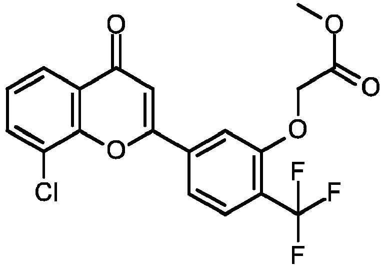

Compound **22a** was prepared in analogy to the procedure described for the preparation of example **1** by using 8-chloro-2-(3-hydroxy-4-(trifluoromethyl)phenyl)-chromen-4-one (compound **20c**) and ethyl 2-bromoacetate as the starting material instead of 2-(4-bromo-5-hydroxy-2-methoxy-phenyl)-8-chloro-chromen-4-one (compound **1c**) and *cis-tert-*butyl 3*-*[2*-*(*p-*tolylsulfonyloxy)ethoxy]cyclobutanecarboxylate (Intermediate **AP**) in **Step 4.** Ethyl 2-(5-(8-chloro-4-*oxo*-chromen-2-yl)-2-(trifluoromethyl)phenoxy)acetate (100 mg, 79.8 % yield, compound **22a**) was obtained as a yellow solid and the residue was used in the next step directly. MS obsd. (ESI⁺) [(M+H)⁺]:.

### Step 2. Synthesis of 2-[5-(8-chloro-4-oxo-chromen-2-yl)-2-(trifluoromethyl)phenoxy]acetic acid

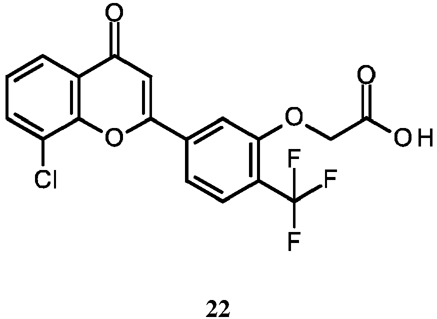

Example **22** was prepared in analogy to the procedure described for the preparation of example **7** by using ethyl 2-(5-(8-chloro-4-*oxo*-chromen-2-yl)-2-(trifluoromethyl)phenoxy)acetate (compound **22a**) as the starting material instead of methyl 3-[2-chloro-5-(8-chloro-4-*oxo*-chromen-2-yl)-4-methoxy-phenoxy]cyclobutanecarboxylate (compound 7c) in **Step 4.** 2-[5-(8-chloro-4-*oxo*-chromen-2-yl)-2-(trifluoromethyl)phenoxy]acetic acid (13 mg, 13.2 % yield, Example **22**) was obtained as a white foam. ¹H NMR (400 MHz, DMSO-*d₆*) δ ppm 8.03 (ddd, 2H, *J* = 1.5, 7.9, 10.2 Hz), 7.8-7.9 (m, 3H), 7.53 (t, 1H, *J* = 7.9 Hz), 7.41 (s, 1H), 5.05 (s, 2H). MS obsd. (ESI⁺) [(M+H)⁺]: 399.

### Example 23

### Cis-3-[5-(8-chloro-4-oxo-chromen-2-yl)-2,3-dimethoxy-phenoxy]cyclobutanecarboxylic acid

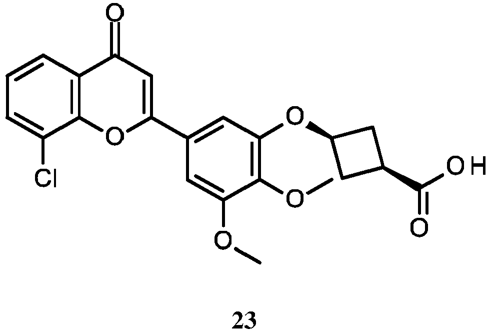

### Step 1. (E)-1-(3-chloro-2-hydroxyphenyl)-3-(3-hydroxy-4,5-dimethoxyphenyl)prop-2-en-1-one

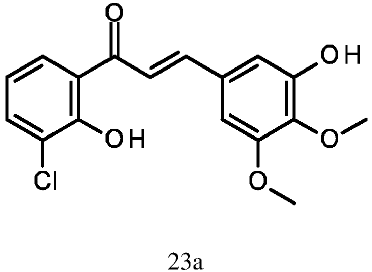

Compound **23a** was prepared in analogy to the procedure described for the preparation of example **1** by using 3-hydroxy-4,5-dimethoxybenzaldehyde as the starting material instead of 4-bromo-2-methoxy-5-[(4-methoxyphenyl)methoxy]benzaldehyde (Intermediate **AA**) in **Step 1.** (*E*)-1-(3-chloro-2-hydroxyphenyl)-3-(3-hydroxy-4,5-dimethoxyphenyl)prop-2-en-1-one (3.5g, 89.2% yield, compound **23a**) was obtained as a yellow solid, which was used in the next step directly without further purification. MS obsd. (ESI⁺) [(M+H)⁺]: 335.

### Step 2. Synthesis of 8-chloro-2-(3-hydroxy-4,5-dimethoxyphenyl)-chromen-4-one

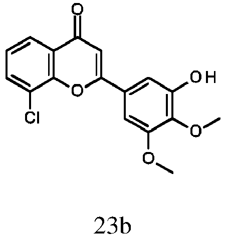

Compound **23b** was prepared in analogy to the procedure described for the preparation of example **1** by using (E)-1-(3-chloro-2-hydroxyphenyl)-3-(3-hydroxy-4,5-dimethoxyphenyl)prop-2-en-1-one (compound **23a**) as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2.** 8-chloro-2-(3-hydroxy-4,5-dimethoxyphenyl)-chromen-4-one (1.5g, 75.5% yield, compound **23b**) was obtained as yellow solid. MS obsd. (ESI⁺) [(M+H)⁺]: 333.

### Step 3. Synthesis of methyl 3-[5-(8-chloro-4-oxo-chromen-2-yl)-2,3-dimethoxy-phenoxy]cyclobutanecarboxylate

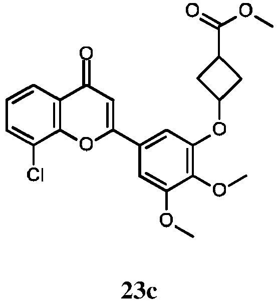

Compound **23c** was prepared in analogy to the procedure described for the preparation of example **1** by using 8-chloro-2-(3-hydroxy-4,5-dimethoxyphenyl)-chromen-4-one (1.5g compound **23b**) and methyl 3-(*p*-tolylsulfonyloxy)cyclobutanecarboxylate as the starting material instead of 2-(4-bromo-5-hydroxy-2-methoxy-phenyl)-8-chloro-chromen-4-one (compound **1c**) and *cis-tert*-butyl 3-[2-(*p*-tolylsulfonyloxy)ethoxy]cyclobutanecarboxylate in **Step 4.** Methyl 3-[5-(8-chloro-4-*oxo*-chromen-2-yl)-2,3-dimethoxy-phenoxy]cyclobutanecarboxylate (350 mg, 87.3% yield, compound **23c**) was obtained as a yellow oil.

Separation of **23c** by Pre-HPLC give methyl 3-[5-(8-chloro-4-*oxo*-chromen-2-yl)-2,3-dimethoxy-phenoxy]cyclobutanecarboxylate with *cis-* and *trans-* configuration, one of which is characterized as *cis*-configuration (Example **23c-1**) and the other is characterized as *trans-*configuration (Example **23c-2**). MS obsd. (ESI⁺) [(M+H)⁺]: 445.

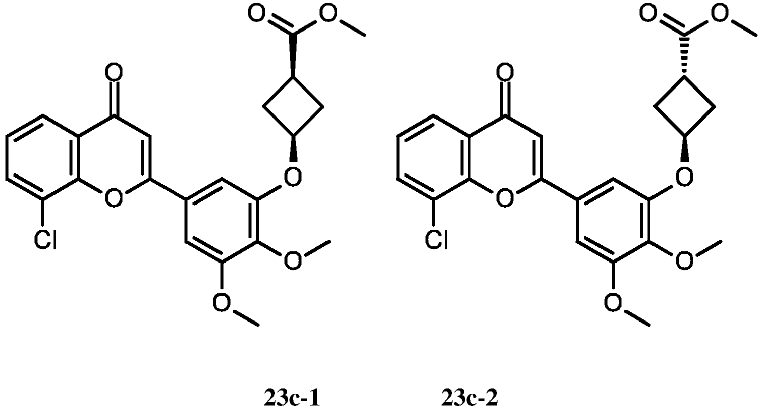

### Step 4. Synthesis of cis-3-[5-(8-chloro-4-oxo-chromen-2-yl)-2,3-dimethoxy-phenoxy]cyclobutanecarboxylic acid

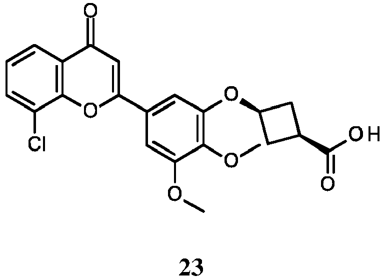

Compound **23** was prepared in analogy to the procedure described for the preparation of example **7** by using *cis*-methyl-3-(5-(8-chloro-4-*oxo*-chromen-2-yl)-2,3-dimethoxyphenoxy)cyclobutane-1-carboxylate (compound **23c-1**) as the starting material instead of methyl 3-[2-chloro-5-(8-chloro-4-*oxo*-chromen-2-yl)-4-methoxy-phenoxy]cyclobutanecarboxylate (compound 7c) in **Step 4.** *Cis*-3-[5-(8-chloro-4-*oxo*-chromen-2-yl)-2,3-dimethoxy-phenoxy]cyclobutanecarboxylic acid (40.5 mg, 79.5 % yield, example **23**) was obtained as an off-white solid ¹H NMR (400 MHz, DMSO-*d₆*) δ ppm 12.30 (br s, 1H), 8.01 (br t, *J* = 7.2 Hz, 2H), 7.50 (br t, *J* = 7.8 Hz, 1H), 7.42 (s, 1H), 7.27 (s, 2H), 4.79 (br d, *J* = 6.5 Hz, 1H), 3.90 (s, 3H), 3.7-3.8 (m, 3H), 2.7-2.9 (m, 3H), 2.2-2.3 (m, 2H). MS obsd. (ESI⁺) [(M+H)⁺]: 431.

### Example 24

### Trans-3-[2-[5-(8-chloro-4-oxo-chromen-2-yl)-2,3-dimethoxy-phenoxy]ethoxy]cyclobutanecarboxylic acid

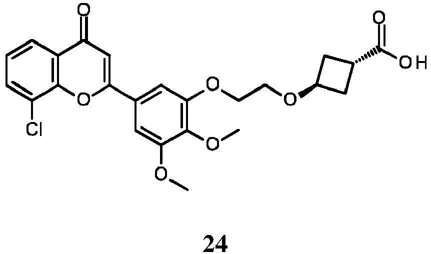

### Step 1. Synthesis of methyl 3-[2-[5-(8-chloro-4-oxo-chromen-2-yl)-2,3-dimethoxy-phenoxy]ethoxy]cyclobutanecarboxylate

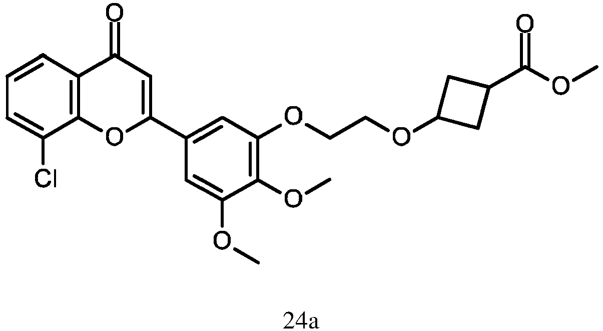

Compound **24a** was prepared in analogy to the procedure described for the preparation of example **1** by using 8-chloro-2-(3-hydroxy-4,5-dimethoxyphenyl)-chromen-4-one (compound **23b**) and methyl 3-(2-(tosyloxy)ethoxy)cyclobutane-1-carboxylate (Intermediate **AO**) as the starting material instead of 2-(4-bromo-5-hydroxy-2-methoxy-phenyl)-8-chloro-chromen-4-one (compound **1c**) and *cis-tert*-butyl 3-[2-(*p*-tolylsulfonyloxy)ethoxy]cyclobutanecarboxylate (Intermediate **AP**) in **Step 4.** Methyl 3-[2-[5-(8-chloro-4-*oxo*-chromen-2-yl)-2,3-dimethoxy-phenoxy]ethoxy]cyclobutanecarboxylate (compound **24a**) was obtained as a yellow solid. MS obsd. (ESI⁺) [(M+H)⁺]: 489.

Separation of compound **24a** give methyl-3-(2-(5-(8-chloro-4-oxo-chromen-2-yl)-2,3-dimethoxyphenoxy)ethoxy)cyclobutane-1-carboxylate as a yellow solid with *cis-* and *trans-*configuration, one of which is characterized as *cis*-configuration (compound **24a-1**) (45mg, 22.5%) and the other is characterized as *trans*-configuration (compound **24a-2**) (36 mg, 18%). MS obsd. (ESI⁺) [(M+H)⁺]: 489. Separation condition: H₂O (0.1% FA) and ACN on waters sun fire C18 column (flow rate: 25 mL/minute).

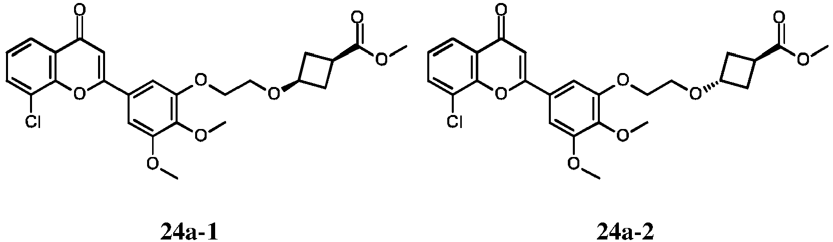

### Step 2. Synthesis of trans-3-[2-[5-(8-chloro-4-oxo-chromen-2-yl)-2,3-dimethoxy-phenoxy]ethoxy]cyclobutanecarboxylic acid

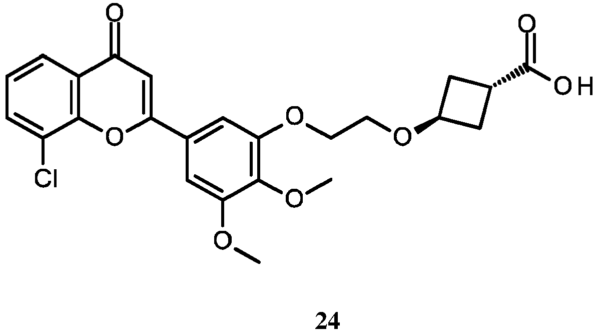

Example **24** was prepared in analogy to the procedure described for the preparation of example **7** by using *trans*-methyl-3-(2-(5-(8-chloro-4-oxo-chromen-2-yl)-2,3-dimethoxyphenoxy)ethoxy)cyclobutane-1-carboxylate (36 mg, compound **24a-2**) as the starting material instead of methyl 3-[2-chloro-5-(8-chloro-4-*oxo*-chromen-2-yl)-4-methoxy-phenoxy]cyclobutanecarboxylate (compound **7c**) in **Step 4.** *Trans-*3-[2-[5-(8-chloro-4-*oxo-*chromen-2-yl)-2,3-dimethoxy-phenoxy]ethoxy]cyclobutanecarboxylic acid (1.8 mg, 4.5 % yield, Example **24**) was obtained as an off-white foam. ¹H NMR (400 MHz, DMSO-*d₆*) δ ppm 12.17 (br s, 1H), 8.0-8.1 (m, 2H), 7.51 (s, 1H), 7.43 (d, *J* = 2.3 Hz, 2H), 7.28 (s, 1H), 4.2-4.3 (m, 3H), 3.91 (s, 3H), 3.80 (s, 3H), 3.6-3.7 (m, 2H), 2.8-3.0 (m, 1H), 2.4-2.4 (m, 2H), 2.1-2.2 (m, 2H). MS obsd. (ESI⁺) [(M+H)⁺]: 475.

### Example 25

### 2-[5-(8-Chloro-4-oxo-chromen-2-yl)-2,3-dimethoxy-phenoxy]acetic acid

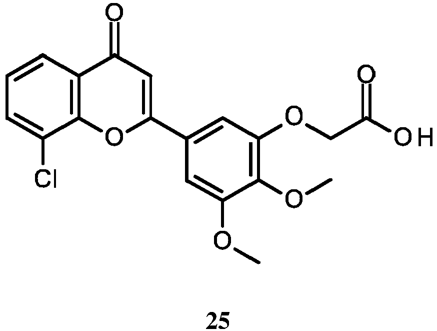

### Step 1. Synthesis of ethyl 2-(5-(8-chloro-4-oxo-chromen-2-yl)-2,3-dimethoxyphenoxy)acetate

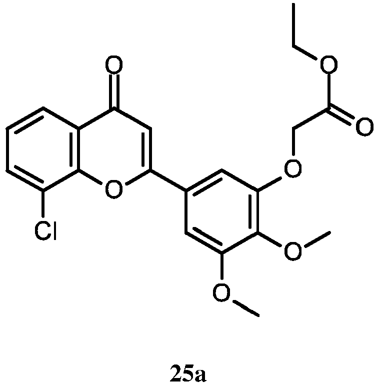

Compound **25a** was prepared in analogy to the procedure described for the preparation of example **1** by using 8-chloro-2-(3-hydroxy-4,5-dimethoxyphenyl)-chromen-4-one **(compound 23b**) and ethyl 2-bromoacetate as the starting material instead of 2-(4-bromo-5-hydroxy-2-methoxy-phenyl)-8-chloro-chromen-4-one (compound **1c)** and *cis-tert-*butyl 3*-*[2-(*p-*tolylsulfonyloxy)ethoxy]cyclobutanecarboxylate (Intermediate **AP**) in **Step 4.** Ethyl 2-(5-(8-chloro-4-*oxo*-chromen-2-yl)-2,3-dimethoxyphenoxy)acetate (240 mg, 95.3 % yield, compound **25a**) was obtained as a yellow solid, which was used in the next step directly. MS obsd. (ESI⁺) [(M+H)⁺]: 405.

### Step 2. Synthesis of 2-[5-(8-chloro-4-oxo-chromen-2-yl)-2,3-dimethoxy-phenoxy]acetic acid

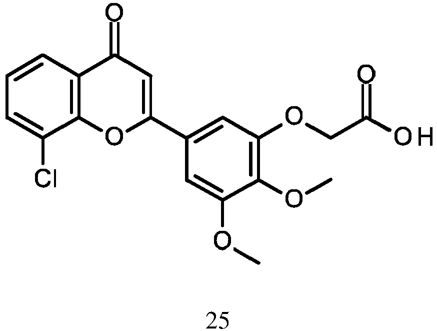

Example **25** was prepared in analogy to the procedure described for the preparation of example **7** by using ethyl 2-(5-(8-chloro-4-*oxo*-chromen-2-yl)-2,3-dimethoxyphenoxy)acetate (compound **25a**) as the starting material instead of methyl 3-[2-chloro-5-(8-chloro-4-*oxo-*chromen-2-yl)-4-methoxy-phenoxy]cyclobutanecarboxylate (compound 7c) in Step **4.** 2-[5-(8-Chloro-4-*oxo*-chromen-2-yl)-2,3-dimethoxy-phenoxy]acetic acid (83.2 mg, 56 % yield, example **25**) was obtained as a yellow foam. ¹H NMR (400 MHz, DMSO-*d₆*) δ ppm 13.11 (br s, 1H), 8.00 (ddd, *J* = 1.5, 6.7, 8.0 Hz, 2H), 7.4-7.5 (m, 2H), 7.36 (d, *J* = 2.0 Hz, 1H), 7.28 (s, 1H), 4.86 (s, 2H), 3.91 (s, 3H), 3.82 (s, 3H). MS obsd. (ESI⁺) [(M+H)⁺]: 391.

### Example 26

### Trans-3-[4-bromo-5-(8-chloro-4-oxo-chromen-2-yl)-2-methoxy-phenoxy]cyclobutanecarboxylic acid

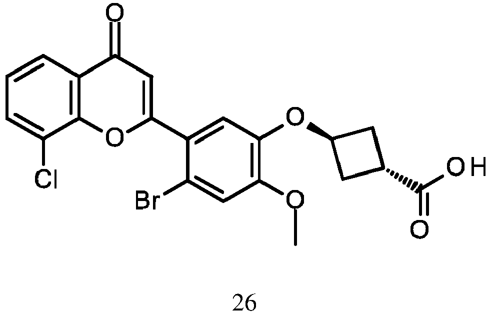

### Step 1. Synthesis of (E)-3-(2-bromo-5-hydroxy-4-methoxy-phenyl)-1-(3-chloro-2-hydroxyphenyl)prop-2-en-1-one

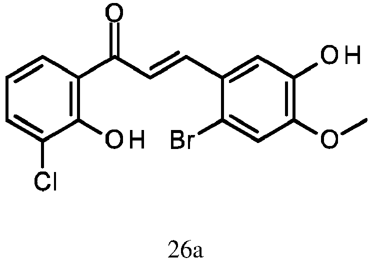

Compound **26a** was prepared in analogy to the procedure described for the preparation of example **1** by using 2-bromo-5-hydroxy-4-methoxybenzaldehyde as the starting material instead of 4-bromo-2-methoxy-5-[(4-methoxyphenyl)methoxy]benzaldehyde (Intermediate **AA**) in **Step 1.** (*E*)-3-(2-Bromo-5-hydroxy-4-methoxy-phenyl)-1-(3-chloro-2-hydroxy-phenyl)prop-2-en-1-one (4.3g, 95.6% yield, compound **26a**) was obtained as a yellow solid, which was used in the next step without further purification. MS obsd. (ESI⁺) [(M+H)⁺]: 383.

### Step 2. Synthesis of 2-(2-bromo-5-hydroxy-4-methoxy-phenyl)-8-chloro-chromen-4-one

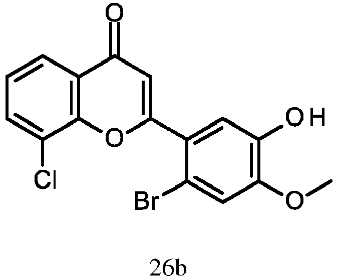

Compound **26b** was prepared in analogy to the procedure described for the preparation of example **1** by (*E*)-3-(2-bromo-5-hydroxy-4-methoxyphenyl)-1-(3-chloro-2-hydroxyphenyl)prop-2-en-1-one (compound **26a**) as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2.** 2-(2-Bromo-5-hydroxy-4-methoxy-phenyl)-8-chloro-chromen-4-one (1.7g, 85.4% yield, compound **26b**) was obtained as a yellow solid. (MS obsd. (ESI⁺) [(M+H)⁺]: 382.

### Step 3. Synthesis of methyl 3-[4-bromo-5-(8-chloro-4-oxo-chromen-2-yl)-2-methoxy-phenoxy]cyclobutanecarboxylate

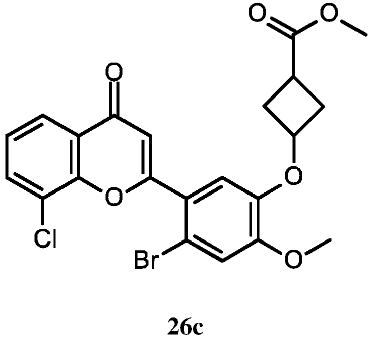

Compound **26c** was prepared in analogy to the procedure described for the preparation of example **1** by using 2-(2-bromo-5-hydroxy-4-methoxy-phenyl)-8-chloro-chromen-4-one (1.7g, compound **26b**) and methyl 3-chlorocyclobutane-1-carboxylate as the starting material instead of 2-(4-bromo-5-hydroxy-2-methoxy-phenyl)-8-chloro-chromen-4-one (compound **1c**) and *cis-tert*-butyl 3-[2-(*p*-tolylsulfonyloxy)ethoxy]cyclobutanecarboxylate (Intermediate **AP**) in **Step 4.** Methyl 3-(4-bromo-5-(8-chloro-4-*oxo*-chromen-2-yl)-2-methoxyphenoxy)cyclobutane-1-carboxylate (compound **26c**) was obtained as a yellow solid.

Separation of compound **26c** with Semi-preparative HPLC give methyl 3-[4-bromo-5-(8-chloro-4-*oxo*-chromen-2-yl)-2-methoxy-phenoxy]cyclobutanecarboxylate with *cis-* and *trans-*configuration, one of which is characterized as *cis*-configuration (compound **26c-1,113** mg, 35.8%) and the other is characterized as *trans*-configuration (compound **26c-2,** 55 mg, 17.4%). MS obsd. (ESI⁺) [(M+H)⁺]: 493. (Separation condition: H₂O (10 mM ammonium hydrogen carbonate) and ACN on YMC-Triart Prep C18, 150 × 40.0 mm, 7µM (flow rate: 60 mL/min)

### Step 4. Synthesis of trans-3-[4-bromo-5-(8-chloro-4-oxo-chromen-2-yl)-2-methoxy-phenoxy]cyclobutanecarboxylic acid

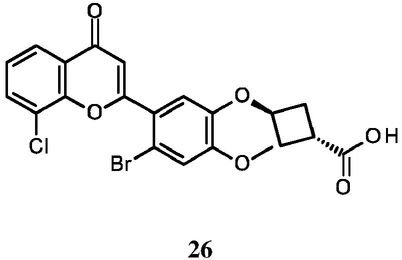

Example **26** was prepared in analogy to the procedure described for the preparation of example **7** by using *trans*-methyl-3-(4-bromo-5-(8-chloro-4-oxo-chromen-2-yl)-2-methoxyphenoxy)cyclobutane-1-carboxylate (45 mg, compound **26c-2**) as the starting material instead of methyl 3-[2-chloro-5-(8-chloro-4-*oxo*-chromen-2-yl)-4-methoxy-phenoxy]cyclobutanecarboxylate (compound 7c) in Step 4. *Trans*-3-[4-bromo-5-(8-chloro-4-*oxo*-chromen-2-yl)-2-methoxy-phenoxy]cyclobutanecarboxylic acid (39.7 mg, 90.8% yield, example **26**) was obtained as an off-white solid. ¹H NMR (400 MHz, DMSO-*d₆*) δ ppm 12.25 (br s, 1H), 8.0-8.1 (m, 2H), 7.5-7.6 (m, 1H), 7.38 (s, 1H), 7.19 (s, 1H), 6.72 (s, 1H), 4.8-5.0 (m, 1H), 3.89 (s, 3H), 3.0-3.1 (m, 1H), 2.6-2.7 (m, 2H), 2.3-2.4 (m, 2H). MS obsd. (ESI⁺) [(M+H)⁺]: 480.

### Example 27

### Trans-3-[2-[4-Bromo-5-(8-chloro-4-oxo-chromen-2-yl)-2-methoxy-phenoxy]ethoxy]cyclobutanecarboxylic acid

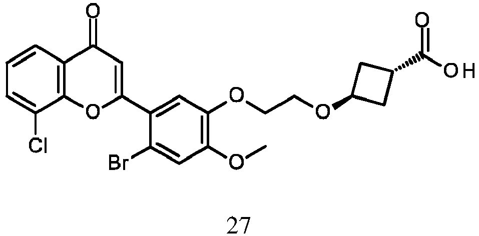

### Step 1. Synthesis of methyl 3-[2-[4-bromo-5-(8-chloro-4-oxo-chromen-2-yl)-2-methoxy-phenoxy]ethoxy]cyclobutanecarboxylate

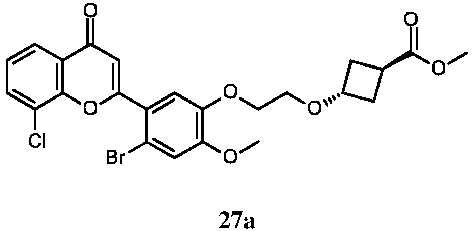

Compound **27a** was prepared in analogy to the procedure described for the preparation of example **1** by using 2-(2-bromo-5-hydroxy-4-methoxyphenyl)-8-chloro-chromen-4-one and *trans*-methyl 3-[2-(*p*-tolylsulfonyloxy)ethoxy]cyclobutanecarboxylate as the starting material instead of 2-(4-bromo-5-hydroxy-2-methoxy-phenyl)-8-chloro-chromen-4-one (compound **1c**) and *cis-tert*-butyl 3-[2-(*p*-tolylsulfonyloxy)ethoxy]cyclobutanecarboxylate (Intermediate **AP**) in **Step 4.** Methyl 3-[2-[4-bromo-5-(8-chloro-4-*oxo*-chromen-2-yl)-2-methoxy-phenoxy]ethoxy]cyclobutanecarboxylate (250 mg, compound **27a**) was obtained as a yellow oil and the residue was purified by preparative HPLC to give *trans*-methyl-3-(2-(4-bromo-5-(8-chloro-4-*oxo*-chromen-2-yl)-2-methoxyphenoxy)ethoxy)cyclobutane-1-carboxylate (52 mg, compound **27a**) was obtained as a light yellow solid. MS obsd. (ESI⁺) [(M+H)⁺]: 538.

### Step 2. Synthesis of trans-3-[2-[4-bromo-5-(8-chloro-4-oxo-chromen-2-yl)-2-methoxyphenoxy]ethoxy]cyclobutanecarboxylic acid

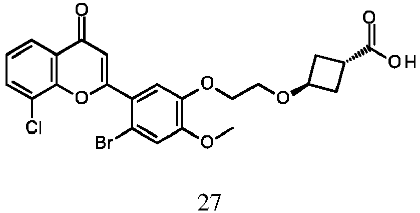

Example **27** was prepared in analogy to the procedure described for the preparation of example **7** by using trans-methyl-3-(2-(4-bromo-5-(8-chloro-4-*oxo*-chromen-2-yl)-2-methoxyphenoxy)ethoxy)cyclobutane-1-carboxylate (52 mg, compound **28a**)as the starting material instead of methyl 3-[2-chloro-5-(8-chloro-4-*oxo*-chromen-2-yl)-4-methoxy-phenoxy]cyclobutanecarboxylate (compound **7c**) in Step 4. *Trans*-3-[2-[4-bromo-5-(8-chloro-4-*oxo*-chromen-2-yl)-2-methoxy-phenoxy]ethoxy]cyclobutanecarboxylic acid (27.4 mg, example **27**) was obtained as an off-white foam. ¹H NMR (400 MHz, DMSO-*d₆*) δ ppm 12.16 (br d, 1H, *J* = 0.6 Hz), 8.0-8.1 (m, 2H), 7.5-7.6 (m, 1H), 7.38 (d, 2H, *J* = 7.9 Hz), 6.71 (s, 1H), 4.14 (br d, 3H, *J* = 5.9 Hz), 3.89 (s, 3H), 3.65 (br s, 2H), 2.8-3.0 (m, 1H), 2.3-2.4 (m, 2H), 2.1-2.2 (m, 2H). MS obsd. (ESI⁺) [(M+H)⁺]: 524.

### Example 28

### 3-[3-(8-Chloro-4-oxo-chromen-2-yl)-2-methoxy-phenoxy]cyclobutanecarboxylic acid

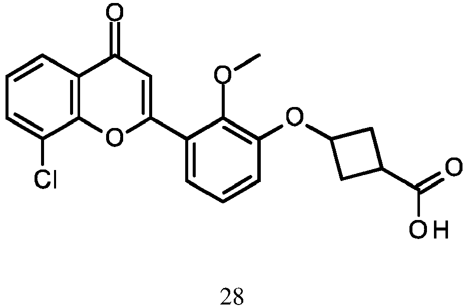

### Step 1. Synthesis of (E)-1-(3-chloro-2-hydroxy-phenyl)-3-(3-hydroxy-2-methoxyphenyl)prop-2-en-1-one

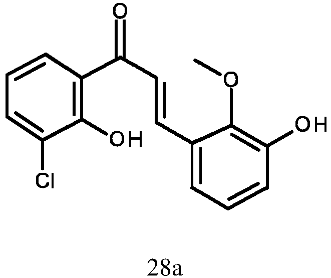

Compound **28a** was prepared in analogy to the procedure described for the preparation of example **1** by using 3-[*tert*-butyl(dimethyl)silyl]oxy-2-methoxy-benzaldehyde (Intermediate **AD** ) as the starting material instead of 4-bromo-2-methoxy-5-[(4-methoxyphenyl)methoxy]benzaldehyde (Intermediate **AA**) in **Step 1.** (*E*)-1-(3-chloro-2-hydroxyphenyl)-3-(3-hydroxy-2-methoxy-phenyl)prop-2-en-1-one (1.0 g, 82% yield, compound **28a**) was obtained as a yellow solid, which was used for next step without purification. MS obsd. (ESI⁺): 305.1 [(M+H)⁺], 307.1 [(M+2+H)⁺].

### Step 2. Synthesis of 8-chloro-2-(3-hydroxy-2-methoxy-phenyl)chromen-4-one

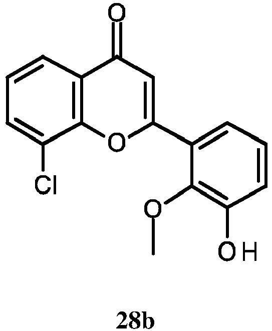

Compound **28b** was prepared in analogy to the procedure described for the preparation of example **1** by using (*E*)-1-(3-chloro-2-hydroxy-phenyl)-3-(3-hydroxy-2-methoxy-phenyl)prop-2-en-1-one (compound **28a**) was obtained as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2**. 8-Chloro-2-(3-hydroxy-2-methoxy-phenyl)chromen-4-one (180 mg, 52% yield, compound **28b**) was obtained as a yellow solid. MS obsd. (ESI⁺): 303.0 [(M+H)⁺], 305.1 [(M+2+H)⁺],

### Step 3. Synthesis of methyl 3-[3-(8-chloro-4-oxo-chromen-2-yl)-2-methoxy-phenoxy]cyclobutanecarboxylate

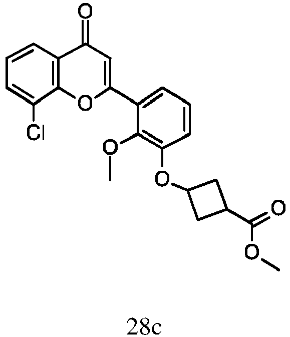

Compound **28c** was prepared in analogy to the procedure described for the preparation of example 1 by using 8-chloro-2-(3-hydroxy-2-methoxy-phenyl)chromen-4-one (180 mg, compound **28b**) and methyl 3-(*p*-tolylsulfonyloxy)cyclobutanecarboxylate as the starting material instead of 2-(4-bromo-5-hydroxy-2-methoxy-phenyl)-8-chloro-chromen-4-one (compound **1c**) and *cis-tert*-butyl 3-[2-(*p*-tolylsulfonyloxy)ethoxy]cyclobutanecarboxylate (Intermediate **AP)** in **Step 4.** Methyl 3-[3-(8-chloro-4-*oxo*-chromen-2-yl)-2-methoxy-phenoxy]cyclobutanecarboxylate (65 mg, 47.43% yield, compound **28c**) was obtained as a yellow oil. MS obsd. (ESI⁺): 415.1 [(M+H)⁺], 417.0 [(M+2+H)⁺].

### Step 4. Synthesis of 3-[3-(8-chloro-4-oxo-chromen-2-yl)-2-methoxy-phenoxy]cyclobutanecarboxylic acid

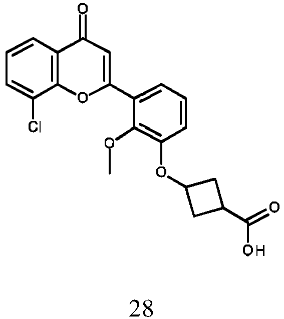

Example **28** was prepared in analogy to the procedure described for the preparation of example **7** by using methyl 3-[3-(8-chloro-4-*oxo*-chromen-2-yl)-2-methoxy-phenoxy]cyclobutanecarboxylate (compound **28c**) was obtained as the starting material instead of methyl 3-[2-chloro-5-(8-chloro-4-*oxo*-chromen-2-yl)-4-methoxy-phenoxy]cyclobutanecarboxylate (compound 7c) in **Step 4.** 3-[3-(8-Chloro-4-*oxo*-chromen-2-yl)-2-methoxy-phenoxy]cyclobutanecarboxylic acid (5 mg, 10% yield, example **28**) was obtained as a white solid. ¹H NMR (400 MHz, MeOD) δ ppm: 8.10 (dd, *J* = 8.0, 1.5 Hz, 1H), 7.92 (dd, *J* = 7.8, 1.5 Hz, 1H), 7.54 (dd, *J* = 8.0, 1.4 Hz, 1H), 7.48 (t, *J* = 7.9 Hz, 1H), 7.23 (t, *J* = 8.1 Hz, 1H), 7.09 (s, 1H), 7.07 (dd, *J* = 8.2, 1.3 Hz, 1H), 4.96-5.03 (m, 1H), 3.97 (s, 3H), 3.17-3.23 (m, 1H), 2.79 (ddd, *J* = 13.4, 7.0, 4.3 Hz, 2H), 2.48-2.58 (m, 2H). MS obsd. (ESI⁺): 401.0 [(M+H)⁺], 403.0 [(M+2+H)⁺].

### Example 29

### Cis-3-[2-[3-(8-chloro-4-oxo-chromen-2-yl)-2-methoxy-phenoxy]ethoxy]cyclobutanecarboxylic acid

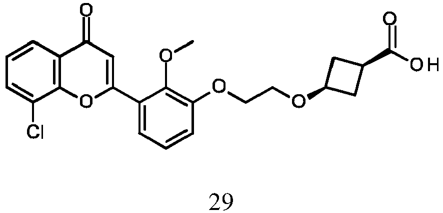

### Step 1. Synthesis of tert-butyl 3-[2-[3-(8-chloro-4-oxo-chromen-2-yl)-2-methoxy-phenoxy]ethoxy]cyclobutanecarboxylate

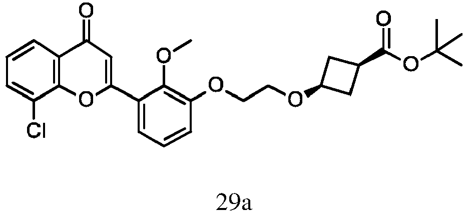

Compound **29a** was prepared in analogy to the procedure described for the preparation of example **1** by using 8-chloro-2-(3-hydroxy-2-methoxy-phenyl)chromen-4-one (compound **28b**) as the starting material instead of 2-(4-bromo-5-hydroxy-2-methoxy-phenyl)-8-chloro-chromen-4-one (compound **1c**) in **Step 4**. *Tert*-butyl 3-[2-[3-(8-chloro-4-*oxo*-chromen-2-yl)-2-methoxy-phenoxy]ethoxy]cyclobutanecarboxylate (150 mg, compound **29a**) was obtained as a yellow oil. MS obsd. (ESI⁺): 501.1 [(M+H)⁺], 503.1 [(M+2+H)⁺],

### Step 2. Synthesis of cis-3-[2-[3-(8-chloro-4-oxo-chromen-2-yl)-2-methoxy-phenoxy]ethoxy]cyclobutanecarboxylic acid

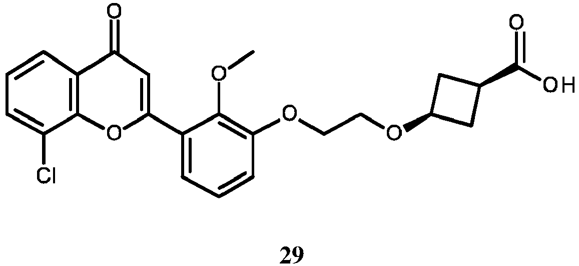

Example **29** was prepared in analogy to the procedure described for the preparation of example **1** by using *tert*-butyl 3-[2-[3-(8-chloro-4-*oxo*-chromen-2-yl)-2-methoxy-phenoxy]ethoxy]cyclobutanecarboxylate (compound **29a**) was obtained as the starting material instead of *tert*-butyl 3-[2-[2-bromo-5-(8-chloro-4-*oxo*-chromen-2-yl)-4-methoxy-phenoxy]ethoxy]cyclobutanecarboxylate (compound **1d)** in **Step 5.** *Cis*-3-[2-[3-(8-Chloro-4-*oxo-*chromen-2-yl)-2-methoxy-phenoxy]ethoxy]cyclobutanecarboxylic acid (80 mg, 0.180 mmol, 60% yield, example **29**) was obtained as a white solid. ¹H NMR (400 MHz, MeOD) δ ppm: 8.09 (dd, *J* = 8.0, 1.4 Hz, 1H), 7.92 (dd, *J* = 7.8, 1.5 Hz, 1H), 7.55 (dd, *J* = 7.5, 1.9 Hz, 1H), 7.47 (t, *J* = 7.9 Hz, 1H), 7.22-7.30 (m, 2H), 7.11 (s, 1H), 4.19-4.25 (m, 2H), 4.03-4.08 (m, 1H), 3.99 (s, 3H), 3.76-3.82 (m, 2H), 2.67 (dd, *J* = 16.8, 8.8 Hz, 1H), 2.55 (ddd, *J* = 14.8, 7.4, 2.7 Hz, 2H), 2.12-2.21 (m, 2H). MS obsd. (ESI⁺): 445.1 [(M+H)⁺], 447.1 [(M+2+H)⁺].

### Example 30

### 3-[3-(8-Chloro-4-oxo-chromen-2-yl)-4-methoxy-phenoxy]cyclobutanecarboxylic acid

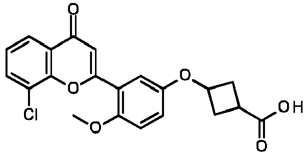

### 30Step 1. Synthesis of (E)-1-(3-chloro-2-hydroxy-phenyl)-3-(4-hydroxy-2-methoxyphenyl)prop-2-en-1-one

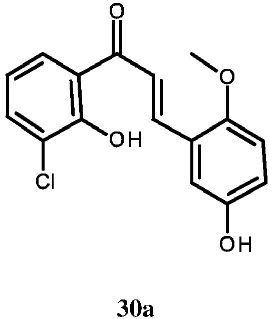

Compound **30a** was prepared in analogy to the procedure described for the preparation of example **1** by using 5-hydroxy-2-methoxy-benzaldehyde (Intermediate **AE**) as the starting material instead of 4-bromo-2-methoxy-5-[(4-methoxyphenyl)methoxy]benzaldehyde (Intermediate **AA**) in **Step 1.** (*E*)-1-(3-chloro-2-hydroxy-phenyl)-3-(4-hydroxy-2-methoxyphenyl)prop-2-en-1-one (300 mg, 28% yield, compound **30a**) was obtained as a yellow oil. MS obsd. (ESI⁺): 305.1 [(M+H)⁺], 307.1 [(M+2+H)⁺],

### Step 2. Synthesis of 8-chloro-2-(5-hydroxy-2-methoxy-phenyl)chromen-4-one

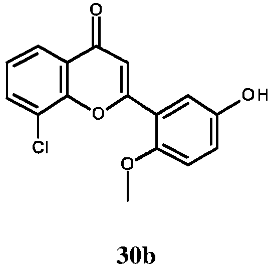

Compound **30b** was prepared in analogy to the procedure described for the preparation of example **1** by using (*E*)-1-(3-chloro-2-hydroxy-phenyl)-3-(4-hydroxy-2-methoxy-phenyl)prop-2-en-1-one (compound **30a**) as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2.** 8-Chloro-2-(5-hydroxy-2-methoxy-phenyl)chromen-4-one (170 mg, 67% yield, compound **31b**) was obtained as a yellow solid. MS obsd. (ESI⁺): 303.1 [(M+H)⁺], 305.0 [(M+2+H)⁺],

### Step 3. Synthesis of methyl 3-[3-(8-chloro-4-oxo-chromen-2-yl)-4-methoxy-phenoxy]cyclobutanecarboxylate

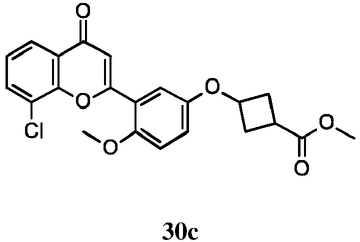

Compound **30c** was prepared in analogy to the procedure described for the preparation of example **1** by using 8-chloro-2-(5-hydroxy-2-methoxy-phenyl)chromen-4-one (compound **30b**) and methyl 3-(*p*-tolylsulfonyloxy)cyclobutanecarboxylate as the starting material instead of 2-(4-bromo-5-hydroxy-2-methoxy-phenyl)-8-chloro-chromen-4-one (compound **1c**) and *cis-tert-butyl* 3-[2-(*p*-tolylsulfonyloxy)ethoxy]cyclobutanecarboxylate (Intermediate **AP**) in **Step** 4. Methyl 3-[3-(8-chloro-4-*oxo*-chromen-2-yl)-4-methoxy-phenoxy]cyclobutanecarboxylate (100 mg, 38% yield, compound **30c**) was obtained as a yellow solid.MS obsd. (ESI⁺): 415.1 [(M+H)⁺], 417.1 [(M+2+H)⁺].

### Step 4. Synthesis of 3-[3-(8-chloro-4-oxo-chromen-2-yl)-4-methoxy-phenoxy]cyclobutanecarboxylic acid

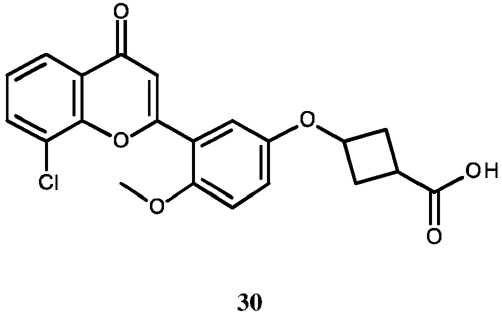

Compound **30** was prepared in analogy to the procedure described for the preparation of example **7** by using methyl 3-[3-(8-chloro-4-*oxo*-chromen-2-yl)-4-methoxy-phenoxy]cyclobutanecarboxylate (compound **30c**) was obtained as the starting material instead of methyl 3-[2-chloro-5-(8-chloro-4-*oxo*-chromen-2-yl)-4-methoxy-phenoxy]cyclobutanecarboxylate (compound **7c**) in **Step 4.** 3-[3-(8-Chloro-4-oxo-chromen-2-yl)-4-methoxy-phenoxy]cyclobutanecarboxylic acid (53 mg, 67% yield, example **30**) was obtained as a yellow solid. ¹H NMR (400 MHz, DMSO-*d₆*) δ ppm: 12.34 (s, 1H), 8.01 (t, *J* = 7.4 Hz, 2H), 7.48-7.55 (m, 2H), 7.23 (d, *J* = 8.8 Hz, 2H), 7.16 (s, 1H), 7.10 (d, *J* = 8.9 Hz, 1H), 4.83 -4.92 (m, 1H), 3.92 (s, 3H), 3.02-3.15 (m, 1H), 2.65-2.75 (m, 2H), 2.25-2.39 (m, 2H). MS obsd. (ESI⁺): 401.0 [(M+H)⁺], 403.0 [(M+2+H)⁺].

### Example 31

### Cis-3-[2-[3-(8-Chloro-4-oxo-chromen-2-yl)-4-methoxy-phenoxy]ethoxy]cyclobutanecarboxylic acid

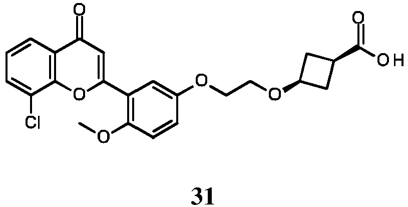

### Step 1. Synthesis of cis-tert-butyl 3-[2-[3-(8-chloro-4-oxo-chromen-2-yl)-4-methoxy-phenoxy]ethoxy]cyclobutanecarboxylate

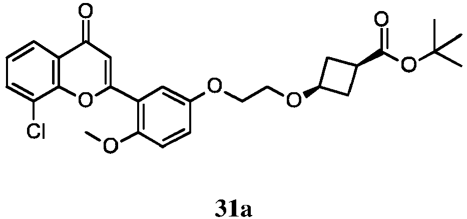

Compound **31a** was prepared in analogy to the procedure described for the preparation of example **1** by using 8-chloro-2-(5-hydroxy-2-methoxy-phenyl)chromen-4-one (compound **30b**) as the starting material instead of 2-(4-bromo-5-hydroxy-2-methoxy-phenyl)-8-chloro-chromen-4-one (compound **1c**) in Step **4**. *cis-tert*-Butyl 3-[2-[3-(8-chloro-4-oxo-chromen-2-yl)-4-methoxy-phenoxy]ethoxy]cyclobutanecarboxylate (90 mg, 80% yield, compound **31a**) was obtained as a yellow oil. MS obsd. (ESI⁺): 501.2 [(M+H)⁺], 503.1 [(M+2+H)⁺],

### Step 2. Synthesis of cis-3-[2-[3-(8-chloro-4-oxo-chromen-2-yl)-4-methoxy-phenoxy]ethoxy]cyclobutanecarboxylic acid

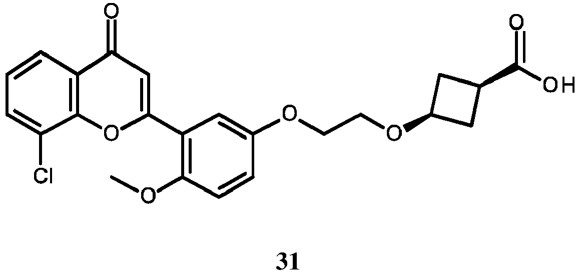

Example **31** was prepared in analogy to the procedure described for the preparation of example 1 by using *tert*-butyl 3-[2-[3-(8-chloro-4-*oxo*-chromen-2-yl)-4-methoxy-phenoxy]ethoxy]cyclobutanecarboxylate (compound **31a**) as the starting material instead of *tert-*butyl 3-[2-[2-bromo-5-(8-chloro-4-*oxo*-chromen-2-yl)-4-methoxy-phenoxy]ethoxy]cyclobutanecarboxylate (compound **1d)** in **Step 5.** *Cis*-3-[2-[3-(8-chloro-4-*oxo-*chromen-2-yl)-4-methoxy-phenoxy]ethoxy]cyclobutanecarboxylic acid (52.5 mg, 65% yield, example **31**) was obtained as a yellow solid. ¹H NMR (400 MHz, MeOD) *δ* ppm: 7.99-8.02 (m, 1H), 7.82 -7.90 (m, 1H), 7.60-7.70 (m, 1H), 7.40-7.50 (m, 1H), 7.20-7.31 (m, 1H), 7.10-7.15 (m, 2H), 4.10-4.15 (m, 2H), 3.95-4.10 (m, 1H), 3.94 (d, *J* = 2.5 Hz, 3H), 3.71-3.78 (m, 2H), 2.66 (dd, *J* = 16.8, 8.8 Hz, 1H), 2.50 -2.59 (m, 2H), 2.11-2.22 (m, 2H). MS obsd. (ESI⁺): 445.1 [(M+H)⁺], 447.1 [(M+2+H)⁺].

### Example 32

### 2-[3-(8-Chloro-4-oxo-chromen-2-yl)-4-methoxy-phenoxy]acetic acid

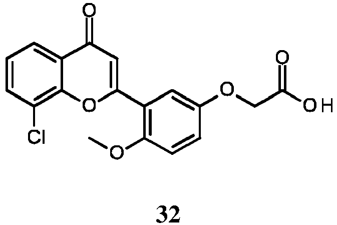

### Step 1. Synthesis of tert-butyl 2-[3-(8-chloro-4-oxo-chromen-2-yl)-4-methoxy-phenoxy]acetate

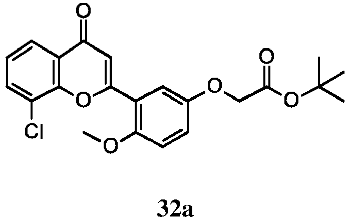

Compound **32a** was prepared in analogy to the procedure described for the preparation of example **1** by using 8-chloro-2-(5-hydroxy-2-methoxy-phenyl)chromen-4-one (compound **31b**) and *tert*-butyl bromoacetate as the starting material instead of 2-(4-bromo-5-hydroxy-2-methoxyphenyl)-8-chloro-chromen-4-one (compound **1c**) and *cis-tert-*butyl 3-[2-(*p-*tolylsulfonyloxy)ethoxy]cyclobutanecarboxylate (Intermediate **AP**) in **Step 4**. *Tert*-butyl 2-[3-(8-chloro-4-oxo-chromen-2-yl)-4-methoxy-phenoxy]acetate (70 mg, 72% yield, compound **32a**) was obtained as a yellow solid. MS obsd. (ESI⁺): 417.1 [(M+H)⁺], 419.1 [(M+2+H)⁺].

### Step 2. Synthesis of 2-[3-(8-chloro-4-oxo-chromen-2-yl)-4-methoxy-phenoxy]acetic acid

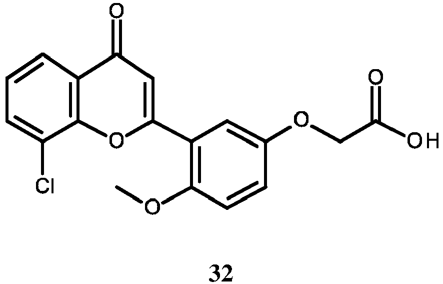

Example **32** was prepared in analogy to the procedure described for the preparation of example **1** by using *tert*-butyl2-[3-(8-chloro-4-*oxo*-chromen-2-yl)-4-methoxy-phenoxy]acetate (compound **32a**) was obtained as the starting material instead of *tert*-butyl 3-[2-[2-bromo-5-(8-chloro-4-*oxo*-chromen-2-yl)-4-methoxy-phenoxy]ethoxy]cyclobutanecarboxylate (compound **1d)** in **Step 5.** 2-[3-(8-Chloro-4-*oxo*-chromen-2-yl)-4-methoxy-phenoxy]acetic acid (45 mg, 74% yield, example **32**) was obtained as a yellow solid. ¹H NMR (400 MHz, DMSO-*d₆*) δ ppm 13.06 (s, 1H), 7.97-8.04 (m, 2H), 7.47-7.55 (m, 1H), 7.21 (dt, *J* = 9.1, 6.1 Hz, 1H), 7.12 (s, 1H), 4.73 (s, 2H), 3.92 (s, 3H). MS obsd. (ESI⁺): 361.0 [(M+H)⁺], 363.0 [(M+2+H)⁺].

### Example 33

### 3-[3-(8-Chloro-4-oxo-chromen-2-yl)-4-methoxy-phenoxy]propanoic acid

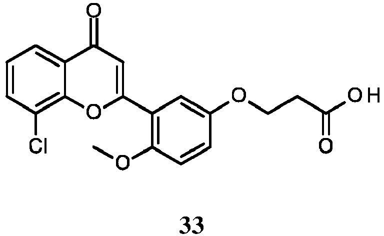

Example **33** was prepared in analogy to the procedure described for the preparation of example **2** by using 8-chloro-2-(5-hydroxy-2-methoxy-phenyl)chromen-4-one (compound **31b**) as the starting material instead of 2-(4-bromo-5-hydroxy-2-methoxy-phenyl)-8-chloro-chromen-4-one (compound **1c**). 3-[3-(8-Chloro-4-oxo-chromen-2-yl)-4-methoxy-phenoxy]propanoic acid (6 mg, 3% yield, example **33**) was obtained as a yellow solid.¹H NMR (400 MHz, DMSO-*d₆*) δ ppm 12.35 (s, 1H), 7.99-7.82 (m, 2H), 7.54 (d, *J* = 2.1 Hz, 1H), 7.50 (t, *J* = 7.9 Hz, 1H), 7.17-7.26 (m, 2H), 7.10 (s, 1H), 4.22 (t, *J* = 6.1 Hz, 2H), 3.91 (s, 3H), 2.72 (t, *J* = 6.0 Hz, 2H). MS obsd. (ESI⁺): 375.1 [(M+H)⁺], 377.1 [(M+2+H)⁺].

### Example 34

### Cis-3-[2-[6-bromo-3-(8-chloro-4-oxo-chromen-2-yl)-2-methoxy-phenoxy]ethoxy]cyclobutanecarboxylic acid

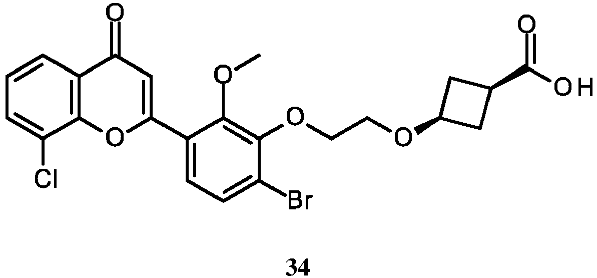

### Step 1. Synthesis of (E)-3-(4-bromo-3-hydroxy-2-methoxy-phenyl)-1-(3-chloro-2-hydroxyphenyl)prop-2-en-1-one

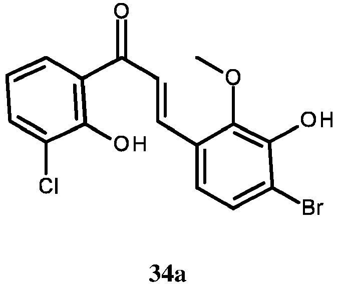

Compound **34a** was prepared in analogy to the procedure described for the preparation of example **1** by using 4-bromo-3-hydroxy-2-methoxy-benzaldehyde (Intermediate **AF**) as the starting material instead of 4-bromo-2-methoxy-5-[(4-methoxyphenyl)methoxy]benzaldehyde (Intermediate **AA**) in **Step 1.** (*E*)-3-(4-Bromo-3-hydroxy-2-methoxy-phenyl)-1-(3-chloro-2-hydroxy-phenyl)prop-2-en-1-one (300 mg, 21% yield, compound **34a**) was obtained as a yellow solid. MS obsd. (ESI⁺): 383.1 [(M+H)⁺], 385.1 [(M+2+H)⁺],

### Step 2. Synthesis of 2-(4-bromo-3-hydroxy-2-methoxy-phenyl)-8-chloro-chromen-4-one

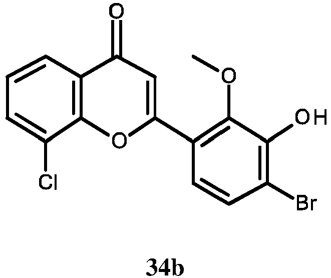

Compound **34b** was prepared in analogy to the procedure described for the preparation of example **1** by using (*E*)-3-(4-bromo-3-hydroxy-2-methoxy-phenyl)-1-(3-chloro-2-hydroxyphenyl)prop-2-en-1-one (compound **34a**) as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2**. 2-(4-Bromo-3-hydroxy-2-methoxy-phenyl)-8-chloro-chromen-4-one (180 mg, 80% yield, compound **34b**) was obtained as a yellow solid. MS obsd. (ESI⁺): 380.9[(M+H)⁺], 382.9[(M+2+H)⁺].

### Step 3. Synthesis of cis-tert-butyl 3-[2-[6-bromo-3-(8-chloro-4-oxo-chromen-2-yl)-2-methoxy-phenoxy]ethoxy]cyclobutanecarboxylate

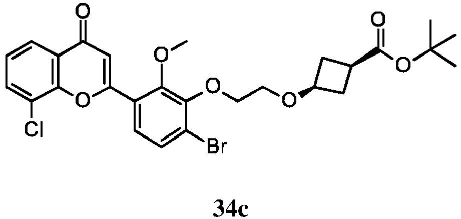

Compound **34c** was prepared in analogy to the procedure described for the preparation of example **1** by 2-(4-bromo-3-hydroxy-2-methoxy-phenyl)-8-chloro-chromen-4-one (180 mg, 80% yield, compound **34b**) as the starting material instead of 2-(4-bromo-5-hydroxy-2-methoxyphenyl)-8-chloro-chromen-4-one (compound **1c**) in **Step 4.** *Cis-tert*-Butyl 3-[2-[6-bromo-3-(8-chloro-4-*oxo*-chromen-2-yl)-2-methoxy-phenoxy]ethoxy]cyclobutanecarboxylate (100 mg, 71% yield, compound **34c**) was obtained as a white solid. MS obsd. (ESI⁺): 579.1 [(M+H)⁺], 581.1 [(M+2+H)⁺],

### Step 4. Synthesis of cis-3-[2-[6-bromo-3-(8-chloro-4-oxo-chromen-2-yl)-2-methoxy-phenoxy]ethoxy]cyclobutanecarboxylic acid

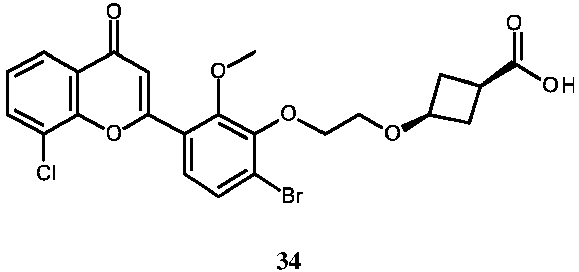

Example **34** was prepared in analogy to the procedure described for the preparation of example **1** by using *cis-tert-*butyl 3-[2-[6-bromo-3-(8-chloro-4-*oxo*-chromen-2-yl)-2-methoxy-phenoxy]ethoxy]cyclobutanecarboxylate (compound **34c**) as the starting material instead of *tert-*butyl 3-[2-[2-bromo-5-(8-chloro-4-*oxo*-chromen-2-yl)-4-methoxy-phenoxy]ethoxy]cyclobutanecarboxylate (compound **1d**) in **Step 5.** *Cis*-3-[2-[6-bromo-3-(8-chloro-4-oxo-chromen-2-yl)-2-methoxy-phenoxy]ethoxy]cyclobutanecarboxylic acid (58 mg, 64% yield, example **34**) was obtained as a white solid. ¹H NMR (400 MHz, DMSO-*d₆*) *δ* ppm 12.08 (s, 1H), 8.03 (d, *J* = 8.2 Hz, 2H), 7.64 (d, *J* = 8.6 Hz, 1H), 7.50-7.61 (m, 2H), 6.93 (s, 1H), 4.11-4.19 (m, 2H), 3.91-4.02 (m, 4H), 3.62-3.72 (m, 2H), 2.57-2.67 (m, 1H), 2.38-2.49 (m, 2H), 1.95 -2.06 (m, 2H). MS obsd. (ESI⁺): 523.0 [(M+H)⁺], 525.0 [(M+2+H)⁺].

### Example 35

### 3-[6-Bromo-3-(8-chloro-4-oxo-chromen-2-yl)-2-methoxy-phenoxy]cyclobutanecarboxylic acid

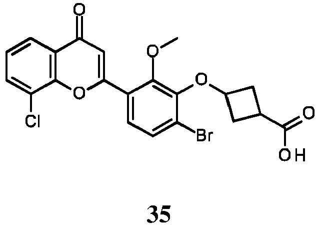

### Step 1. Synthesis of methyl 3-[6-bromo-3-(8-chloro-4-oxo-chromen-2-yl)-2-methoxy-phenoxy]cyclobutanecarboxylate

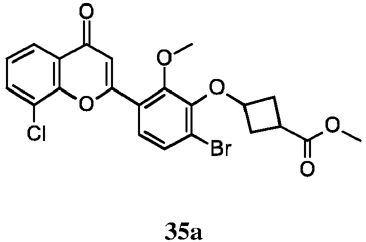

Compound **35a** was prepared in analogy to the procedure described for the preparation of example **1** by using 2-(4-bromo-3-hydroxy-2-methoxy-phenyl)-8-chloro-chromen-4-one (compound **34b**) and methyl 3-(*p*-tolylsulfonyloxy)cyclobutanecarboxylate as the starting material instead of 2-(4-bromo-5-hydroxy-2-methoxy-phenyl)-8-chloro-chromen-4-one (compound **1c**) and *cis-tert*-butyl 3-[2-(*p*-tolylsulfonyloxy)ethoxy]cyclobutanecarboxylate in **Step 4.** Methyl 3-[6-bromo-3-(8-chloro-4-*oxo*-chromen-2-yl)-2-methoxy-phenoxy]cyclobutanecarboxylate (100 mg, 74% yield, compound **35a**) was obtained as a yellow solid. MS obsd. (ESI⁺): 493.0 [(M+H)⁺], 495.0 [(M+2+H)⁺],

### Step 2. Synthesis of 3-[6-bromo-3-(8-chloro-4-oxo-chromen-2-yl)-2-methoxy-phenoxy]cyclobutanecarboxylic acid

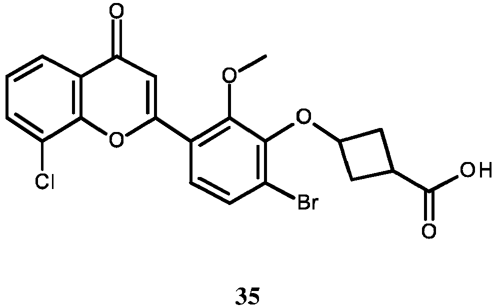

Example **35** was prepared in analogy to the procedure described for the preparation of example **7** by using methyl 3-[6-bromo-3-(8-chloro-4-*oxo*-chromen-2-yl)-2-methoxy-phenoxy]cyclobutanecarboxylate (compound **35a**) as the starting material instead of methyl 3-[2-chloro-5-(8-chloro-4-*oxo*-chromen-2-yl)-4-methoxy-phenoxy]cyclobutanecarboxylate (compound **7c**) in **Step 4.** 3-[6-Bromo-3-(8-chloro-4-*oxo*-chromen-2-yl)-2-methoxy-phenoxy]cyclobutanecarboxylic acid (55 mg, 49% yield, example **35**) obtained as an off-white solid. ¹H NMR (400 MHz, MeOD) *δ* ppm: 8.09 (dd, *J* = 8.0, 1.5 Hz, 1H), 7.92 (dd, *J* = 7.8, 1.5 Hz, 1H), 7.67 (d, *J* = 8.7 Hz, 1H), 7.58 (d, *J* = 8.7 Hz, 1H), 7.48 (t, *J* = 7.9 Hz, 1H), 7.12 (s, 1H), 4.95-5.05 (m, 1H), 3.95 (s, 3H), 3.12-3.19 (m, 1H), 2.56-2.71 (m, 4H). MS obsd. (ESI⁺): 479.0 [(M+H)⁺], 481.0 [(M+2+H)⁺].

### Example 36

### 3-[3-(8-Chloro-4-oxo-chromen-2-yl)-2-methoxy-6-methyl-phenoxy]cyclobutanecarboxylic acid

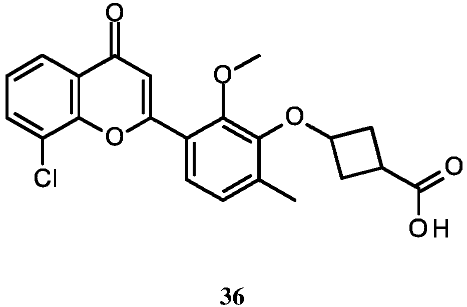

### Step 1. Synthesis of (E)-1-(3-chloro-2-hydroxy-phenyl)-3-[2-methoxy-3-(methoxymethoxy)-4-methyl-phenyl]prop-2-en-1-one

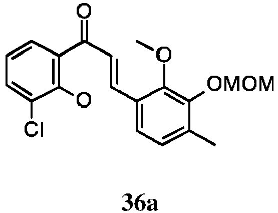

Compound **36a** was prepared in analogy to the procedure described for the preparation of example **1** by using 2-methoxy-3-(methoxymethoxy)-4-methyl-benzaldehyde (Intermediate **AG**) as the starting material instead of 4-bromo-2-methoxy-5-[(4-methoxyphenyl)methoxy]benzaldehyde (Intermediate **AA**) in **Step 1.** (*E*)-1-(3-chloro-2-hydroxyphenyl)-3-[2-methoxy-3-(methoxymethoxy)-4-methyl-phenyl]prop-2-en-1-one (180 mg, compound **36a**) was obtained as a yellow solid, which was used for next step without purification. MS obsd. (ESI⁺): 363.1 [(M+H)⁺], 365.2 [(M+2+H)⁺],

### Step 2. Synthesis of 8-chloro-2-(3-hydroxy-2-methoxy-4-methyl-phenyl)chromen-4-one

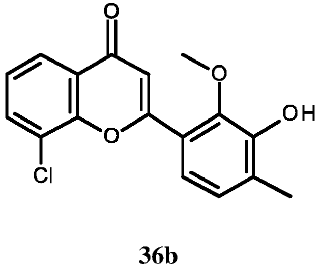

Compound **36b** was prepared in analogy to the procedure described for the preparation of example **1** by using (*E*)-1-(3-chloro-2-hydroxy-phenyl)-3-[2-methoxy-3-(methoxymethoxy)-4-methyl-phenyl]prop-2-en-1-one (compound **36a**) as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2.** 8-Chloro-2-(3-hydroxy-2-methoxy-4-methylphenyl)chromen-4-one (100 mg, 42% yield, compound **36b**) was obtained as a brown solid. MS obsd. (ESI⁺): 317.1 [(M+H)⁺], 319.0 [(M+2+H)⁺],

### Step 3. Synthesis of methyl 3-[3-(8-chloro-4-oxo-chromen-2-yl)-2-methoxy-6-methyl-phenoxy]cyclobutanecarboxylate

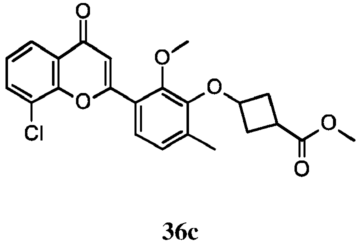

Compound **36c** was prepared in analogy to the procedure described for the preparation of example **1** by using 8-chloro-2-(3-hydroxy-2-methoxy-4-methyl-phenyl)chromen-4-one (compound **36b**) and methyl 3-(*p*-tolylsulfonyloxy)cyclobutanecarboxylate as the starting material instead of 2-(4-bromo-5-hydroxy-2-methoxy-phenyl)-8-chloro-chromen-4-one (compound **1c**) and *cis-tert*-butyl 3-[2-(p-tolylsulfonyloxy)ethoxy]cyclobutanecarboxylate (Intermediate **AP**) in **Step 4.** Methyl 3-[3-(8-chloro-4-*oxo*-chromen-2-yl)-2-methoxy-6-methyl-phenoxy]cyclobutanecarboxylate (70 mg, 77% yield, compound **36c**) was obtained as a yellow oil. MS obsd. (ESI⁺): 429.2 [(M+H)⁺], 431.2 [(M+2+H)⁺].

### Step 4. Synthesis of 3-[3-(8-chloro-4-oxo-chromen-2-yl)-2-methoxy-6-methyl-phenoxy]cyclobutanecarboxylic acid

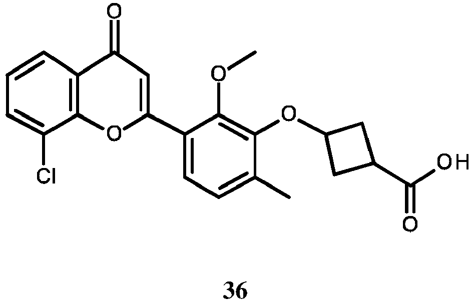

Example **36** was prepared in analogy to the procedure described for the preparation of example **7** by using methyl 3-[3-(8-chloro-4-*oxo*-chromen-2-yl)-2-methoxy-6-methyl-phenoxy]cyclobutanecarboxylate (70 mg, compound **36c**) as the starting material instead of methyl 3-[2-chloro-5-(8-chloro-4-*oxo*-chromen-2-yl)-4-methoxy-phenoxy]cyclobutanecarboxylate (compound **7c**) in **Step 4.** 3-[3-(8-Chloro-4-*oxo*-chromen-2-yl)-2-methoxy-6-methyl-phenoxy]cyclobutanecarboxylic acid (20 mg, 29% yield, example **36**) was obtained as a white solid. ¹H NMR (400 MHz, MeOD) *δ* ppm: 8.09 (dd, *J* = 8.0, 1.5 Hz, 1H), 7.91 (dd, *J* = 7.8, 1.5 Hz, 1H), 7.67 (d, *J* = 8.2 Hz, 1H), 7.47 (t, *J* = 7.9 Hz, 1H), 7.17 (d, *J* = 8.9 Hz, 2H), 4.85-4.89 (m, 1H), 3.89 (s, 3H), 3.09-3.15 (m, 1H), 2.55-2.65 (m, 4H), 2.36 (s, 3H). MS obsd. (ESI⁺): 415.1 [(M+H)⁺], 417.1 [(M+2+H)⁺].

### Example 37

### 2-[6-Chloro-3-(8-chloro-4-oxo-chromen-2-yl)-2-methoxy-phenoxy]acetic acid

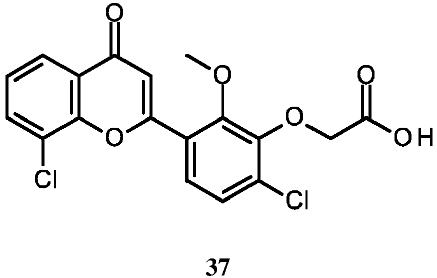

### Step 1. Synthesis of tert-butyl 2-[6-chloro-3-(8-chloro-4-oxo-chromen-2-yl)-2-methoxy-phenoxy]acetate

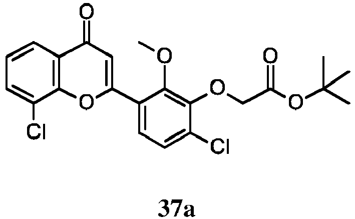

Compound **37a** was prepared in analogy to the procedure described for the preparation of example **1** by using 8-chloro-2-(4-chloro-3-hydroxy-2-methoxy-phenyl)chromen-4-one and *tert-*butyl bromoacetate as the starting material instead of 2-(4-bromo-5-hydroxy-2-methoxy-phenyl)-8-chloro-chromen-4-one (compound **1c**) and *cis-tert*-butyl 3-[2-(*p-*tolylsulfonyloxy)ethoxy]cyclobutanecarboxylate in **Step 4.** *Tert*-butyl 2-[6-chloro-3-(8-chloro-4-oxo-chromen-2-yl)-2-methoxy-phenoxy]acetate (65 mg, 46% yield, compound **37a**) was obtained as a yellow oil. MS obsd. (ESI⁺): 451.0 [(M+H)⁺], 453.1 [(M+2+H)⁺],

### Step 2. Synthesis of 2-[6-chloro-3-(8-chloro-4-oxo-chromen-2-yl)-2-methoxy-phenoxy]acetic acid

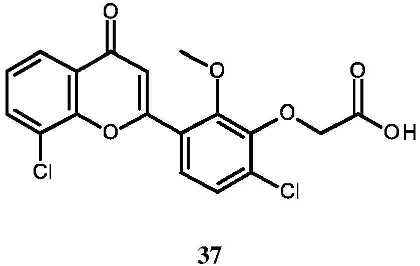

Example **37** was prepared in analogy to the procedure described for the preparation of example **1** by using *tert*-butyl 2-[6-chloro-3-(8-chloro-4-*oxo*-chromen-2-yl)-2-methoxy-phenoxy]acetate (65 mg, compound **37a**) was obtained as the starting material instead of *tert-*butyl 3-[2-[2-bromo-5-(8-chloro-4-*oxo*-chromen-2-yl)-4-methoxy-phenoxy]ethoxy]cyclobutanecarboxylate (compound **1d**) in **Step 5.** 2-[6-Chloro-3-(8-chloro-4-oxo-chromen-2-yl)-2-methoxy-phenoxy]acetic acid (50 mg, 87% yield, Example **37**) was obtained as a white solid. ¹H NMR (400 MHz, DMSO-*d₆*) δ ppm 13.02 (br s, 1H), 8.02-8.10 (m, 2H), 7.56 (t, *J* = 7.9 Hz, 1H), 7.37 (d, *J* = 9.0 Hz, 1H), 7.25 (d, *J* = 9.1 Hz, 1H), 6.66 (s, 1H), 4.87 (s, 2H), 3.88 (s, 3H). MS obsd. (ESI⁺): 395.0 [(M+H)⁺], 397.1 [(M+2+H)⁺].

### Example 38

### 2-[3-(8-Chloro-4-oxo-chromen-2-yl)-2,6-dimethoxy-phenoxy]acetic acid

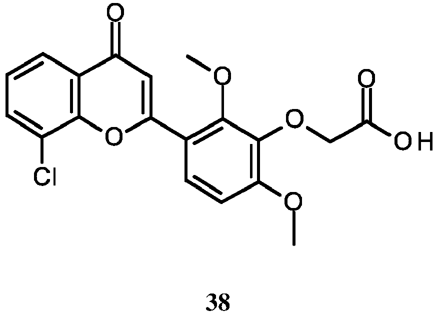

### Step 1. Synthesis of (E)-1-(3-chloro-2-hydroxy-phenyl)-3-[2,4-dimethoxy-3-[(4-methoxyphenyl)methoxy]phenyl]prop-2-en-1-one

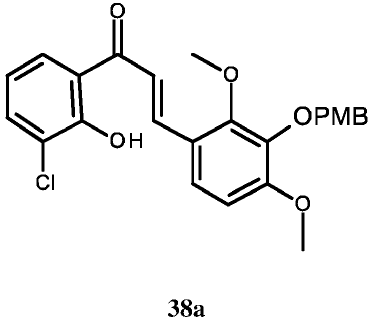

Compound **38a** was prepared in analogy to the procedure described for the preparation of example 1 by using 2,4-dimethoxy-3-[(4-methoxyphenyl)methoxy]benzaldehyde (**Intermediate AI**) was obtained as the starting material instead of 4-bromo-2-methoxy-5-[(4-methoxyphenyl)methoxy]benzaldehyde (Intermediate **AA**) in **Step 1**. (*E*)-1-(3-chloro-2-hydroxyphenyl)-3-[2,4-dimethoxy-3-[(4-methoxyphenyl)methoxy]phenyl]prop-2-en-1-one (1 g, 51% yield, compound **38a**) was obtained as a yellow solid MS obsd. (ESI⁺): 455.1 [(M+H)⁺], 457.1 [(M+2+H)⁺],

### Step 2. Synthesis of 8-chloro-2-(3-hydroxy-2,4-dimethoxy-phenyl)chromen-4-one

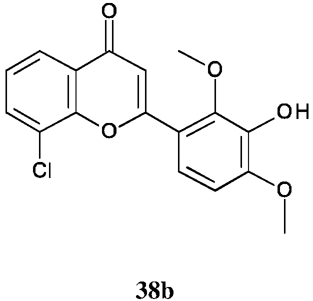

Compound **38b** was prepared in analogy to the procedure described for the preparation of example **1** by using (*E*)-1-(3-chloro-2-hydroxy-phenyl)-3-[2,4-dimethoxy-3-[(4-methoxyphenyl)methoxy]phenyl]prop-2-en-1-one (1.0 g, compound **38a**) as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2.** 8-chloro-2-(3-hydroxy-2,4-dimethoxy-phenyl)chromen-4-one (400 mg, 54% yield, compound **38b**) was obtained as a grey solid. MS obsd. (ESI⁺): 333.1 [(M+H)⁺], 335.0 [(M+2+H)⁺],

### Step 3. Synthesis of tert-butyl 2-[3-(8-chloro-4-oxo-chromen-2-yl)-2,6-dimethoxy-phenoxy]acetate

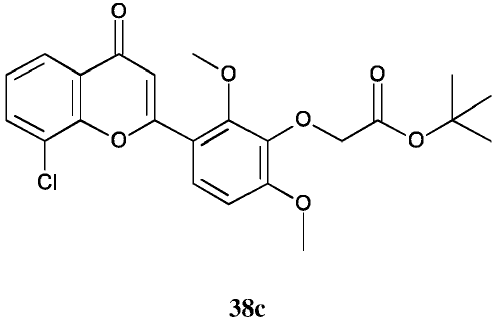

Compound **38c** was prepared in analogy to the procedure described for the preparation of example **1** by using 8-chloro-2-(3-hydroxy-2,4-dimethoxy-phenyl)chromen-4-one (400 mg, compound **38b**) and *tert*-butyl bromoacetate as the starting material instead of 2-(4-bromo-5-hydroxy-2-methoxy-phenyl)-8-chloro-chromen-4-one (compound **1c**) and *cis-tert*-butyl 3-[2-(*p-*tolylsulfonyloxy)ethoxy]cyclobutanecarboxylate in **Step 4.** *Tert*-butyl 2-[3-(8-chloro-4-*oxo-*chromen-2-yl)-2,6-dimethoxy-phenoxy]acetate (100 mg, 74% yield, compound **38c**) was obtained as a yellow solid. MS obsd. (ESI⁺): 447.1 [(M+H)⁺], 449.1 [(M+2+H)⁺].

### Step 4. Synthesis of 2-[3-(8-chloro-4-oxo-chromen-2-yl)-2,6-dimethoxy-phenoxy]acetic acid

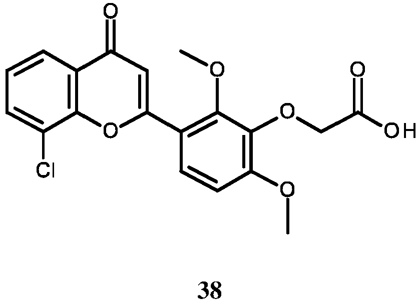

**Example 38** was prepared in analogy to the procedure described for the preparation of example 1 by using *tert*-butyl 2-[3-(8-chloro-4-*oxo*-chromen-2-yl)-2,6-dimethoxy-phenoxy]acetate (100 mg, compound **38c**) as the starting material instead of *tert*-butyl 3-[2-[2-bromo-5-(8-chloro-4-*oxo*-chromen-2-yl)-4-methoxy-phenoxy]ethoxy]cyclobutanecarboxylate (compound **1d**) in **Step** 5. 2-[3-(8-Chloro-4-*oxo*-chromen-2-yl)-2,6-dimethoxy-phenoxy]acetic acid (68.9 mg, 85% yield, Example **38**) was obtained as a yellow solid. ¹H NMR (400 MHz, DMSO-*d₆*) *δ* ppm 13.02 (br, 1H), 8.02 (d, *J* = 8.0 Hz, 2H), 7.67 (d, *J* = 8.0 Hz, 1H), 7.50 (t, *J*= 8.0 Hz, 1H), 7.09 (d, *J* = 8.0 Hz, 1H), 6.89 (s, 1H), 4.60 (s, 2H), 3.95 (s, 3H), 3.90 (s, 3H). MS obsd. (ESI⁺): 391.0 [(M+H)⁺], 393.0 [(M+2+H)⁺].

### Example 39

### 3-[2-Bromo-5-(8-chloro-4-oxo-chromen-2-yl)-4-ethoxy-phenoxy]propanoic acid

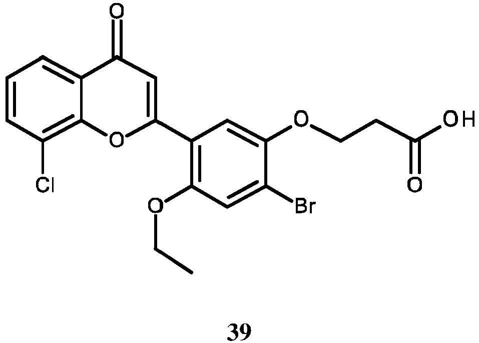

### Step 1. Synthesis of (E)-3-[4-bromo-2-ethoxy-5-(methoxymethoxy)phenyl]-1-(3-chloro-2-hydroxy-phenyl)prop-2-en-1-one

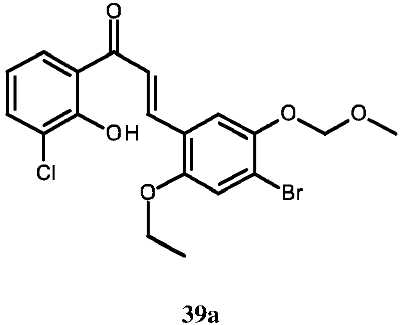

Compound **39a** was prepared in analogy to the procedure described for the preparation of example **1** by using4-bromo-2-ethoxy-5-(methoxymethoxy)benzaldehyde (Intermediate **AJ**) as the starting material instead of 4-bromo-2-methoxy-5-[(4-methoxyphenyl)methoxy]benzaldehyde (Intermediate **AA**) in **Step 1.** (*E*)-3-[4-Bromo-2-ethoxy-5-(methoxymethoxy)phenyl]-1-(3-chloro-2-hydroxy-phenyl)prop-2-en-1-one (100 mg, 93% yield, compound **39 a**) was obtained as a white solid. MS obsd. (ESI⁺): 441.0 [(M+H)⁺], 443.0 [(M+2+H)⁺], 445.0 [(M+4+H)⁺],

### Step 2. Synthesis of 2-(4-bromo-2-ethoxy-5-hydroxy-phenyl)-8-chloro-chromen-4-one

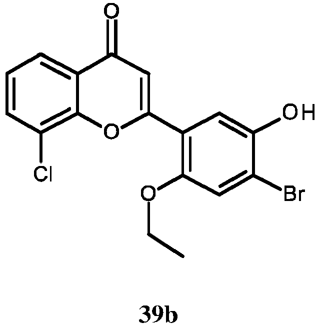

Compound **39b** was prepared in analogy to the procedure described for the preparation of example **1** by using (*E*)-3-[4-bromo-2-ethoxy-5-(methoxymethoxy)phenyl]-1-(3-chloro-2-hydroxy-phenyl)prop-2-en-1-one (100 mg, compound **39 a**) as the starting material instead of (*E*)-3-[4-bromo-2-methoxy-5-[(4-methoxyphenyl)methoxy]phenyl]-1-(3-chloro-2-hydroxyphenyl)prop-2-en-1-one (compound **1a**) in **Step 2.** 2-(4-bromo-2-ethoxy-5-hydroxy-phenyl)-8-chloro-chromen-4-one (90 mg, 90% yield, compound **39b**) was obtained as a yellow solid. MS obsd. (ESI⁺): 395.0 [(M+H)⁺], 397.0 [(M+2+H)⁺], 398.9 [(M+4+H)⁺],

### Step 3. Synthesis of 2-[4-bromo-5-[2-(1,3-dioxolan-2-yl)ethoxy]-2-ethoxy-phenyl]-8-chloro-chromen-4-one

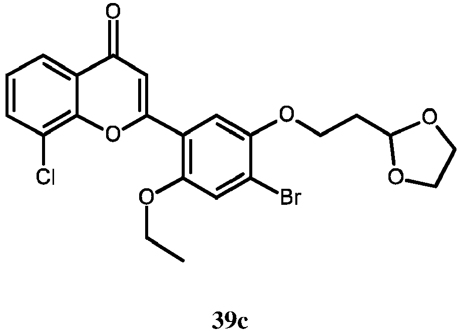

Compound **39c** was prepared in analogy to the procedure described for the preparation of example **1** by using 2-(4-bromo-2-ethoxy-5-hydroxy-phenyl)-8-chloro-chromen-4-one (90 mg, compound **39b**) and 2-(2-bromoethyl)-1,3-dioxolane as the starting material instead of 2-(4-bromo-5-hydroxy-2-methoxy-phenyl)-8-chloro-chromen-4-one (compound **1c**) and *cis-tert*-butyl 3-[2-(*p*-tolylsulfonyloxy)ethoxy]cyclobutanecarboxylate in **Step 4.** 2-[4-Bromo-5-[2-(1,3-dioxolan-2-yl)ethoxy]-2-ethoxy-phenyl]-8-chloro-chromen-4-one (70 mg, 85%, compound **39c**) was obtained as an white solid. MS obsd. (ESI⁺): 495.0 [(M+H)⁺], 497.0 [(M+2+H)⁺], 499.0 [(M+4+H)⁺],

### Step 4. Synthesis of 3-[2-bromo-5-(8-chloro-4-oxo-chromen-2-yl)-4-ethoxy-phenoxy]propanal

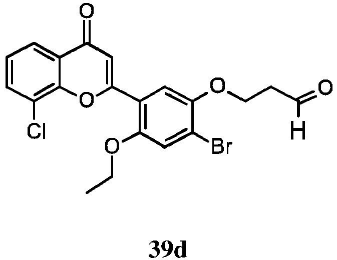

To a solution of 2-[4-bromo-5-[2-(1,3-dioxolan-2-yl)ethoxy]-2-ethoxy-phenyl]-8-chloro-chromen-4-one (70.0 mg) in water (0.5 mL) was added trifluoroacetic acid dropwise (2.0 mL). The mixture was stirred at 25 °C for 2 hrs. After reaction, water (500 mL) was then added to the solution. The solution was extracted with EtOAc (200 mL) three times. The combined organic layer was washed with brine (300 mL) two times, dried with MgSO₄, filtered and concentrated filtrate to afford 3-[2-bromo-5-(8-chloro-4-*oxo*-chromen-2-yl)-4-ethoxy-phenoxy]propanal (60 mg, 94% yield, Compound **39d**) was obtained as a yellow solid. MS obsd. (ESI⁺): 451.0 [(M+H)⁺], 453.0 [(M+2+H)⁺], 455.0 [(M+4+H)⁺].

### Step 5. Synthesis of 3-[2-bromo-5-(8-chloro-4-oxo-chromen-2-yl)-4-ethoxy-phenoxy]propanoic acid

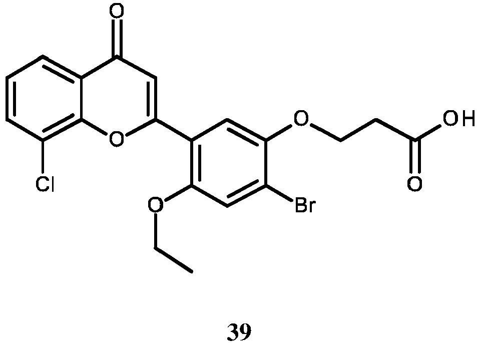

A mixture of 3-[2-bromo-5-(8-chloro-4-*oxo*-chromen-2-yl)-4-ethoxy-phenoxy]propanal (60.0 mg, 0.130 mmol), 2-methyl-2-butene (83.84 mg, 1.2 mmol), chlorosyloxysodium (36.04 mg, 0.4 mmol), sodium phosphate monobasic (44.62 mg, 0.370 mmol) in 1,4-dioxane (2.51 mL) and water (0.835 mL). The pH was adjusted to around 6 by 1*M* HCl. The mixture was added water (400 mL). The mixture was filtered. The filter cake was washed with water (400 mL) and DCM (150 mL) to give the crude product. TFA (15 ml) was added dropwise to the mixture of the crude product in DCM (400 mL) until the solid was dissolved. Then ethyl acetate (100 mL) was dropwise to the solution until the solid separated out. The mixture was filtered. The filter cake was purified by prep-HPLC (Kromasil-C18 100 × 21.2 mm 5µm Mobile Phase: ACN/H₂O (0.1%FA) Gradient: 30-40%) to give 3-[2-bromo-5-(8-chloro-4-*oxo*-chromen-2-yl)-4-ethoxy-phenoxy]propanoic acid (16.5 mg, 27% yield, example **39**) as a white solid. ¹H NMR (400 MHz, DMSO-*d₆*) δ ppm 12.67 (s, 1H), 7.99 (t, *J* = 6.8 Hz, 2H), 7.67 (s, 1H), 7.49 (t, *J* = 7.7 Hz, 2H), 7.12 (s, 1H), 4.29 (t, *J* = 5.9 Hz, 2H), 4.19 (q, *J* = 6.8 Hz, 2H), 2.75 (t, *J* = 5.9 Hz, 2H), 1.38 (t, *J* = 6.9 Hz, 3H). MS obsd. (ESI⁺): 467.0 [(M+H)⁺], 469.0 [(M+2+H)⁺].

### Example 40

### 3-[2-Bromo-5-(8-chloro-4-oxo-chromen-2-yl)-4-(difluoromethoxy)phenoxy]propanoic acid

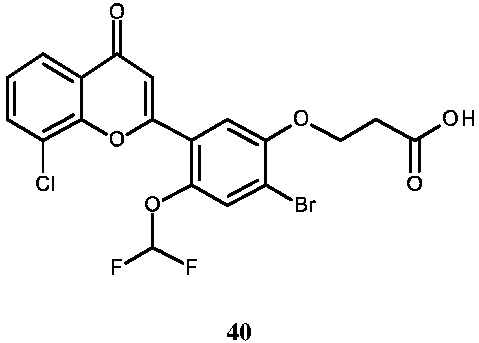

### Step 1. Synthesis of (E)-3-[4-bromo-2-(difluoromethoxy)-5-(methoxymethoxy)phenyl]-1-(3-chloro-2-hydroxy-phenyl)prop-2-en-1-one

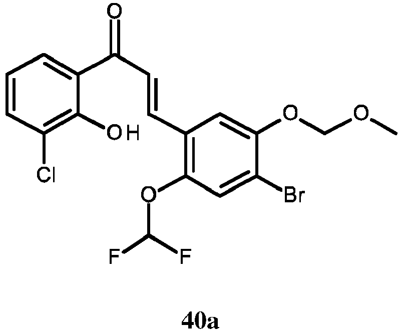

Compound **40a** was prepared in analogy to the procedure described for the preparation of example **1** by using 4-bromo-2-(difluoromethoxy)-5-(methoxymethoxy)benzaldehyde (Intermediate **AK**) as the starting material instead of 4-bromo-2-methoxy-5-[(4-methoxyphenyl)methoxy]benzaldehyde (Intermediate **AA**) in **Step 1.** (*E*)-3-[4-Bromo-2-(difluoromethoxy)-5-(methoxymethoxy)phenyl]-1-(3-chloro-2-hydroxy-phenyl)prop-2-en-1-one (120 mg, 94% yield, compound **40a**) was obtained as a yellow solid. MS obsd. (ESI⁺): 375.0 [(M+H)⁺], 377.0 [(M+2+H)⁺],

### Step 2. Synthesis of 2-[4-bromo-2-(difluoromethoxy)-5-hydroxy-phenyl]-8-chloro-chromen-4-one

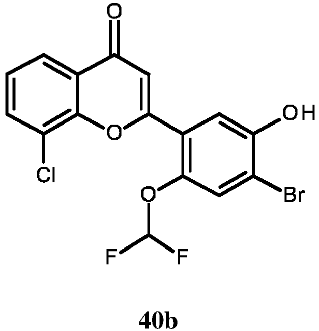

Compound **40b** was prepared in analogy to the procedure described for the preparation of example **1** by using (*E*)-3-[4-bromo-2-(difluoromethoxy)-5-(methoxymethoxy)phenyl]-1-(3-chloro-2-hydroxy-phenyl)prop-2-en-1-one (120 mg, compound **40a**) as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step** 2. 2-[4-bromo-2-(difluoromethoxy)-5-hydroxy-phenyl]-8-chloro-chromen-4-one (100 mg, 85% yield, compound **40b**) was obtained as a yellow solid. MS obsd. (ESI⁺): 417.0 [(M+H)⁺], 419.0 [(M+2+H)⁺],

### Step 3. Synthesis of 2-[4-bromo-2-(difluoromethoxy)-5-[2-(1,3-dioxolan-2-yl)ethoxy]phenyl]-8-chloro-chromen-4-one

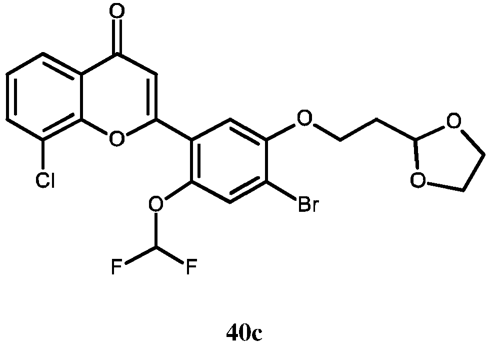

Compound **40c** was prepared in analogy to the procedure described for the preparation of example **1** by using 2-[4-bromo-2-(difluoromethoxy)-5-hydroxy-phenyl]-8-chloro-chromen-4-one (100 mg, compound **40b**)and 2-(2-bromoethyl)-1,3-dioxolane as the starting material instead of 2-(4-bromo-5-hydroxy-2-methoxy-phenyl)-8-chloro-chromen-4-one (compound **1c**) and cis-*tert*-butyl 3-[2-(*p*-tolylsulfonyloxy)ethoxy]cyclobutanecarboxylate in **Step 4.** 2-[4-Bromo-2-(difluoromethoxy)-5-[2-(1,3-dioxolan-2-yl)ethoxy]phenyl]-8-chloro-chromen-4-one (80 mg, 64% yield, compound **40c**) was obtained as a light yellow solid. MS obsd. (ESI⁺): 517.0 [(M+H)⁺], 519.0 [(M+2+H)⁺].

### Step 4. Synthesis of 3-[2-bromo-5-(8-chloro-4-oxo-chromen-2-yl)-4-(difluoromethoxy)phenoxy]propanal

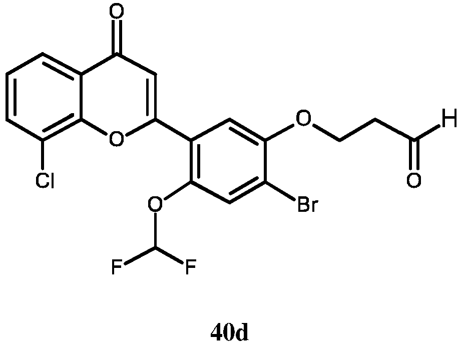

Compound **40d** was prepared in analogy to the procedure described for the preparation of example **1** by using 2-[4-bromo-2-(difluoromethoxy)-5-[2-(1,3-dioxolan-2-yl)ethoxy]phenyl]-8-chloro-chromen-4-one (80 mg, compound **40c**) as the starting material instead of 2-(4-bromo-2-ethoxy-5-hydroxy-phenyl)-8-chloro-chromen-4-one (90 mg, 90% yield, compound **39b**) in **Step 3**. 3-[2-bromo-5-(8-chloro-4-*oxo*-chromen-2-yl)-4-(difluoromethoxy)phenoxy]propanal (70 mg, 95% yield, compound **40d**) was obtained as a yellow solid. MS obsd. (ESI⁺): 475.0 [(M+H)⁺], 476.9 [(M+2+H)⁺].

### Step 5. Synthesis of 3-[2-bromo-5-(8-chloro-4-oxo-chromen-2-yl)-4-(difluoromethoxy)phenoxy]propanoic acid

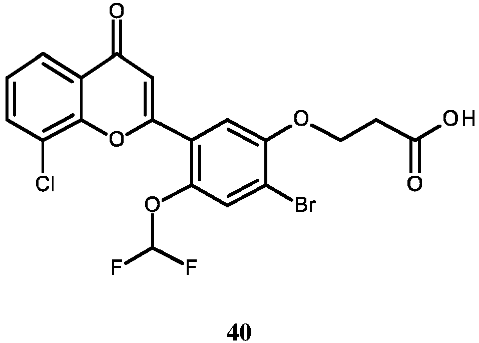

Example **40** was prepared in analogy to the procedure described for the preparation of example **1** by using 3-[2-bromo-5-(8-chloro-4-oxo-chromen-2-yl)-4-(difluoromethoxy)phenoxy]propanal (70 mg, compound **40d**) as the starting material instead of 3-[2-bromo-5-(8-chloro-4-*oxo*-chromen-2-yl)-4-ethoxy-phenoxy]propanal (60 mg, 94% yield. Compound **39d**) in **Step 4.** 3-[2-Bromo-5-(8-chloro-4-oxo-chromen-2-yl)-4-(difluoromethoxy)phenoxy]propanoic acid (11.1 mg, 15 % yield, Example **40**) was obtained as a white solid. ¹H NMR (400 MHz, DMSO-*d₆*) δ ppm 12.49 (s, 1H), 8.04 (ddd, *J* = 8.0, 3.7, 1.5 Hz, 2H), 7.73 (s, 1H), 7.66 (s, 1H), 7.53 (t, *J* = 7.9 Hz, 1H), 7.30 (t, *J* = 73.3 Hz, 1H), 6.90 (s, 1H), 4.37 (t, *J* = 6.1 Hz, 2H), 2.78 (t, *J* = 6.0 Hz, 2H). MS obsd. (ESI⁺): 489.0 [(M+H)⁺], 491.0 [(M+2+H)⁺],

### Example 41

### 3-[2-Bromo-5-(8-chloro-4-oxo-chromen-2-yl)-4-fluoro-phenoxy]propanoic acid

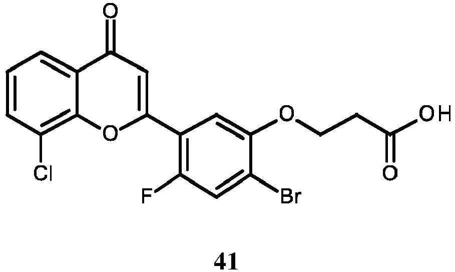

### Step 1. Synthesis of (E)-3-[4-bromo-2-fluoro-5-(methoxymethoxy)phenyl]-1-(3-chloro-2-hydroxy-phenyl)prop-2-en-1-one

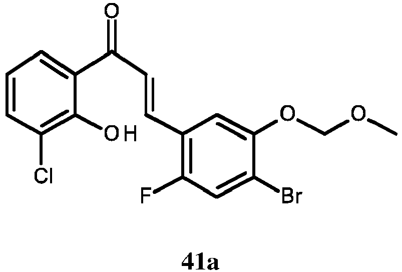

Compound **41a** was prepared in analogy to the procedure described for the preparation of example **1** by using 4-bromo-2-fluoro-5-(methoxymethoxy)benzaldehyde (Intermediate **AM**) as the starting material instead of 4-bromo-2-methoxy-5-[(4-methoxyphenyl)methoxy]benzaldehyde (Intermediate **AA**) in **Step 1.** (*E*)-3-[4-Bromo-2-fluoro-5-(methoxymethoxy)phenyl]-1-(3-chloro-2-hydroxy-phenyl)prop-2-en-1-one (350 mg, 85.2% yield, compound **41a**) was obtained as a yellow solid. MS obsd. (ESI⁺): 415.0[(M+H)⁺], 416.9 [(M+H+2)⁺].

### Step 2. Synthesis of 2-(4-Bromo-2-fluoro-5-hydroxy-phenyl)-8-chloro-chromen-4-one

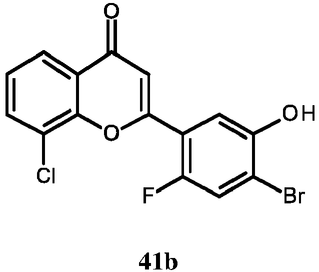

Compound **41b** was prepared in analogy to the procedure described for the preparation of example **1** by using (*E*)-3-[4-bromo-2-fluoro-5-(methoxymethoxy)phenyl]-1-(3-chloro-2-hydroxy-phenyl)prop-2-en-1-one (350 mg, compound **41a**) as the starting material instead of (*E*)-3-[4-bromo-2-methoxy-5-[(4-methoxyphenyl)methoxy]phenyl]-1-(3-chloro-2-hydroxyphenyl)prop-2-en-1-one (compound **1a**) in **Step 2.** 2-(4-bromo-2-fluoro-5-hydroxy-phenyl)-8-chloro-chromen-4-one (300 mg, 96.4% yield, compound **41b**) was obtained as a light yellow solid. MS obsd. (ESI⁺): 368.9 [(M+H)⁺], 370.9 [(M+H+2)⁺].

### Step 3. Synthesis of 2-[4-bromo-5-[2-(1,3-dioxolan-2-yl)ethoxy]-2-fluoro-phenyl]-8-chloro-chromen-4-one

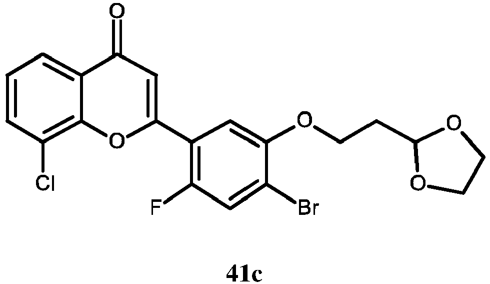

Compound **41c** was prepared in analogy to the procedure described for the preparation of example **1** by using 2-(4-bromo-2-fluoro-5-hydroxy-phenyl)-8-chloro-chromen-4-one (300 mg, compound **41b**) and 2-(2-bromoethyl)-1,3-dioxolane as the starting material instead of 2-(4-bromo-5-hydroxy-2-methoxy-phenyl)-8-chloro-chromen-4-one (compound **1c**) and *cis-tert*-butyl 3-[2-(p-tolylsulfonyloxy)ethoxy]cyclobutanecarboxylate in **Step 4.** 2-[4-Bromo-5-[2-(1,3-dioxolan-2-yl)ethoxy]-2-fluoro-phenyl]-8-chloro-chromen-4-one (60 mg, 47.21% yield, compound **41c**) was obtained as a light yellow solid. MS obsd. (ESI⁺): 469.0 [(M+H)⁺], 471.0 [(M+H+2)⁺].

### Step 4. Synthesis of 3-[2-bromo-5-(8-chloro-4-oxo-chromen-2-yl)-4-fluoro-phenoxy]propanal

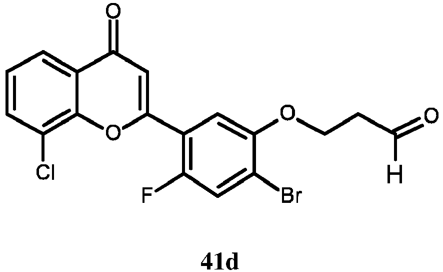

Compound **41d** was prepared in analogy to the procedure described for the preparation of example **39** by using 2-[4-bromo-5-[2-(1,3-dioxolan-2-yl)ethoxy]-2-fluoro-phenyl]-8-chloro-chromen-4-one (60 mg, compound **41c**) as the starting material instead of 2-(4-bromo-2-ethoxy-5-hydroxy-phenyl)-8-chloro-chromen-4-one (compound **39b**) in **Step 3.** 3-[2-Bromo-5-(8-chloro-4-oxo-chromen-2-yl)-4-fluoro-phenoxy]propanal (60 mg, compound **41d**) was obtained as a light yellow solid. MS obsd. (ESI⁺): 425.0 [(M+H)⁺], 427.0 [(M+H+2)⁺].

### Step 5. Synthesis of 3-[2-bromo-5-(8-chloro-4-oxo-chromen-2-yl)-4-fluoro-phenoxy]propanoic acid

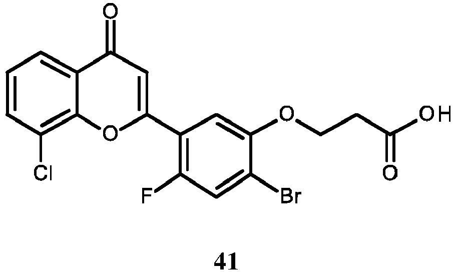

Example **41** was prepared in analogy to the procedure described for the preparation of example **1** by using 3-[2-bromo-5-(8-chloro-4-*ox*o-chromen-2-yl)-4-fluoro-phenoxy]propanal (60 mg, compound **41d**) as the starting material instead of 3-[2-bromo-5-(8-chloro-4-oxo-chromen-2-yl)-4-ethoxy-phenoxy]propanal (Compound **39d**) in **Step 4.** 3-[2-Bromo-5-(8-chloro-4-*oxo*-chromen-2-yl)-4-fluoro-phenoxy]propanoic acid (26 mg, 41% yield, example **41**) was obtained as a light yellow solid. MS obsd. ¹H NMR (400 MHz, DMSO-*d₆*) δ ppm 12.46 (s, 1H), 8.04 (t, *J* = 7.6 Hz, 2H), 7.90 (d, *J* = 10.5 Hz, 1H), 7.66 (d, *J* = 6.5 Hz, 1H), 7.53 (t, *J* = 7.9 Hz, 1H), 6.96 (s, 1H), 4.37 (t, *J* = 5.9 Hz, 2H), 2.79 (t, *J* = 5.9 Hz, 2H). MS obsd. (ESI⁺) [(M+H)⁺]: 441.0 [(M+H)⁺], 443.0 [(M+2+H)⁺].

### Example 42

### 3-[2-Bromo-5-(8-chloro-4-oxo-chromen-2-yl)-4-(trifluoromethoxy)phenoxy]propanoic acid

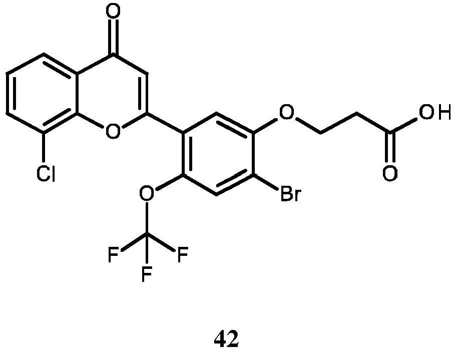

### Step 1. Synthesis of 3-[2-bromo-5-[(E)-3-(3-chloro-2-hydroxy-phenyl)-3-oxo-prop-1-enyl]-4-(trifluoromethoxy)phenoxy]propanoic acid

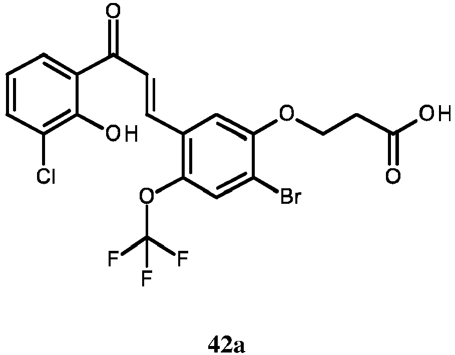

Compound **42a** was prepared in analogy to the procedure described for the preparation of example **1** by using 3-[2-bromo-5-formyl-4-(trifluoromethoxy)phenoxy]propanoic acid (Intermediate **AN**) as the starting material instead of 4-bromo-2-methoxy-5-[(4-methoxyphenyl)methoxy]benzaldehyde (Intermediate **AA**) in **Step 1.** 3-[2-Bromo-5-[(*E*)-3-(3-chloro-2-hydroxy-phenyl)-3-*oxo*-prop- 1-enyl]-4-(trifluoromethoxy)phenoxy]propanoic acid (800 mg, 56% yield, compound **42a**) was obtained as a light yellow solid. MS obsd. (ESI⁺): 508.9 [(M+H)⁺], 511.0 [(M+2+H)⁺],

### Step 2. Synthesis of 3-[2-bromo-5-(8-chloro-4-oxo-chromen-2-yl)-4-(trifluoromethoxy)phenoxy]propanoic acid

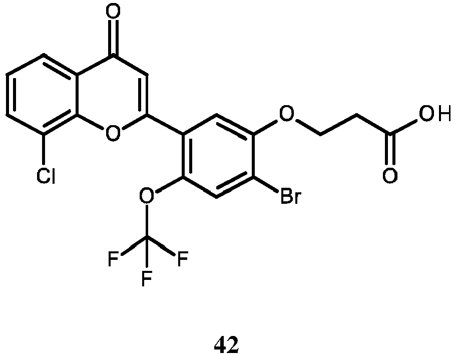

Example **42** was prepared in analogy to the procedure described for the preparation of example **1** by using 3-[2-bromo-5-[(*E*)-3-(3-chloro-2-hydroxy-phenyl)-3-oxo-prop-1-enyl]-4-(trifluoromethoxy)phenoxy]propanoic acid (800 mg, compound **42a**) as the starting material instead of (*E*)-3-[4-bromo-2-methoxy-5-[(4-methoxyphenyl)methoxy]phenyl]-1-(3-chloro-2-hydroxy-phenyl)prop-2-en-1-one (compound **1a**) in **Step 2.** 3-[2-bromo-5-(8-chloro-4-*oxo-*chromen-2-yl)-4-(trifluoromethoxy)phenoxy]propanoic acid (58.2 mg, 29% yield, example **42**) was obtained as a light yellow solid. ¹H NMR (400 MHz, DMSO-*d₆*) δ ppm 12.54 (s, 1H), 7.89-8.18 (m, 3H), 7.79 (s, 1H), 7.50 (s, 1H), 7.07 (s, 1H), 4.45 (t, *J* = 5.8 Hz, 2H), 2.81 (d, *J* = 5.7 Hz, 2H). MS obsd. (ESI⁺): 507.0 [(M+H)⁺], 509.0 [(M+2+H)⁺].

### Example 43

### 3-[2-Bromo-4-chloro-5-(8-chloro-4-oxo-chromen-2-yl)phenoxy]propanoic acid

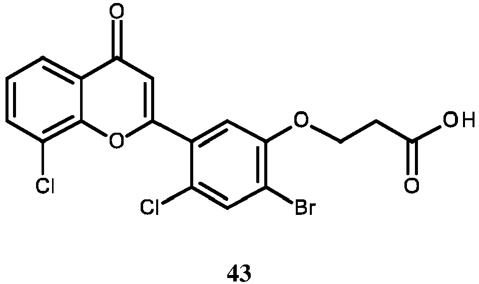

### Step 1. Synthesis of (E)-3-[4-bromo-2-chloro-5-(methoxymethoxy)phenyl]-1-(3-chloro-2-hydroxy-phenyl)prop-2-en-1-one

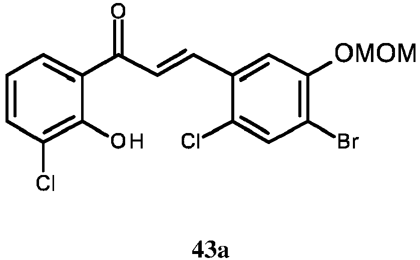

Compound **43a** was prepared in analogy to the procedure described for the preparation of example **1** by using 4-bromo-2-chloro-5-(methoxymethoxy)benzaldehyde (Intermediate **AL**) as the starting material instead of 4-bromo-2-methoxy-5-[(4-methoxyphenyl)methoxy]benzaldehyde (Intermediate **AA**) in **Step 1.** (*E*)-3-[4-Bromo-2-chloro-5-(methoxymethoxy)phenyl]-1-(3-chloro-2-hydroxy-phenyl)prop-2-en-1-one (327 mg, 70% yield, compound **43a**) was obtained as a light yellow solid. MS obsd. (ESI⁺): 430.9 [(M+H)⁺], 432.9 [(M+2+H)⁺],

### Step 2. Synthesis of 2-[4-bromo-2-chloro-5-(methoxymethoxy)phenyl]-8-chloro-chromen-4-one

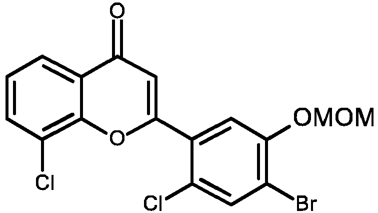

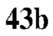

Compound **43b** was prepared in analogy to the procedure described for the preparation of example **1** by using (*E*)-3-[4-bromo-2-chloro-5-(methoxymethoxy)phenyl]-1-(3-chloro-2-hydroxy-phenyl)prop-2-en-1-one (327 mg, compound **43a**) as the starting material instead of (*E*)-3-[4-bromo-2-methoxy-5-[(4-methoxyphenyl)methoxy]phenyl]-1-(3-chloro-2-hydroxyphenyl)prop-2-en-1-one (compound **1a**) in **Step 2.** 2-[4-bromo-2-chloro-5-(methoxymethoxy)phenyl]-8-chloro-chromen-4-one (288 mg, 96% yield, compound **43b**) was obtained as a light yellow solid.MS obsd. (ESI⁺): 428.9 [(M+H)⁺], 430.9 [(M+2+H)⁺], 432.9 [(M+4+H)⁺],

### Step 3. Synthesis of 2-(4-bromo-2-chloro-5-hydroxy-phenyl)-8-chloro-chromen-4-one

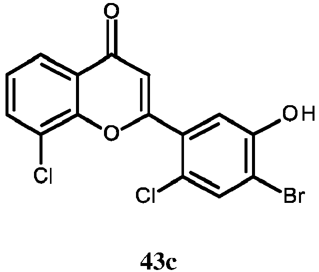

To a solution of 2-[4-bromo-2-chloro-5-(methoxymethoxy)phenyl]-8-chloro-chromen-4-one (288.0 mg) in DCM (3 mL) was added trifluoroacetic acid (2.0 mL) and stirred at room temperature for 2 hrs. The reaction mixture was concentrated in vacuo to give 2-(4-bromo-2-chloro-5-hydroxy-phenyl)-8-chloro-chromen-4-one (240 mg, 92% yield, compound **43c**) was obtained as a light yellow solid. MS obsd. (ESI⁺): 384.9 [(M+H)⁺], 386.9 [(M+2+H)⁺], 388.9 [(M+4+H)⁺]

### Step 4. Synthesis of 8-chloro-2-[2-[2-(1,3-dioxolan-2-yl)ethoxy]-4-(trifluoromethyl)phenyl]-7-(1-methylpyrazol-3-yl)chromen-4-one

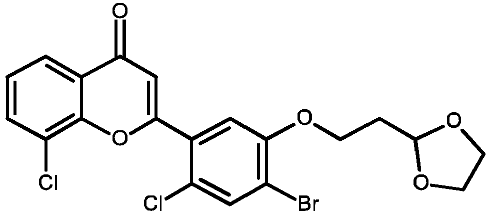

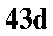

Compound **43d** was prepared in analogy to the procedure described for the preparation of example **1** by using 2-(4-bromo-2-chloro-5-hydroxy-phenyl)-8-chloro-chromen-4-one (compound **43c**) and 2-(2-bromoethyl)-1,3-dioxolane as the starting material instead of 2-(4-bromo-5-hydroxy-2-methoxy-phenyl)-8-chloro-chromen-4-one (compound **1c**) and *tert*-butyl 3-[2-(*p*-tolylsulfonyloxy)ethoxy]cyclobutanecarboxylate in **Step 4.** 8-Chloro-2-[2-[2-(1,3-dioxolan-2-yl)ethoxy]-4-(trifluoromethyl)phenyl]-7-(1-methylpyrazol-3-yl)chromen-4-one (160 mg, 81% yield, compound **43d** ) was obtained as a light yellow solid. MS obsd. (ESI⁺): 485.0 [(M+H)⁺], 487.0 [(M+2+H)⁺], 489.0 [(M+4+H)⁺]

### Step 5. Synthesis of 3-[2-bromo-4-chloro-5-(8-chloro-4-oxo-chromen-2-yl)phenoxy]propanal

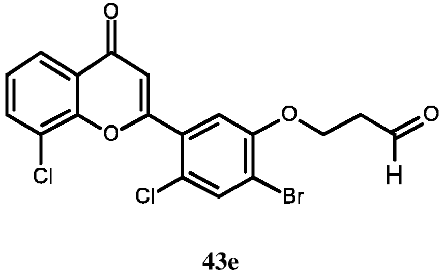

Compound **43e** was prepared in analogy to the procedure described for the preparation of example **1** by using 8-chloro-2-[2-[2-(1,3-dioxolan-2-yl)ethoxy]-4-(trifluoromethyl)phenyl]-7-(1-methylpyrazol-3-yl)chromen-4-one (160 mg, compound **43d** ) as the starting material instead of 2-(4-bromo-2-ethoxy-5-hydroxy-phenyl)-8-chloro-chromen-4-one (90 mg, 90% yield, compound **39b**) in **Step 3.** 3-[2-bromo-4-chloro-5-(8-chloro-4-*oxo*-chromen-2-yl)phenoxy]propanal (198 mg, compound **43e**) was obtained as a crude solid. MS obsd. (ESI⁺): 440.9 [(M+H)⁺], 443.0 [(M+2+H)⁺], 445.0 [(M+4+H)⁺].

### Step 6. Synthesis of 3-[2-bromo-4-chloro-5-(8-chloro-4-oxo-chromen-2-yl)phenoxy]propanoic acid

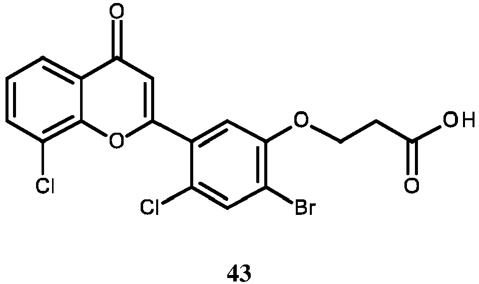

Example **43** was prepared in analogy to the procedure described for the preparation of example **1** by using 3-[2-bromo-4-chloro-5-(8-chloro-4-*oxo*-chromen-2-yl)phenoxy]propanal (198 mg, compound **43e**) as the starting material instead of 3-[2-bromo-5-(8-chloro-4-*oxo-*chromen-2-yl)-4-ethoxy-phenoxy]propanal (Compound **39d**) in **Step 4.** 3-[2-Bromo-4-chloro-5-(8-chloro-4-*oxo*-chromen-2-yl)phenoxy]propanoic acid (48.9 mg, Example **43**) was obtained as a white solid. ¹H NMR (400 MHz, DMSO-*d₆*) δ ppm 8.07 (t, *J* = 8.3 Hz, 2H), 7.69 (d, *J* = 9.0 Hz, 1H), 7.56 (d, *J* = 7.9 Hz, 1H), 7.42 (d, *J* = 9.1 Hz, 1H), 6.71 (s, 1H), 4.33 (t, *J* = 5.9 Hz, 2H), 2.74 (t, *J* = 5.9 Hz, 2H). MS obsd. (ESI⁺): 456.9 [(M+H)⁺], 458.9 [(M+2+H)⁺], 460.9. [(M+4+H)⁺] .

### Example 44

### 2-[3-[6-Bromo-3-(8-chloro-4-oxo-chromen-2-yl)-2-methoxy-phenoxy]propylamino]-2-oxo-acetic acid

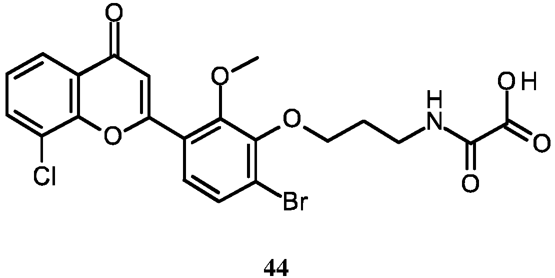

### Step 1. Synthesis of tert-butyl N-[3-[6-bromo-3-(8-chloro-4-oxo-chromen-2-yl)-2-methoxy-phenoxy]propyl]carbamate

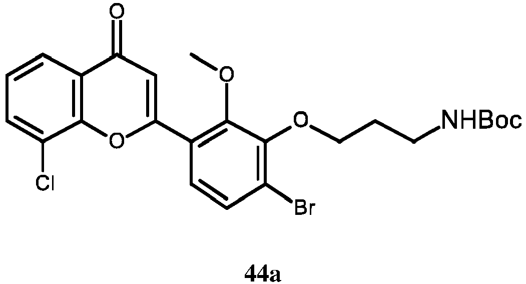

To a solution of 2-(4-bromo-3-hydroxy-2-methoxy-phenyl)-8-chloro-chromen-4-one (200.0 mg, 0.520 mmol, compound **34b**) and potassium iodide (174.0 mg) in dry DMF (20 mL) was added potassium *tert*-butoxide (117.62 mg). After addition, the reaction mixture was stirred at 25 °C for 0.5 h. Then 3-(BOC-amino)propyl bromide (250 mg) was added. After addition, the reaction was stirred at 25 °C for 16 hrs. Then it was cooled to 25 °C, potassium *tert-*butoxide (117.62 mg) was added. After addition, the reaction was stirred at 25 °C for 0.5 h. Then 3-(BOC-amino)propyl bromide (250 mg) was added. After addition, the reaction was stirred at 25 °C for 16 hrs. Then it was poured into H₂O (50 mL), extracted with EtOAc (20 mL) three times. The combined organics were washed with brine (100 mL) three times, dried over anhydrous Na₂SO₄, filtered and concentrated in vacuo. The resulting oil was purified by flash silica gel column chromatography (eluting with EtOAc/PE (V/V)= 3/7) to give *tert-butyl N-*[3-[6-bromo-3-(8-chloro-4-*oxo*-chromen-2-yl)-2-methoxy-phenoxy]propyl]carbamate (90 mg, 31.87% yield, compound **44a**) as a yellow solid. MS obsd. (ESI⁺): 560.0 [(M+Na)⁺], 562.0 [(M+2+Na)⁺],

### Step 2. Synthesis of 2-[3-(3-aminopropoxy)-4-bromo-2-methoxy-phenyl]-8-chloro-chromen-4-one

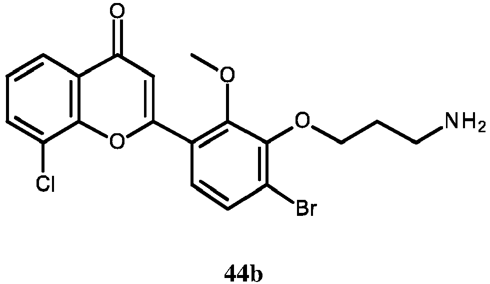

To a solution of *tert*-butyl *N*-[3-[6-bromo-3-(8-chloro-4-*oxo*-chromen-2-yl)-2-methoxy-phenoxy]propyl]carbamate (90.0 mg, 0.170 mmol) in DCM (10 mL) at 25 °C was added trifluoroacetic acid (1.0 mL, 13 mmol) dropwise. After addition, this mixture was stirred at 25 °C for 3 hrs. Then the reaction mixture was concentrated *in vacuo* to give 2-[3-(3-aminopropoxy)-4-bromo-2-methoxy-phenyl]-8-chloro-chromen-4-one (72 mg, compound **44b**) as a yellow solid. MS obsd. (ESI⁺): 438.0 [(M+H)⁺], 440.0 [(M+2+H)⁺],

### Step 3. Synthesis of ethyl 2-[3-[6-bromo-3-(8-chloro-4-oxo-chromen-2-yl)-2-methoxy-phenoxy]propylamino]-2-oxo-acetate

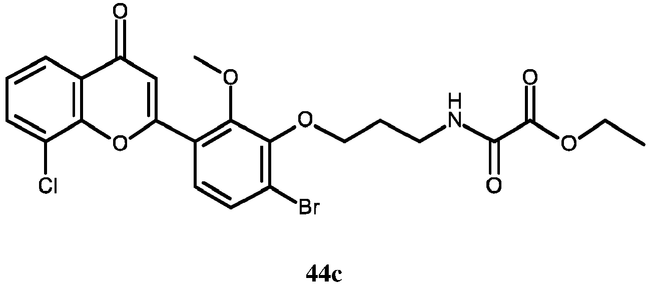

To a solution of 2-[3-(3-aminopropoxy)-4-bromo-2-methoxy-phenyl]-8-chloro-chromen-4-one (72.0 mg), *N,N*-diisopropylethylamine (0.11 mL) in DCM (10 mL) at 0 °C was added ethyl oxalyl chloride (33.61 mg) dropwise. After addition, this mixture was stirred at 25 °C for 6 hrs. Then it was concentrated in vacuo. The crude product was purified by reverse silica gel chromatography (eluting with ACN/water from 0% to 50%, 0.1% FA in water) to give ethyl 2-[3-[6-bromo-3-(8-chloro-4-*oxo*-chromen-2-yl)-2-methoxy-phenoxy]propylamino]-2-oxo-acetate (40 mg, compound **44c**) as a white solid. MS obsd. (ESI⁺): 560.0 [(M+H)⁺], 562.0 [(M+2+H)⁺].

### Step 4. Synthesis of 2-[3-[6-bromo-3-(8-chloro-4-oxo-chromen-2-yl)-2-methoxy-phenoxy]propylamino]-2-oxo-acetic acid

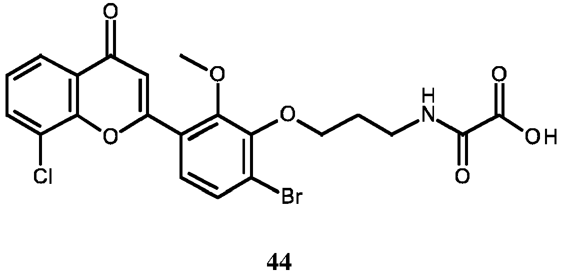

To a solution of ethyl 2-[3-[6-bromo-3-(8-chloro-4-*oxo*-chromen-2-yl)-2-methoxy-phenoxy]propylamino]-2-*oxo*-acetate (40.0 mg) in THF (10 mL) at 0 °C was added lithium hydroxide (0.74 mL). After being stirred at 25 °C for 16 hrs, the reaction mixture was cooled to 0 °C, acidified to pH = 3 with 1 *M* HCl at 0 °C and concentrated *in vacuo.* The residue was purified by prep-HPLC (eluting with ACN in water 0% to 20%, 0.1% NH₄OH in water) to afford 2-[3-[6-bromo-3-(8-chloro-4-oxo-chromen-2-yl)-2-methoxy-phenoxy]propylamino]-2-oxo-acetic acid (15 mg, example 44) as an off-white solid. ¹H NMR (400 MHz, DMSO-*d₆*) δ ppm 13.85 (s, 1H), 8.94 (s, 1H), 8.03 (d, *J* = 7.8 Hz, 2H), 7.66 (d, *J* = 8.6 Hz, 1H), 7.59 (d, *J* = 8.9 Hz, 1H), 7.53 (t, *J* = 7.5 Hz, 1H), 6.93 (s, 1H), 4.05 (s, 1H), 3.92 (s, 3H), 3.39 (s, 2H), 2.01 (s, 2H). MS obsd. (ESI⁺): 510.0 [(M+H)⁺], 512.0 [(M+2+H)⁺].

### Example 45

### 2-[2-[2-Bromo-5-(8-chloro-4-oxo-chromen-2-yl)-4-methoxy-phenoxy]ethylamino]-2-oxo-acetic acid

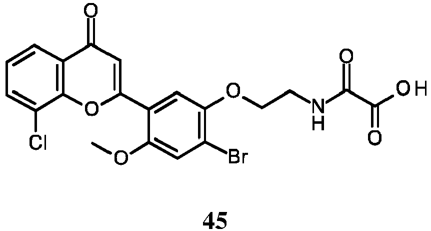

### Step 1. Synthesis of tert-butyl N-[2-[2-bromo-5-(8-chloro-4-oxo-chromen-2-yl)-4-methoxy-phenoxy]ethyl]carbamate

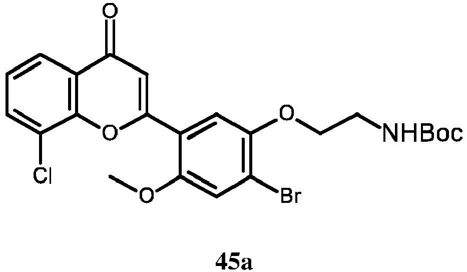

Compound **45a** was prepared in analogy to the procedure described for the preparation of example **44** by using 2-(4-bromo-5-hydroxy-2-methoxy-phenyl)-8-chloro-chromen-4-one (compound **1c**) and *tert*-butyl *N*-(2-bromoethyl)carbamate as the starting material instead of 2-(4-bromo-3-hydroxy-2-methoxy-phenyl)-8-chloro-chromen-4-one (compound **34b**) and 3-(BOC-amino)propyl bromide in **Step 1.** *Tert*-Butyl *N*-[2-[2-bromo-5-(8-chloro-4-oxo-chromen-2-yl)-4-methoxy-phenoxy]ethyl]carbamate (240 mg, compound **45a**) was obtained as a yellow solid.MS obsd. (ESI⁺): 524.1 [(M+H)⁺], 526.1 [(M+2+H)⁺],

### Step 2. Synthesis of 2-[5-(2-aminoethoxy)-4-bromo-2-hydroxy-phenyl]-8-chloro-chroman-4-one

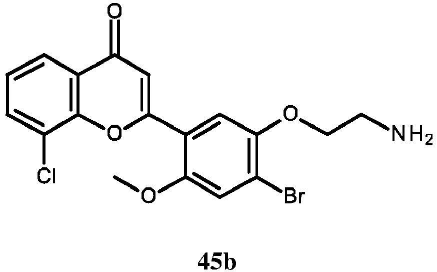

Compound **45b** was prepared in analogy to the procedure described for the preparation of example **44** by using *tert*-butyl *N*-[2-[2-bromo-5-(8-chloro-4-*oxo*-chromen-2-yl)-4-methoxy-phenoxy]ethyl]carbamate (240 mg, compound **45a**) as the starting material instead of *tert*-butyl N-[3-[6-bromo-3-(8-chloro-4-*oxo*-chromen-2-yl)-2-methoxy-phenoxy]propyl]carbamate (compound **44a**) in **Step 2.** 2-[5-(2-Aminoethoxy)-4-bromo-2-hydroxy-phenyl]-8-chloro-chroman-4-one (198 mg, compound **45b**) was obtained as a yellow solid. MS obsd. (ESI⁺): 424.0 [(M+H)⁺], 426.1 [(M+2+H)⁺],

### Step 3. Synthesis of ethyl 2-[2-[2-bromo-5-(8-chloro-4-oxo-chromen-2-yl)-4-methoxy-phenoxy]ethylamino]-2-oxo-acetate

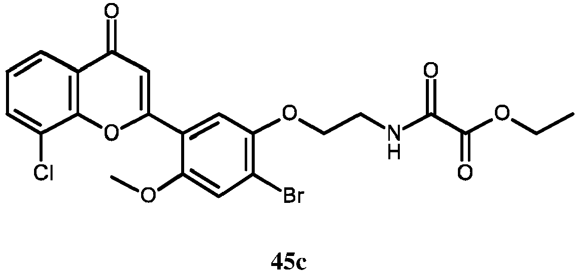

Compound **46c** was prepared in analogy to the procedure described for the preparation of example **44** by using 2-[5-(2-aminoethoxy)-4-bromo-2-hydroxy-phenyl]-8-chloro-chroman-4-one (198 mg, compound **45b**) as the starting material instead of 2-[3-(3-aminopropoxy)-4-bromo-2-methoxy-phenyl]-8-chloro-chromen-4- (72 mg, compound **44b**) in **Step 3.** Ethyl 2-[2-[2-bromo-5-(8-chloro-4-oxo-chromen-2-yl)-4-methoxy-phenoxy]ethylamino]-2-*oxo*-acetate (100 mg, compound **45c**) was obtained as a yellow solid. MS obsd. (ESI⁺): 524.0 [(M+H)⁺], 526.0 [(M+2+H)⁺],

### Step 4. Synthesis of 2-[2-[2-bromo-5-(8-chloro-4-oxo-chromen-2-yl)-4-methoxy-phenoxy]ethylamino]-2-oxo-acetic acid

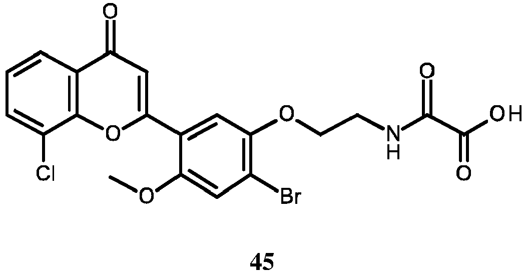

Example **45** was prepared in analogy to the procedure described for the preparation of example **44** by using ethyl 2-[2-[2-bromo-5-(8-chloro-4-*oxo*-chromen-2-yl)-4-methoxy-phenoxy]ethylamino]-2-oxo-acetate (100 mg, compound **45c**) as the starting material instead of ethyl 2-[3-[6-bromo-3-(8-chloro-4-*oxo*-chromen-2-yl)-2-methoxy-phenoxy]propylamino]-2-*oxo-*acetate (40 mg, compound **44c**) in **Step 4.** 2-[2-[2-Bromo-5-(8-chloro-4-oxo-chromen-2-yl)-4-methoxy-phenoxy]ethylamino]-2-*ox*o-acetic acid (30 mg, example **45**) was obtained as a yellow solid. ¹H NMR (400 MHz, DMSO-*d₆*) δ ppm 8.90 (t, *J* = 5.5 Hz, 1H), 8.00 (t, *J* = 6.9 Hz, 2H), 7.66 (s, 1H), 7.42-7.51 (m, 2H), 7.06 (s, 1H), 4.20 (t, *J* = 5.7 Hz, 2H), 3.92 (s, 3H), 3.58 (dd, *J*= 11.8, 6.0 Hz, 2H). MS obsd. (ESI⁺): 496.0 [(M+H)⁺], 498.0 [(M+2+H)⁺].

### Example 46

### 2-[3-[2-Bromo-5-(8-chloro-4-oxo-chromen-2-yl)-4-methoxy-phenoxy]propylamino]-2-oxo-acetic acid

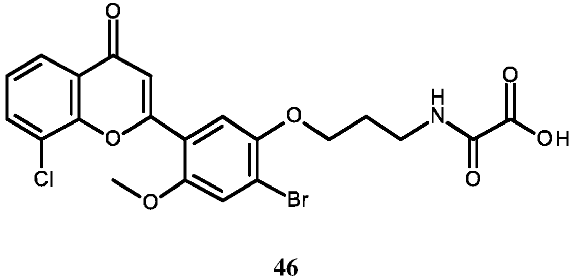

### Step 1. Synthesis of tert-butyl N-[3-[2-bromo-5-(8-chloro-4-oxo-chromen-2-yl)-4-methoxy-phenoxy]propyl]carbamate

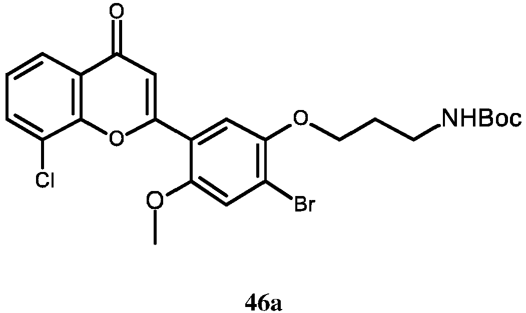

Compound **46a** was prepared in analogy to the procedure described for the preparation of example **44** by using 2-(4-bromo-5-hydroxy-2-methoxy-phenyl)-8-chloro-chromen-4-one (compound **1c**) as the starting material instead of 2-(4-bromo-3-hydroxy-2-methoxy-phenyl)-8-chloro-chromen-4-one (compound **34b**) in **Step 1.** *tert*-Butyl *N*-[3-[2-bromo-5-(8-chloro-4-oxo-chromen-2-yl)-4-methoxy-phenoxy]propyl]carbamate (170 mg, 60.2% yield, compound **45a**) was obtained as a yellow solid.

### Step 2. Synthesis of 2-[5-(3-aminopropoxy)-4-bromo-2-hydroxy-phenyl]-8-chloro-chroman-4-one

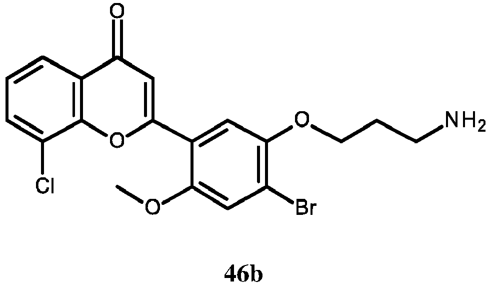

Compound **46b** was prepared in analogy to the procedure described for the preparation of example **44** by using *tert*-Butyl *N*-[3-[2-bromo-5-(8-chloro-4-oxo-chromen-2-yl)-4-methoxy-phenoxy]propyl]carbamate (compound **45a**) as the starting material instead of *tert*-butyl *N*-[3-[6-bromo-3-(8-chloro-4-*oxo*-chromen-2-yl)-2-methoxy-phenoxy]propyl]carbamate (compound **44a**) in **Step 2.** 2-[5-(3-aminopropoxy)-4-bromo-2-hydroxy-phenyl]-8-chloro-chroman-4-one (134 mg, compound **46b**) was obtained as a yellow solid. MS obsd. (ESI⁺): 438.0 [(M+H)⁺], 440.0 [(M+2+H)⁺],

### Step 3. Synthesis of ethyl 2-[3-[2-bromo-5-(8-chloro-4-oxo-chromen-2-yl)-4-methoxy-phenoxy]propylamino]-2-oxo-acetate

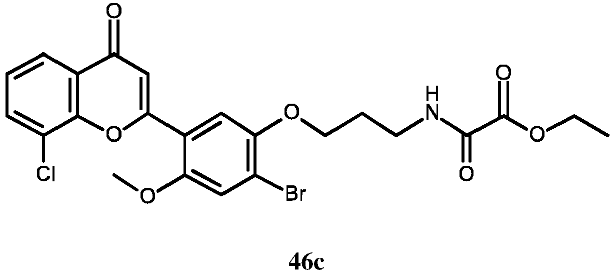

Compound **46c** was prepared in analogy to the procedure described for the preparation of example **44** by using 2-[5-(3-aminopropoxy)-4-bromo-2-hydroxy-phenyl]-8-chloro-chroman-4-one (134 mg, compound **46b**) as the starting material instead of 2-[3-(3-aminopropoxy)-4-bromo-2-methoxy-phenyl]-8-chloro-chromen-4-(compound **44b**) in **Step 3.** Ethyl 2-[3-[2-bromo-5-(8-chloro-4-oxo-chromen-2-yl)-4-methoxy-phenoxy]propylamino]-2-oxo-acetate (130 mg, 69% yield, compound **46c**) was obtained as a yellow solid. MS obsd. (ESI⁺): 538.1 [(M+H)⁺], 540.1 [(M+2+H)⁺].

### Step 4. Synthesis of 2-[3-[2-bromo-5-(8-chloro-4-oxo-chromen-2-yl)-4-methoxy-phenoxy]propylamino]-2-oxo-acetic acid

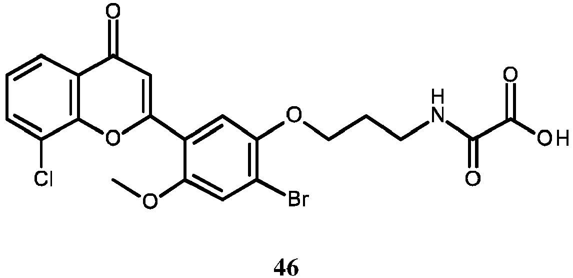

Example **46** was prepared in analogy to the procedure described for the preparation of example **44** by using ethyl 2-[3-[2-bromo-5-(8-chloro-4-oxo-chromen-2-yl)-4-methoxy-phenoxy]propylamino]-2-oxo-acetate (compound **46c**) as the starting material instead of ethyl 2-[3-[6-bromo-3-(8-chloro-4-*oxo*-chromen-2-yl)-2-methoxy-phenoxy]propylamino]-2-oxo-acetate (40 mg, compound **44c**) in **Step 4.** 2-[3-[2-Bromo-5-(8-chloro-4-oxo-chromen-2-yl)-4-methoxy-phenoxy]propylamino]-2-oxo-acetic acid (25 mg, 68% yield, example **46**) was obtained as a yellow solid. ¹H NMR (400 MHz, DMSO-*d₆*) δ ppm 13.73 (s, 1H), 8.85 (s, 1H), 8.00 (t, *J* = 6.8 Hz, 2H), 7.64-7.68 (m, 1H), 7.54 (s, 0.83H), 7.51 (t, *J* = 7.8 Hz, 1H), 7.42 (s, 0.18H), 7.07 (s, 1H), 4.11 (t, *J* = 5.7 Hz, 2H), 3.94 (s, 3H), 3.33-3.36 (m, 2H), 1.98 (t, *J* = 5.7 Hz, 2H). MS obsd. (ESI⁺): 510.0 [(M+H)⁺], 512.0 [(M+2+H)⁺].

### BIOLOGICAL EXAMPLES

### BIO-Example 1: Engineered HepDES 19 primary screen assay

The assay was employed to screen for novel cccDNA inhibitors. HepDES 19 is a cccDNA-producing cell line. In this cell line, HBeAg in the cell culture supernatant as surrogate marker, as HBeAg production depends on cccDNA level and activity. HepDES 19 is an engineered cell line which contains a 1.1 unit length HBV genome, and pgRNA transcription from the transgene is controlled by Tetracycline (Tet). In the absence of Tet, pgRNA transcription will be induced, but HBV e antigen (HBeAg) could not be produced from this pgRNA due to very short leader sequence before the HBeAg start codon and the start codon is disrupted. Only after cccDNA is formed, the missing leader sequence and start codon mutation would be restored from the 3'-terminal redundancy of pgRNA, and then HBeAg could be synthesized. Therefore, HBeAg could be used as a surrogate marker for cccDNA (Zhou, T. et al., Antiviral Res. (2006), 72(2), 116-124; Guo, H. et al., J. Virol. (2007), 81(22), 12472-12484).

HepDES19 cells were seeded at 2×10⁶ cells per T150 flask and cultured with the culture medium (Dulbecco's Modified Eagle Medium: Nutrient Mixture F-12 [DMEM-F12, Gibco Cat. 11320-82], 10% Fetal Bovine Serum [FBS, Clontech Cat. 631101], 0.1mM Non-Essential Amino Acids Solution [NEAA, Gibco Cat. 11140-050], 50µg/mL Penicillin-Streptomycin [PS, Invitrogen Cat. 15140-163], 500 µg/mL Geneticin [G418, Invitrogen Cat. 10131-027]) containing 3µg/mL Tet (Sigma, Cat. 87128) for 5 days. Cells were then seeded at 4×10⁶ cells per T150 in the same culture medium as described above in the absence of Tet for 8 days. Cells were then harvested and frozen at density of 2×10⁶ cells per mL. For compound testing, the frozen cells were thawed and seeded into 96-well plates at a density of 6 ×10⁴ cells per well. At 24hours after seeding, half log serial dilutions of compounds made with Dimethyl sulfoxide (DMSO, Sigma, Cat. D2650) were further diluted with the same culture medium as described above before they were added to the cells to reach desired final compound concentrations and 1% DMSO concentration. Plates were then incubated at 37 °C for another 5 days before measurement of HBeAg level and cell viability. Intracellular HBeAg level were measured with enzyme-linked immunosorbent assay (ELISA) kit (Shanghai Kehua Diagnostic Medical Products Co., Ltd). Cell viability was assessed using Cell Counting Kit-8 (DonJindo, Cat. CK04-20). IC₅₀ values were derived from the dose-response curve using 4 parameter logistic curve fit method.

The compounds of the present invention were tested for their capacity to inhibit extracellular HBeAg level as described herein. The compounds of this invention were found to have IC₅₀ below 50 µM. Particular compounds of formula (I) were found to have IC₅₀ below 5.0 µM. Results of HepDES 19 primary screen assay are given in Table 1.

**Table 1: Activity data in HepDES19 primary screen assay**

| Example No. | IC₅₀ (µM) | Example No. | IC₅₀ (µM) | Example No. | IC₅₀ (µM) |
|---|---|---|---|---|---|
| **1** | 3.18 | **17** | 0.72 | **33** | 6.75 |
| **2** | 1.19 | **18** | 0.52 | **34** | 0.94 |
| **3** | 0.58 | **19** | 7.93 | **35** | 0.19 |
| **4** | 1.96 | **20** | 2.14 | **36** | 3.13 |
| **5** | 6.08 | **21** | 4.12 | **37** | 10.1 |
| **6** | 1.66 | **22** | 0.32 | **38** | 1.13 |
| **7** | 4.51 | **23** | 7.36 | **39** | 0.11 |
| **8** | 1.50 | **24** | 1.46 | **40** | 1.39 |
| **9** | 5.20 | **25** | 7.51 | **41** | 0.133 |
| **10** | 1.54 | **26** | 1.07 | **42** | 2.63 |
| **11** | 2.43 | **27** | 3.24 | **43** | 11.7 |
| **12** | 0.62 | **28** | 0.56 | **44** | 1.44 |
| **13** | 0.19 | **29** | 3.24 | **45** | 1.40 |
| **14** | 6.32 | **30** | 7.51 | **46** | 0.61 |
| **15** | 0.49 | **31** | 4.66 | | |
| **16** | 6.36 | **32** | 7.13 | | |

### BIO-Example 2: Cryopreserved primary human hepatocytes (PHH) assay

This assay is used to confirm the anti-HBV effect of the compounds in HBV PHH infection assay. Cryopreserved PHH (BioreclamationIVT, Lot YJM) was thawed at 37°C and gently transferred into pre-warmed InVitroGRO HT medium(BioreclamationIVT, Cat. S03317). The mixture was centrifuged at 70 relative centrifugal force (RCF) for 3 minutes at RT, and the supernatant was discarded. Pre-warmed InVitroGRO CP medium (BioreclamationIVT, Cat# S03316) was added to the cell pellet to gently re-suspend cells. The cells were seeded at the density of 5.8 × 10⁴ cells per well to collagen I coated 96-well plate (Gibco, Cat. A1142803) with the InVitroGRO CP medium. All plates were incubated at 37°C with 5% CO₂ and 85% humidity.

At 20 hours after plating, the medium was changed to PHH culture medium (Dulbecco's Modified Eagle Medium (DMEM)/F12 (1:1) (Gibco, Cat. 11320-033), 10% fetal bovine serum (Gibco Cat. 10099141), 100 U/mL penicillin, 100 µg/mL streptomycin (Gibco, Cat. 151401-122), 5 ng/mL human epidermal growth factor (Invitrogen Cat. PHG0311L), 20 ng/mL dexamethasone (Sigma, Cat. D4902) and 250 ng/mL human recombinant insulin (Gibco, Cat. 12585-014)). And the cells were incubated at 37°C with 5% CO₂ and 85% humidity for 4 hours. The medium was then changed to pre-warmed PHH culture medium containing 4% polyethylene glycol (PEG) MW8000 (Sigma, Cat. P1458-50ML) and 1% DMSO (Sigma, Cat. D2650). 5.8 × 10⁶ genomic equivalents of HBV were added into the medium.

At 24 hours post-infection, the cells were gently washed with PBS and refreshed with PHH culture medium supplemented with 1% DMSO, and 0.25mg/mL Matrix gel (Corning, Cat. 356237) at 200µL per well. All plates were immediately placed in at 37°C CO₂ incubator.

24 hours later, serial dilutions of compounds made with DMSO were further diluted with the same culture medium (PHH culture medium supplemented with 1% DMSO and 0.25mg/mL Matrix gel as described above) before they were added to the cells to reach desired final compound concentrations and 1% DMSO concentration. The medium containing the compounds were refreshed every three days.

At 9 days post-compound treatment, extracellular HBsAg level were measured with Chemiluminescence Immuno Assay (CLIA) kit (Autobio, HBsAg Quantitative CLIA). Extracellular HBV DNA was extracted by MagNA Pure 96 system (Roche) and then determined by quantitative PCR with the following primers and probe:
HBV-Forward Primer (SEQ ID NO: 1): AAGAAAAACCCCGCCTGTAA (5' to 3');
HBV-Reverse Primer (SEQ ID NO: 2): CCTGTTCTGACTACTGCCTCTCC (5' to 3');
HBV-Probe: 5'+ tetramethylrhodamine + SEQ ID NO: 3 + black hole quencher 2-3', wherein SEQ ID NO: 3 is CCTGATGTGATGTTCTCCATGTTCAGC.

HBsAg IC₅₀ and HBV DNA IC₅₀ values were derived from the dose-response curve using 4 parameter logistic curve fit method. The compounds of formula (I) have HBsAg IC₅₀ <20 µM, particularly < 1 µM; and HBV DNA IC₅₀ < 50 µM. Results of Cryopreserved PHH assay are given in Table D2.

**Table D2: HBsAg IC₅₀ data in Cryopreserved PHH assay**

| Example No. | HBsAg IC₅₀ (µM) |
|---|---|
| **1** | 8.02 |
| **2** | 3.85 |
| **5** | 4.09 |
| **8** | 5.13 |
| **11** | 7.40 |
| **15** | 1.96 |
| **22** | 6.59 |
| **31** | 10.2 |
| **35** | 3.91 |
| **36** | 9.26 |

## Claims

1. A compound of the formula (I), 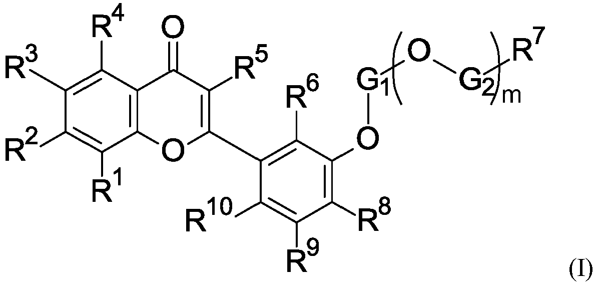 wherein
R¹ is halogen;
R² is selected from H, OH, halogen, C₁₋₆alkyl, haloC₁₋₆alkyl and C₁₋₆alkoxy;
R³ is selected from H, OH, halogen, C₁₋₆alkyl, haloC₁₋₆alkyl and C₁₋₆alkoxy;
R⁴ is selected from H, OH, halogen, C₁₋₆alkyl, haloC₁₋₆alkyl and C₁₋₆alkoxy;
R⁵ is selected from H, OH, halogen, C₁₋₆alkyl, haloC₁₋₆alkyl and C₁₋₆alkoxy;
R⁶ is selected from H, OH, halogen, C₁₋₆alkyl, haloC₁₋₆alkyl and C₁₋₆alkoxy;
R⁷ is selected from carboxy, C₁₋₆alkoxycarbonyl, carboxycarbonylamino, C₃₋₇cycloalkylsulfonylaminocarbonyl and C₁₋₆alkoxycarbonylcarbonylamino;
R⁸ is selected from H, OH, halogen, C₁₋₆alkyl, haloC₁₋₆alkyl and C₁₋₆alkoxy;
R⁹ is selected from H, OH, halogen, C₁₋₆alkyl, haloC₁₋₆alkyl and C₁₋₆alkoxy;
R¹⁰ is selected from H, OH, halogen, C₁₋₆alkyl, haloC₁₋₆alkyl, C₁₋₆alkoxy and haloC₁₋₆alkoxy;
G₁ is selected from C₁₋₆alkyl and C₃₋₇cycloalkyl;
G₂ is selected from C₁₋₆alkyl and C₃₋₇cycloalkyl;
m is selected from 0 and 1;
or a pharmaceutically acceptable salt thereof.

2. A compound according to claim 1, wherein
R¹ is halogen;
R² is H;
R³ is H;
R⁴ is H;
R⁵ is H;
R⁶ is selected from H and C₁₋₆alkoxy;
R⁷ is selected from carboxy, carboxycarbonylamino and C₃₋₇cycloalkylsulfonylaminocarbonyl;
R⁸ is selected from H, halogen, C₁₋₆alkyl, haloC₁₋₆alkyl and C₁₋₆alkoxy;
R⁹ is selected from H and C₁₋₆alkoxy;
R¹⁰ is selected from H, halogen, C₁₋₆alkoxy and haloC₁₋₆alkoxy;
G₁ is selected from C₁₋₆alkyl and C₃₋₇cycloalkyl;
G₂ is selected from C₁₋₆alkyl and C₃₋₇cycloalkyl;
m is selected from 0 and 1;
or a pharmaceutically acceptable salt thereof.

3. A compound according to claim 2, wherein
R¹ is Cl;
R² is H;
R³ is H;
R⁴ is H;
R⁵ is H;
R⁶ is selected from H and methoxy;
R⁷ is selected from carboxy, carboxycarbonylamino and cyclopropylsulfonylaminocarbonyl;
R⁸ is selected from H, Cl, Br, methyl, CF₃ and methoxy;
R⁹ is selected from H and methoxy;
R¹⁰ is selected from H, F, Cl, Br, methoxy, ethoxy, difluoromethoxy and trifluoromethoxy;
G₁ is selected from methyl, ethyl, propyl and cyclobutyl;
G₂ is selected from methyl and cyclobutyl;
m is selected from 0 and 1;
or a pharmaceutically acceptable salt thereof.

4. A compound according to claim 1 or 2, or a pharmaceutically acceptable salt thereof, wherein R⁷ is selected from carboxy and C₃₋₇cycloalkylsulfonylaminocarbonyl.

5. A compound according to any one of claims 1, 2, and 4, or a pharmaceutically acceptable salt thereof, wherein R⁹ is H.

6. A compound according to any one of claims 1, 2, 4 and 5, or a pharmaceutically acceptable salt thereof, wherein R¹⁰ is selected from H, halogen and C₁₋₆alkoxy.

7. A compound according to claim 1 or 2 having the formula (II), 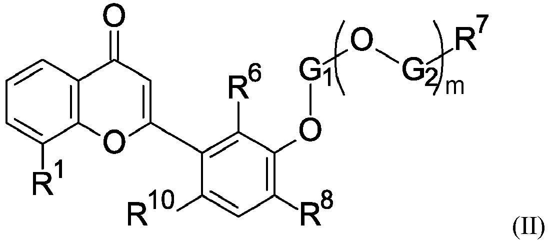 wherein
R¹ is halogen;
R⁶ is selected from H and C₁₋₆alkoxy;
R⁷ is selected from carboxy and C₃₋₇cycloalkylsulfonylaminocarbonyl;
R⁸ is selected from H, halogen, C₁₋₆alkyl, haloC₁₋₆alkyl and C₁₋₆alkoxy;
R¹⁰ is selected from H, halogen and C₁₋₆alkoxy;
G₁ is selected from C₁₋₆alkyl and C₃₋₇cycloalkyl;
G₂ is selected from C₁₋₆alkyl and C₃₋₇cycloalkyl;
m is selected from 0 and 1;
or a pharmaceutically acceptable salt thereof.

8. A compound according to claim 7, wherein
R¹ is Cl;
R⁶ is selected from H and methoxy;
R⁷ is selected from carboxy and cyclopropylsulfonylaminocarbonyl;
R⁸ is selected from H, Cl, Br, methyl, CF₃ and methoxy;
R¹⁰ is selected from H, F, methoxy and ethoxy;
G₁ is selected from methyl, ethyl, propyl and cyclobutyl;
G₂ is selected from methyl and cyclobutyl;
m is selected from 0 and 1;
or a pharmaceutically acceptable salt thereof.

9. A compound according to any one of claims 1 to 3, selected from
cis-3-[2-[2-bromo-5-(8-chloro-4-oxo-chromen-2-yl)-4-methoxy-phenoxy]ethoxy]cyclobutanecarboxylic acid;
3-[2-bromo-5-(8-chloro-4-oxo-chromen-2-yl)-4-methoxy-phenoxy]propanoic acid;
3-[2-bromo-5-(8-chloro-4-oxo-chromen-2-yl)-4-methoxy-phenoxy]-N-cyclopropylsulfonyl-propanamide;
2-[2-bromo-5-(8-chloro-4-oxo-chromen-2-yl)-4-methoxy-phenoxy]acetic acid;
3-[2-bromo-5-(8-chloro-4-oxo-chromen-2-yl)-4-methoxy-phenoxy]cyclobutanecarboxylic acid;
2-[3-[2-bromo-5-(8-chloro-4-oxo-chromen-2-yl)-4-methoxy-phenoxy]propoxy]acetic acid;
3-[2-chloro-5-(8-chloro-4-oxo-chromen-2-yl)-4-methoxy-phenoxy]cyclobutanecarboxylic acid;
cis-3-[2-[2-chloro-5-(8-chloro-4-oxo-chromen-2-yl)-4-methoxy-phenoxy]ethoxy]cyclobutanecarboxylic acid;
3-[2-[5-(8-chloro-4-oxo-chromen-2-yl)-4-methoxy-2-methyl-phenoxy]ethoxy]cyclobutanecarboxylic acid;
3-[5-(8-chloro-4-oxo-chromen-2-yl)-4-methoxy-2-methyl-phenoxy]propanoic acid;
2-[3-[5-(8-chloro-4-oxo-chromen-2-yl)-4-methoxy-2-methyl-phenoxy]propoxy]acetic acid;
3-[5-(8-chloro-4-oxo-chromen-2-yl)-2,4-dimethoxy-phenoxy]cyclobutanecarboxylic acid;
3-[2-[5-(8-chloro-4-oxo-chromen-2-yl)-2,4-dimethoxy-phenoxy]ethoxy]cyclobutanecarboxylic acid;
2-[5-(8-chloro-4-oxo-chromen-2-yl)-2-methyl-phenoxy] acetic acid;
cis-3-[5-(8-chloro-4-oxo-chromen-2-yl)-2-methyl-phenoxy]cyclobutanecarboxylic acid;
trans-3-[5-(8-chloro-4-oxo-chromen-2-yl)-2-methyl-phenoxy]cyclobutanecarboxylic acid;
3-[5-(8-chloro-4-oxo-chromen-2-yl)-2-methoxy-phenoxy]cyclobutanecarboxylic acid;
3-[2-[5-(8-chloro-4-oxo-chromen-2-yl)-2-methoxy-phenoxy]ethoxy]cyclobutanecarboxylic acid;
3-[5-(8-chloro-4-oxo-chromen-2-yl)-2-methoxy-phenoxy]propanoic acid;
cis-3-[5-(8-chloro-4-oxo-chromen-2-yl)-2-(trifluoromethyl)phenoxy]cyclobutanecarboxylic acid;
trans-3-[5-(8-chloro-4-oxo-chromen-2-yl)-2-(trifluoromethyl)phenoxy]cyclobutanecarboxylic acid;
2-[5-(8-chloro-4-oxo-chromen-2-yl)-2-(trifluoromethyl)phenoxy]acetic acid;
cis-3-[5-(8-chloro-4-oxo-chromen-2-yl)-2,3-dimethoxy-phenoxy]cyclobutanecarboxylic acid;
trans-3-[2-[5-(8-chloro-4-oxo-chromen-2-yl)-2,3-dimethoxy-phenoxy]ethoxy]cyclobutanecarboxylic acid;
2-[5-(8-chloro-4-oxo-chromen-2-yl)-2,3-dimethoxy-phenoxy]acetic acid;
trans-3-[4-bromo-5-(8-chloro-4-oxo-chromen-2-yl)-2-methoxy-phenoxy]cyclobutanecarboxylic acid;
trans-3-[2-[4-bromo-5-(8-chloro-4-oxo-chromen-2-yl)-2-methoxy-phenoxy]ethoxy]cyclobutanecarboxylic acid;
3-[3-(8-chloro-4-oxo-chromen-2-yl)-2-methoxy-phenoxy]cyclobutanecarboxylic acid;
cis-3-[2-[3-(8-chloro-4-oxo-chromen-2-yl)-2-methoxy-phenoxy]ethoxy]cyclobutanecarboxylic acid;
3-[3-(8-chloro-4-oxo-chromen-2-yl)-4-methoxy-phenoxy]cyclobutanecarboxylic acid;
cis-3-[2-[3-(8-chloro-4-oxo-chromen-2-yl)-4-methoxy-phenoxy]ethoxy]cyclobutanecarboxylic acid;
2-[3-(8-chloro-4-oxo-chromen-2-yl)-4-methoxy-phenoxy]acetic acid;
3-[3-(8-chloro-4-oxo-chromen-2-yl)-4-methoxy-phenoxy]propanoic acid;
cis-3-[2-[6-bromo-3-(8-chloro-4-oxo-chromen-2-yl)-2-methoxy-phenoxy]ethoxy]cyclobutanecarboxylic acid;
3-[6-bromo-3-(8-chloro-4-oxo-chromen-2-yl)-2-methoxy-phenoxy]cyclobutanecarboxylic acid;
3-[3-(8-chloro-4-oxo-chromen-2-yl)-2-methoxy-6-methyl-phenoxy]cyclobutanecarboxylic acid;
2-[6-chloro-3-(8-chloro-4-oxo-chromen-2-yl)-2-methoxy-phenoxy]acetic acid;
2-[3-(8-chloro-4-oxo-chromen-2-yl)-2,6-dimethoxy-phenoxy]acetic acid;
3-[2-bromo-5-(8-chloro-4-oxo-chromen-2-yl)-4-ethoxy-phenoxy]propanoic acid;
3-[2-bromo-5-(8-chloro-4-oxo-chromen-2-yl)-4-(difluoromethoxy)phenoxy]propanoic acid;
3-[2-bromo-5-(8-chloro-4-oxo-chromen-2-yl)-4-fluoro-phenoxy]propanoic acid;
3-[2-bromo-5-(8-chloro-4-oxo-chromen-2-yl)-4-(trifluoromethoxy)phenoxy]propanoic acid;
3-[2-bromo-4-chloro-5-(8-chloro-4-oxo-chromen-2-yl)phenoxy]propanoic acid;
2-[3-[6-bromo-3-(8-chloro-4-oxo-chromen-2-yl)-2-methoxy-phenoxy]propylamino]-2-oxo-acetic acid;
2-[2-[2-bromo-5-(8-chloro-4-oxo-chromen-2-yl)-4-methoxy-phenoxy]ethylamino]-2-oxo-acetic acid; and
2-[3-[2-bromo-5-(8-chloro-4-oxo-chromen-2-yl)-4-methoxy-phenoxy]propylamino]-2-oxo-acetic acid;
or a pharmaceutically acceptable salt thereof.

10. A compound according to any one of claims 1 to 8, selected from
3-[2-bromo-5-(8-chloro-4-oxo-chromen-2-yl)-4-methoxy-phenoxy]propanoic acid;
3-[2-bromo-5-(8-chloro-4-oxo-chromen-2-yl)-4-methoxy-phenoxy]-N-cyclopropylsulfonyl-propanamide;
2-[2-bromo-5-(8-chloro-4-oxo-chromen-2-yl)-4-methoxy-phenoxy]acetic acid;
2-[3-[2-bromo-5-(8-chloro-4-oxo-chromen-2-yl)-4-methoxy-phenoxy]propoxy]acetic acid;
cis-3-[2-[2-chloro-5-(8-chloro-4-oxo-chromen-2-yl)-4-methoxy-phenoxy]ethoxy]cyclobutanecarboxylic acid;
3-[5-(8-chloro-4-oxo-chromen-2-yl)-2,4-dimethoxy-phenoxy]cyclobutanecarboxylic acid;
3-[2-[5-(8-chloro-4-oxo-chromen-2-yl)-2,4-dimethoxy-phenoxy]ethoxy]cyclobutanecarboxylic acid;
cis-3-[5-(8-chloro-4-oxo-chromen-2-yl)-2-methyl-phenoxy]cyclobutanecarboxylic acid;
2-[5-(8-chloro-4-oxo-chromen-2-yl)-2-(trifluoromethyl)phenoxy]acetic acid;
3-[3-(8-chloro-4-oxo-chromen-2-yl)-2-methoxy-phenoxy]cyclobutanecarboxylic acid;
cis-3-[2-[6-bromo-3-(8-chloro-4-oxo-chromen-2-yl)-2-methoxy-phenoxy]ethoxy]cyclobutanecarboxylic acid;
3-[6-bromo-3-(8-chloro-4-oxo-chromen-2-yl)-2-methoxy-phenoxy]cyclobutanecarboxylic acid;
2-[3-(8-chloro-4-oxo-chromen-2-yl)-2,6-dimethoxy-phenoxy]acetic acid;
3-[2-bromo-5-(8-chloro-4-oxo-chromen-2-yl)-4-ethoxy-phenoxy]propanoic acid; and
3-[2-bromo-5-(8-chloro-4-oxo-chromen-2-yl)-4-fluoro-phenoxy]propanoic acid;
or a pharmaceutically acceptable salt thereof.

11. A process for the preparation of a compound according to any one of claims 1 to 10 comprising at least one of the following steps:
(a) Hydrolysis of a compound of formula (III-6), 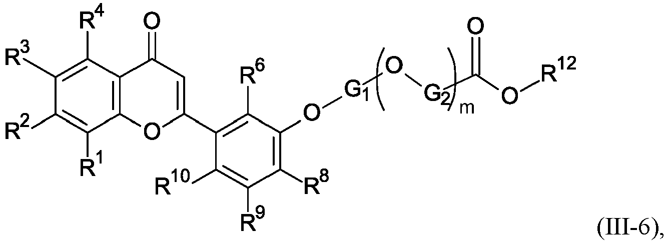 in the presence of a base or an acid;
(b) Hydrolysis of a compound of formula (IV-1), 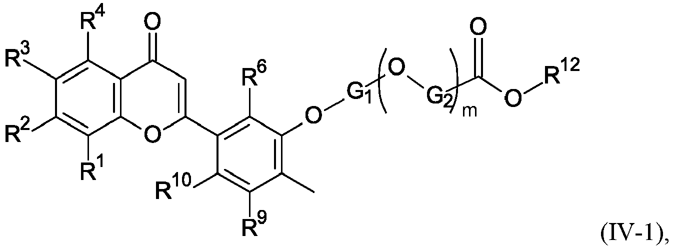 in the presence of a base or an acid;
(c) Reaction of a compound of formula (III-4), 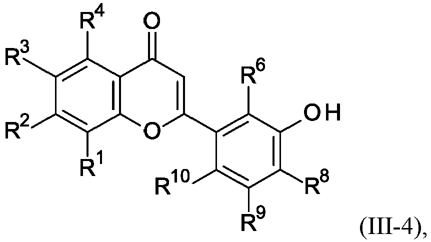 with oxetan-2-one in the presence of a base;
(d) Oxidation of a compound of formula (V-1), 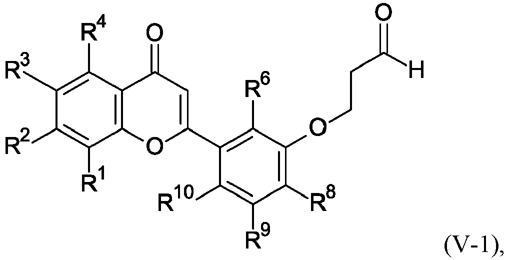 with NaClO₂;
(e) Reaction of a compound of formula (III-7), 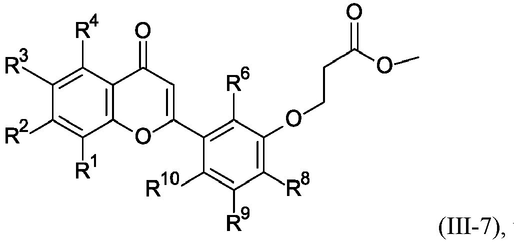 with sulfonamide in the presence of a Lewis acid;
(f) Hydrolysis of a compound of formula (VI-3), 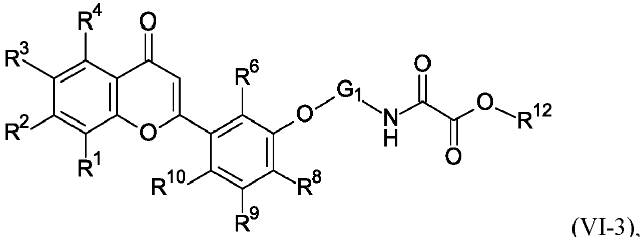 in the presence of a base; wherein R¹ to R⁴, R⁶, R⁸ to R¹⁰, Gi, G₂ and m are defined as any one of claims 1 to 3; R¹² is C₁₋₆alkyl.

12. A compound according to any one of claims 1 to 10 for use as therapeutically active substance.

13. A pharmaceutical composition comprising a compound in accordance with any one of claims 1 to 10 and a therapeutically inert carrier.

14. A compound according to any one of claims 1 to 10 for use in the treatment or prophylaxis of HBV infection.

15. A compound according to any one of claims 1 to 10, when manufactured according to a process of claim 11.

## Patentansprüche

1. Verbindung der Formel (I), 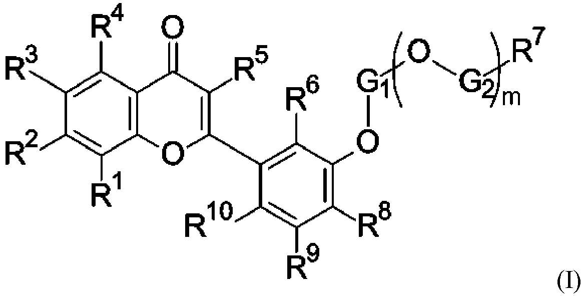 wobei
R¹ Halogen ist;
R² ausgewählt ist aus H, OH, Halogen, C₁₋₆-Alkyl, HaloC₁₋₆-Alkyl und C₁₋₆-Alkoxy;
R³ ausgewählt ist aus H, OH, Halogen, C₁₋₆-Alkyl, HaloC₁₋₆-Alkyl und C₁₋₆-Alkoxy;
R⁴ ausgewählt ist aus H, OH, Halogen, C₁₋₆-Alkyl, HaloC₁₋₆-Alkyl und C₁₋₆-Alkoxy;
R⁵ ausgewählt ist aus H, OH, Halogen, C₁₋₆-Alkyl, HaloC₁₋₆-Alkyl und C₁₋₆-Alkoxy;
R⁶ ausgewählt ist aus H, OH, Halogen, C₁₋₆-Alkyl, HaloC₁₋₆-Alkyl und C₁₋₆-Alkoxy;
R⁷ ausgewählt ist aus Carboxy, C₁₋₆-Alkoxycarbonyl, Carboxycarbonylamino, C₃₋₇-Cycloalkylsulfonylaminocarbonyl und C₁₋₆-Alkoxycarbonylcarbonylamino;
R⁸ ausgewählt ist aus H, OH, Halogen, C₁₋₆-Alkyl, HaloC₁₋₆-Alkyl und C₁₋₆-Alkoxy;
R⁹ ausgewählt ist aus H, OH, Halogen, C₁₋₆-Alkyl, HaloC₁₋₆-Alkyl und C₁₋₆-Alkoxy;
R¹⁰ ausgewählt ist aus H, OH, Halogen, C₁₋₆-Alkyl, HaloC₁₋₆-Alkyl, C₁₋₆-Alkoxy und HaloC₁₋₆-Alkoxy;
G₁ ausgewählt ist aus C₁₋₆-Alkyl und C₃₋₇-Cycloalkyl;
G₂ ausgewählt ist aus C₁₋₆-Alkyl und C₃₋₇-Cycloalkyl;
m ausgewählt ist aus 0 und 1;
oder ein pharmazeutisch akzeptables Salz davon.

2. Verbindung nach Anspruch 1, wobei
R¹ Halogen ist;
R² H ist;
R³ H ist;
R⁴ H ist;
R⁵ H ist;
R⁶ ausgewählt ist aus H und C₁₋₆-Alkoxy;
R⁷ ausgewählt ist aus Carboxy, Carboxycarbonylamino und C₃₋₇-Cycloalkylsulfonylaminocarbonyl;
R⁸ ausgewählt ist aus H, Halogen, C₁₋₆-Alkyl, HaloC₁₋₆-Alkyl und C₁₋₆-Alkoxy;
R⁹ ausgewählt ist aus H und C₁₋₆-Alkoxy;
R¹⁰ ausgewählt ist aus H, Halogen, C₁₋₆-Alkoxy und HaloC₁₋₆-Alkoxy;
G₁ ausgewählt ist aus C₁₋₆-Alkyl und C₃₋₇-Cycloalkyl;
G₂ ausgewählt ist aus C₁₋₆-Alkyl und C₃₋₇-Cycloalkyl;
m ausgewählt ist aus 0 und 1;
oder ein pharmazeutisch akzeptables Salz davon.

3. Verbindung gemäß Anspruch 2, wobei
R¹ Cl ist;
R² H ist;
R³ H ist;
R⁴ H ist;
R⁵ H ist;
R⁶ ausgewählt ist aus H und Methoxy;
R⁷ ausgewählt ist aus Carboxy, Carboxycarbonylamino und Cyclopropylsulfonylaminocarbonyl;
R⁸ ausgewählt ist aus H, Cl, Br, Methyl, CF₃ und Methoxy;
R⁹ ausgewählt ist aus H und Methoxy;
R¹⁰ ausgewählt ist aus H, F, Cl, Br, Methoxy, Ethoxy, Difluormethoxy und Trifluormethoxy;
G₁ ausgewählt ist aus Methyl, Ethyl, Propyl und Cyclobutyl;
G₂ ausgewählt ist aus Methyl und Cyclobutyl;
m ausgewählt ist aus 0 und 1;
oder ein pharmazeutisch akzeptables Salz davon.

4. Verbindung gemäß Anspruch 1 oder 2 oder ein pharmazeutisch akzeptables Salz davon, wobei
R⁷ ausgewählt ist aus Carboxy und C₃₋₇-Cycloalkylsulfonylaminocarbonyl.

5. Verbindung gemäß einem beliebigen der Ansprüche 1, 2 und 4 oder ein pharmazeutisch akzeptables Salz davon, wobei R⁹ H ist.

6. Verbindung gemäß einem beliebigen der Ansprüche 1, 2, 4 und 5 oder ein pharmazeutisch akzeptables Salz davon, wobei R¹⁰ ausgewählt ist aus H, Halogen und C₁₋₆-Alkoxy.

7. Verbindung gemäß Anspruch 1 oder 2 mit der Formel (II), 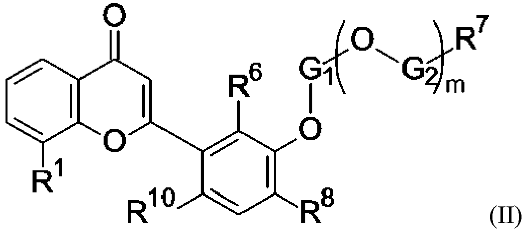 wobei
R¹ Halogen ist;
R⁶ ausgewählt ist aus H und C₁₋₆-Alkoxy;
R⁷ ausgewählt ist aus Carboxy und C₃₋₇-Cycloalkylsulfonylaminocarbonyl;
R⁸ ausgewählt ist aus H, Halogen, C₁₋₆-Alkyl, HaloC₁₋₆-Alkyl und C₁₋₆-Alkoxy;
R¹⁰ ausgewählt ist aus H, Halogen und C₁₋₆-Alkoxy;
G₁ ausgewählt ist aus C₁₋₆-Alkyl und C₃₋₇-Cycloalkyl;
G₂ ausgewählt ist aus C₁₋₆-Alkyl und C₃₋₇-Cycloalkyl;
m ausgewählt ist aus 0 und 1;
oder ein pharmazeutisch akzeptables Salz davon.

8. Verbindung gemäß Anspruch 7, wobei
R¹ Cl ist;
R⁶ ausgewählt ist aus H und Methoxy;
R⁷ ausgewählt ist aus Carboxy und Cyclopropylsulfonylaminocarbonyl;
R⁸ ausgewählt ist aus H, Cl, Br, Methyl, CF₃ und Methoxy;
R¹⁰ ausgewählt ist aus H, F, Methoxy und Ethoxy;
G₁ ausgewählt ist aus Methyl, Ethyl, Propyl und Cyclobutyl;
G₂ ausgewählt ist aus Methyl und Cyclobutyl;
m ausgewählt ist aus 0 und 1;
oder ein pharmazeutisch akzeptables Salz davon.

9. Verbindung gemäß einem beliebigen der Ansprüche 1 bis 3, ausgewählt aus cis-3-[2-[2-Brom-5-(8-chlor-4-oxo-chromen-2-yl)-4-methoxy-phenoxy]ethoxy]cyclobutancarbonsäure;
3-[2-Brom-5-(8-chlor-4-oxo-chromen-2-yl)-4-methoxy-phenoxy]propansäure;
3-[2-Brom-5-(8-chlor-4-oxo-chromen-2-yl)-4-methoxy-phenoxy]-N-cyclopropylsulfonyl-propanamid;
2-[2-Brom-5-(8-chlor-4-oxo-chromen-2-yl)-4-methoxy-phenoxy]essigsäure;
3-[2-Brom-5-(8-chlor-4-oxo-chromen-2-yl)-4-methoxy-phenoxy]cyclobutancarbonsäure;
2-[3-Brom-5-(8-chlor-4-oxo-chromen-2-yl)-4-methoxy-phenoxy]propoxy]essigsäure;
3-[2-Chlor-5-(8-chlor-4-oxo-chromen-2-yl)-4-methoxy-phenoxy]cyclobutancarbonsäure;
cis-3-[2-[2-Chlor-5-(8-chlor-4-oxo-chromen-2-yl)-4-methoxy-phenoxy]ethoxy]cyclobutancarbonsäure;
3-[2-[5-(8-Chlor-4-oxo-chromen-2-yl)-4-methoxy-2-methyl-phenoxy]ethoxy]cyclobutancarbonsäure;
3-[5-(8-Chlor-4-oxo-chromen-2-yl)-4-methoxy-2-methyl-phenoxy]propansäure;
2-[3-[5-(8-Chlor-4-oxo-chromen-2-yl)-4-methoxy-2-methyl-phenoxy]propoxy]essigsäure;
3-[5-(8-Chlor-4-oxo-chromen-2-yl)-2,4-dimethoxy-phenoxy]cyclobutancarbonsäure;
3-[2-[5-(8-Chlor-4-oxo-chromen-2-yl)-2,4-dimethoxy-phenoxy]ethoxy]cyclobutancarbonsäure;
2-[5-(8-Chlor-4-oxo-chromen-2-yl)-2-methyl-phenoxy]essigsäure;
cis-3-[5-(8-Chlor-4-oxo-chromen-2-yl)-2-methyl-phenoxy]cyclobutancarbonsäure;
trans-3-[5-(8-Chlor-4-oxo-chromen-2-yl)-2-methyl-phenoxy]cyclobutancarbonsäure;
3-[5-(8-Chlor-4-oxo-chromen-2-yl)-2-methoxy-phenoxy]cyclobutancarbonsäure;
3-[2-[5-(8-Chlor-4-oxo-chromen-2-yl)-2-methoxy-phenoxy]ethoxy]cyclobutancarbonsäure;
3-[5-(8-Chlor-4-oxo-chromen-2-yl)-2-methoxy-phenoxy]propansäure;
cis-3-[5-(8-Chlor-4-oxo-chromen-2-yl)-2-(trifluormethyl)phenoxy]cyclobutancarbonsäure;
trans-3-[5-(8-Chlor-4-oxo-chromen-2-yl)-2-(trifluormethyl)phenoxy]cyclobutancarbonsäure;
2-[5-(8-Chlor-4-oxo-chromen-2-yl)-2-(trifluormethyl)phenoxy]essigsäure;
cis-3-[5-(8-Chlor-4-oxo-chromen-2-yl)-2,3-dimethoxy-phenoxy]cyclobutancarbonsäure;
trans-3-[2-[5-(8-Chlor-4-oxo-chromen-2-yl)-2,3-dimethoxy-phenoxy]ethoxy]cyclobutancarbonsäure;
2-[5-(8-Chlor-4-oxo-chromen-2-yl)-2,3-dimethoxy-phenoxy]essigsäure;
trans-3-[4-Brom-5-(8-chlor-4-oxo-chromen-2-yl)-2-methoxy-phenoxy]cyclobutancarbonsäure;
trans-3-[2-[4-Brom-5-(8-chlor-4-oxo-chromen-2-yl)-2-methoxy-phenoxy] ethoxy] cyclobutancarbonsäure;
3-[3-(8-Chlor-4-oxo-chromen-2-yl)-2-methoxy-phenoxy]cyclobutancarbonsäure;
cis-3-[2-[3-(8-Chlor-4-oxo-chromen-2-yl)-2-methoxy-phenoxy] ethoxy] cyclobutancarbonsäure;
3-[3-(8-Chlor-4-oxo-chromen-2-yl)-4-methoxy-phenoxy]cyclobutancarbonsäure;
cis-3-[2-[3-(8-Chlor-4-oxo-chromen-2-yl)-4-methoxy-phenoxy] ethoxy] cyclobutancarbonsäure;
2-[3-(8-Chlor-4-oxo-chromen-2-yl)-4-methoxy-phenoxy]essigsäure;
3-[3-(8-Chlor-4-oxo-chromen-2-yl)-4-methoxy-phenoxy]propansäure;
cis-3-[2-[6-Brom-3-(8-chlor-4-oxo-chromen-2-yl)-2-methoxy-phenoxy] ethoxy] cyclobutancarbonsäure;
3-[6-Brom-3-(8-chlor-4-oxo-chromen-2-yl)-2-methoxy-phenoxy]cyclobutancarbonsäure;
3-[3-(8-Chlor-4-oxo-chromen-2-yl)-2-methoxy-6-methyl-phenoxy]cyclobutancarbonsäure;
2-[6-Chlor-3-(8-chlor-4-oxo-chromen-2-yl)-2-methoxy-phenoxy]essigsäure;
2-[3-(8-Chlor-4-oxo-chromen-2-yl)-2,6-dimethoxy-phenoxy]essigsäure;
3-[2-Brom-5-(8-chlor-4-oxo-chromen-2-yl)-4-ethoxy-phenoxy]propansäure;
3-[2-Brom-5-(8-chlor-4-oxo-chromen-2-yl)-4-(difluormethoxy)phenoxy]propansäure;
3-[2-Brom-5-(8-chlor-4-oxo-chromen-2-yl)-4-fluor-phenoxy]propansäure;
3-[2-Brom-5-(8-chlor-4-oxo-chromen-2-yl)-4-(trifluormethoxy)phenoxy]propansäure;
3-[2-Brom-4-chlor-5-(8-chlor-4-oxo-chromen-2-yl)phenoxy]propansäure;
2-[3-[6-Brom-3-(8-chlor-4-oxo-chromen-2-yl)-2-methoxy-phenoxy]propylamino]-2-oxo-essigsäure;
2-[2-[2-Brom-5-(8-chlor-4-oxo-chromen-2-yl)-4-methoxy-phenoxy]ethylamino]-2-oxo-essigsäure; und
2-[3-[2-Brom-5-(8-chlor-4-oxo-chromen-2-yl)-4-methoxy-phenoxy]propylamino]-2-oxo-essigsäure;
oder ein pharmazeutisch akzeptables Salz davon.

10. Verbindung gemäß einem beliebigen der Ansprüche 1 bis 8, ausgewählt aus
3-[2-Brom-5-(8-chlor-4-oxo-chromen-2-yl)-4-methoxy-phenoxy]propansäure;
3-[2-Brom-5-(8-chlor-4-oxo-chromen-2-yl)-4-methoxy-phenoxy]-N-cyclopropylsulfonyl-propanamid;
2-[2-Brom-5-(8-chlor-4-oxo-chromen-2-yl)-4-methoxy-phenoxy]essigsäure;
2-[3-[2-Brom-5-(8-chlor-4-oxo-chromen-2-yl)-4-methoxy-phenoxy]propoxy]essigsäure;
cis-3-[2-[2-Chlor-5-(8-chlor-4-oxo-chromen-2-yl)-4-methoxy-phenoxy] ethoxy] cyclobutancarbonsäure;
3-[5-(8-Chlor-4-oxo-chromen-2-yl)-2,4-dimethoxy-phenoxy]cyclobutancarbonsäure;
3-[2-[5-(8-Chlor-4-oxo-chromen-2-yl)-2,4-dimethoxy-phenoxy] ethoxy] cyclobutancarbonsäure;
cis-3-[5-(8-Chlor-4-oxo-chromen-2-yl)-2-methyl-phenoxy]cyclobutancarbonsäure;
2-[5-(8-Chlor-4-oxo-chromen-2-yl)-2-(trifluormethyl)phenoxy]essigsäure;
3-[3-(8-Chlor-4-oxo-chromen-2-yl)-2-methoxy-phenoxy]cyclobutancarbonsäure;
cis-3-[2-[6-Brom-3-(8-chlor-4-oxo-chromen-2-yl)-2-methoxy-phenoxy] ethoxy] cyclobutancarbonsäure;
3-[6-Brom-3-(8-chlor-4-oxo-chromen-2-yl)-2-methoxy-phenoxy]cyclobutancarbonsäure;
2-[3-(8-Chlor-4-oxo-chromen-2-yl)-2,6-dimethoxy-phenoxy]essigsäure;
3-[2-Brom-5-(8-chlor-4-oxo-chromen-2-yl)-4-ethoxy-phenoxy]propansäure; und
3-[2-Brom-5-(8-chlor-4-oxo-chromen-2-yl)-4-fluoro-phenoxy]propansäure;
oder ein pharmazeutisch akzeptables Salz davon.

11. Verfahren zur Herstellung einer Verbindung gemäß einem der Ansprüche 1 bis 10, umfassend mindestens einen der folgenden Schritte:
(a) Hydrolyse einer Verbindung der Formel (III-6), 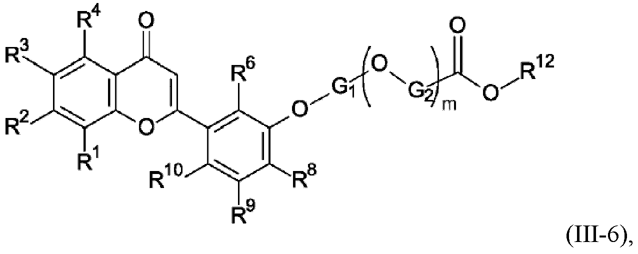 in Gegenwart einer Base oder einer Säure;
(b) Hydrolyse einer Verbindung der Formel (IV-1), 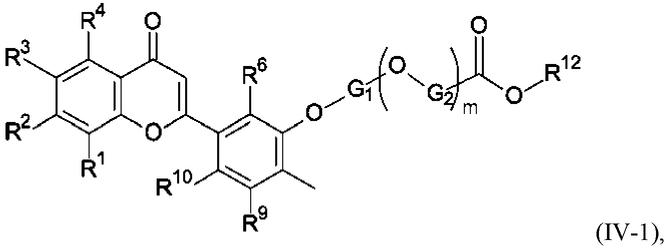 in Gegenwart einer Base oder einer Säure;
(c) Reaktion einer Verbindung der Formel (III-4), 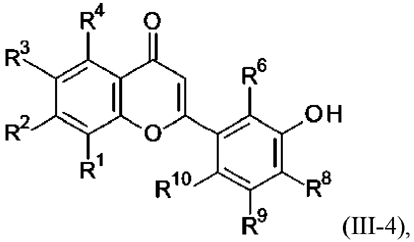 mit Oxetan-2-on in Gegenwart einer Base;
(d) Oxidation einer Verbindung der Formel (V-1), 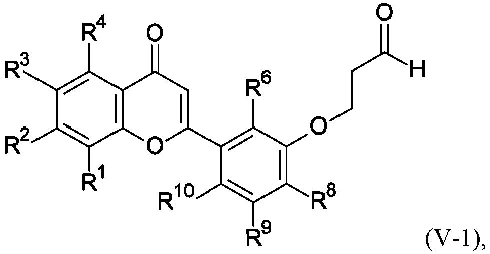 mit NaClO₂;
(e) Reaktion einer Verbindung der Formel (III-7), 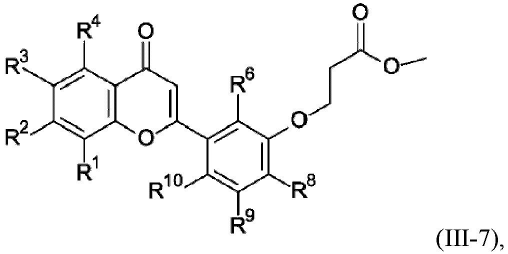 mit Sulfonamid in Gegenwart einer LewisSäure;
(f) Hydrolyse einer Verbindung der Formel (VI-3), 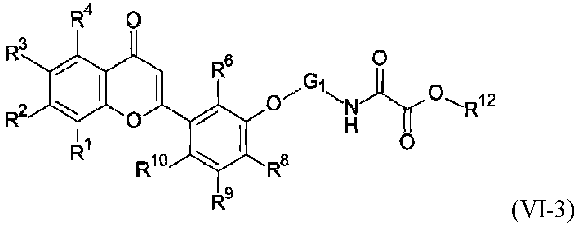 in Gegenwart einer Base; wobei R¹ bis R⁴, R⁶, R⁸ bis R¹⁰, Gi, G₂ und m wie in einem beliebigen der Ansprüche 1 bis 3 definiert sind; R¹² ist C₁₋₆-Alkyl.

12. Verbindung gemäß einem beliebigen der Ansprüche 1 bis 10 zur Verwendung als therapeutisch wirksame Substanz.

13. Pharmazeutische Zusammensetzung, umfassend eine Verbindung in Übereinstimmung mit einem beliebigen der Ansprüche 1 bis 10 und einen therapeutisch inerten Träger.

14. Verbindung gemäß einem beliebigen der Ansprüche 1 bis 10 zur Verwendung bei der Behandlung oder Prophylaxe einer HBV-Infektion.

15. Verbindung gemäß einem beliebigen der Ansprüche 1 bis 10, wenn sie nach einem Verfahren gemäß Anspruch 11 hergestellt wird.

## Revendications

1. Composé de formule (I), 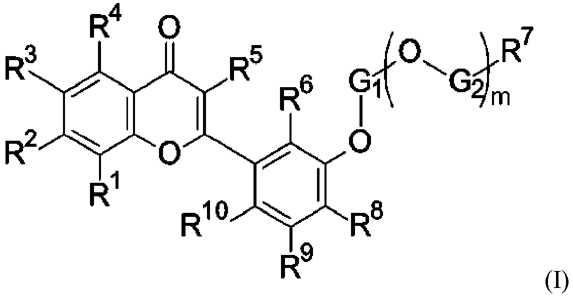 dans lequel
R¹ représente un halogène ;
R² est choisi parmi H, OH, un halogène, un alkyle en C₁₋₆, un halogénoalkyle en C₁₋₆ et un alcoxy en C₁₋₆ ;
R³ est choisi parmi H, OH, un halogène, un alkyle en C₁₋₆, un halogénoalkyle en C₁₋₆ et un alcoxy en C₁₋₆ ;
R⁴ est choisi parmi H, OH, un halogène, un alkyle en C₁₋₆, un halogénoalkyle en C₁₋₆ et un alcoxy en C₁₋₆ ;
R⁵ est choisi parmi H, OH, un halogène, un alkyle en C₁₋₆, un halogénoalkyle en C₁₋₆ et un alcoxy en C₁₋₆ ;
R⁶ est choisi parmi H, OH, un halogène, un alkyle en C₁₋₆, un halogénoalkyle en C₁₋₆ et un alcoxy en C₁₋₆ ;
R⁷ est choisi parmi un carboxy, un alcoxy en C₁₋₆-carbonyle, un carboxycarbonylamino, un cycloalkyle en C₃₋₇-sulfonylaminocarbonyle et un alcoxy en C₁₋₆-carbonylcarbonylamino ;
R⁸ est choisi parmi H, OH, un halogène, un alkyle en C₁₋₆, un halogénoalkyle en C₁₋₆ et un alcoxy en C₁₋₆ ;
R⁹ est choisi parmi H, OH, un halogène, un alkyle en C₁₋₆, un halogénoalkyle en C₁₋₆ et un alcoxy en C₁₋₆ ;
R¹⁰ est choisi parmi H, OH, un halogène, un alkyle en C₁₋₆, un halogénoalkyle en C₁₋₆, un alcoxy en C₁₋₆ et un halogénoalcoxy en C₁₋₆ ;
G₁ est choisi parmi un alkyle en C₁₋₆ et un cycloalkyle en C₃₋₇ ;
G₂ est choisi parmi un alkyle en C₁₋₆ et un cycloalkyle en C₃₋₇ ;
m est choisi parmi 0 et 1 ;
ou un sel pharmaceutiquement acceptable de celui-ci.

2. Composé selon la revendication 1, dans lequel
R¹ représente un halogène ;
R² représente H ;
R³ représente H ;
R⁴ représente H ;
R⁵ représente H ;
R⁶ est choisi parmi H et un alcoxy en C₁₋₆ ;
R⁷ est choisi parmi un carboxy, un carboxycarbonylamino et un cycloalkyle en C₃₋₇-sulfonylaminocarbonyle ;
R⁸ est choisi parmi H, un halogène, un alkyle en C₁₋₆, un halogénoalkyle en C₁₋₆ et un alcoxy en C₁₋₆ ;
R⁹ est choisi parmi H et un alcoxy en C₁₋₆ ;
R¹⁰ est choisi parmi H, un halogène, un alcoxy en C₁₋₆ et un halogénoalcoxy en C₁₋₆ ;
G₁ est choisi parmi un alkyle en C₁₋₆ et un cycloalkyle en C₃₋₇ ;
G₂ est choisi parmi un alkyle en C₁₋₆ et un cycloalkyle en C₃₋₇ ;
m est choisi parmi 0 et 1 ;
ou un sel pharmaceutiquement acceptable de celui-ci.

3. Composé selon la revendication 2, dans lequel
R¹ représente Cl ;
R² représente H ;
R³ représente H ;
R⁴ représente H ;
R⁵ représente H ;
R⁶ est choisi parmi H et un méthoxy ;
R⁷ est choisi parmi un carboxy, un carboxycarbonylamino et un cyclopropylsulfonylaminocarbonyle ;
R⁸ est choisi parmi H, Cl, Br, un méthyle, CF₃ et un méthoxy ;
R⁹ est choisi parmi H et un méthoxy ;
R¹⁰ est choisi parmi H, F, Cl, Br, un méthoxy, un éthoxy, un difluorométhoxy et un trifluorométhoxy ;
G₁ est choisi parmi un méthyle, un éthyle, un propyle et un cyclobutyle ;
G₂ est choisi parmi un méthyle et un cyclobutyle ;
m est choisi parmi 0 et 1 ;
ou un sel pharmaceutiquement acceptable de celui-ci.

4. Composé selon la revendication 1 ou 2, ou un sel pharmaceutiquement acceptable de celui-ci, dans lequel
R⁷ est choisi parmi un carboxy et un cycloalkyle en C₃₋₇-sulfonylaminocarbonyle.

5. Composé selon l'une quelconque des revendications 1, 2 et 4, ou un sel pharmaceutiquement acceptable de celui-ci, dans lequel R⁹ représente H.

6. Composé selon l'une quelconque des revendications 1, 2, 4 et 5, ou un sel pharmaceutiquement acceptable de celui-ci, dans lequel R¹⁰ est choisi parmi H, un halogène et un alcoxy en C₁₋₆.

7. Composé selon la revendication 1 ou 2 ayant la formule (II), 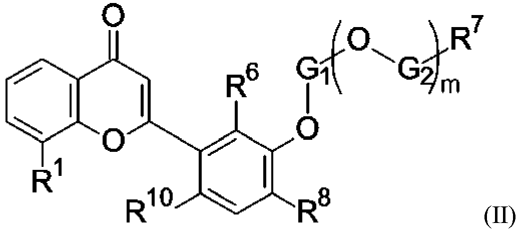 dans lequel
R¹ représente un halogène ;
R⁶ est choisi parmi H et un alcoxy en C₁₋₆ ;
R⁷ est choisi parmi un carboxy et un cycloalkyle en C₃₋₇-sulfonylaminocarbonyle ;
R⁸ est choisi parmi H, un halogène, un alkyle en C₁₋₆, un halogénoalkyle en C₁₋₆ et un alcoxy en C₁₋₆ ;
R¹⁰ est choisi parmi H, un halogène et un alcoxy en C₁₋₆ ;
G₁ est choisi parmi un alkyle en C₁₋₆ et un cycloalkyle en C₃₋₇ ;
G₂ est choisi parmi un alkyle en C₁₋₆ et un cycloalkyle en C₃₋₇ ;
m est choisi parmi 0 et 1 ;
ou un sel pharmaceutiquement acceptable de celui-ci.

8. Composé selon la revendication 7, dans lequel
R¹ représente Cl ;
R⁶ est choisi parmi H et un méthoxy ;
R⁷ est choisi parmi un carboxy et un cyclopropylsulfonylaminocarbonyle ;
R⁸ est choisi parmi H, Cl, Br, un méthyle, CF₃ et un méthoxy ;
R¹⁰ est choisi parmi H, F, un méthoxy et un éthoxy ;
G₁ est choisi parmi un méthyle, un éthyle, un propyle et un cyclobutyle ;
G₂ est choisi parmi un méthyle et un cyclobutyle ;
m est choisi parmi 0 et 1 ;
ou un sel pharmaceutiquement acceptable de celui-ci.

9. Composé selon l'une quelconque des revendications 1 à 3, choisi parmi l'acide cis-3-[2-[2-bromo-5-(8-chloro-4-oxo-chromén-2-yl)-4-méthoxy-phénoxy]éthoxy]cyclobutanecarboxylique ;
l'acide 3-[2-bromo-5-(8-chloro-4-oxo-chromén-2-yl)-4-méthoxy-phénoxy]propanoïque ;
le 3-[2-bromo-5-(8-chloro-4-oxo-chromén-2-yl)-4-méthoxy-phénoxy]-N-cyclopropylsulfonyl-propanamide ;
l'acide 2-[2-bromo-5-(8-chloro-4-oxo-chromén-2-yl)-4-méthoxy-phénoxy]acétique ;
l'acide 3-[2-bromo-5-(8-chloro-4-oxo-chromén-2-yl)-4-méthoxy-phénoxy]cyclobutanecarboxylique ;
l'acide 2-[3-[2-bromo-5-(8-chloro-4-oxo-chromén-2-yl)-4-méthoxy-phénoxy]propoxy]acétique ;
l'acide 3-[2-chloro-5-(8-chloro-4-oxo-chromén-2-yl)-4-méthoxy-phénoxy]cyclobutanecarboxylique ;
l'acide cis-3-[2-[2-chloro-5-(8-chloro-4-oxo-chromén-2-yl)-4-méthoxy-phénoxy]éthoxy]cyclobutanecarboxylique ;
l'acide 3-[2-[5-(8-chloro-4-oxo-chromén-2-yl)-4-méthoxy-2-méthyl-phénoxy]éthoxy]cyclobutanecarboxylique ;
l'acide 3-[5-(8-chloro-4-oxo-chromén-2-yl)-4-méthoxy-2-méthyl-phénoxy]propanoïque ;
l'acide 2-[3-[5-(8-chloro-4-oxo-chromén-2-yl)-4-méthoxy-2-méthyl-phénoxy]propoxy]acétique ;
l'acide 3-[5-(8-chloro-4-oxo-chromén-2-yl)-2,4-diméthoxy-phénoxy]cyclobutanecarboxylique ;
l'acide 3-[2-[5-(8-chloro-4-oxo-chromén-2-yl)-2,4-diméthoxy-phénoxy]éthoxy]cyclobutanecarboxylique ;
l'acide 2-[5-(8-chloro-4-oxo-chromén-2-yl)-2-méthyl-phénoxy]acétique ;
l'acide cis-3-[5-(8-chloro-4-oxo-chromén-2-yl)-2-méthyl-phénoxy]cyclobutanecarboxylique ;
l'acide trans-3-[5-(8-chloro-4-oxo-chromén-2-yl)-2-méthyl-phénoxy]cyclobutanecarboxylique ;
l'acide 3-[5-(8-chloro-4-oxo-chromén-2-yl)-2-méthoxy-phénoxy]cyclobutanecarboxylique ;
l'acide 3-[2-[5-(8-chloro-4-oxo-chromén-2-yl)-2-méthoxy-phénoxy]éthoxy]cyclobutanecarboxylique ;
l'acide 3-[5-(8-chloro-4-oxo-chromén-2-yl)-2-méthoxy-phénoxy]propanoïque ;
l'acide cis-3-[5-(8-chloro-4-oxo-chromén-2-yl)-2-(trifluorométhyl)phénoxy]cyclobutanecarboxylique ;
l'acide trans-3-[5-(8-chloro-4-oxo-chromén-2-yl)-2-(trifluorométhyl)phénoxy]cyclobutanecarboxylique ;
l'acide 2-[5-(8-chloro-4-oxo-chromén-2-yl)-2-(trifluorométhyl)phénoxy]acétique ;
l'acide cis-3-[5-(8-chloro-4-oxo-chromén-2-yl)-2,3-diméthoxy-phénoxy]cyclobutanecarboxylique ;
l'acide trans-3-[2-[5-(8-chloro-4-oxo-chromén-2-yl)-2,3-diméthoxy-phénoxy]éthoxy]cyclobutanecarboxylique ;
l'acide 2-[5-(8-chloro-4-oxo-chromén-2-yl)-2,3-diméthoxy-phénoxy]acétique ;
l'acide trans-3-[4-bromo-5-(8-chloro-4-oxo-chromén-2-yl)-2-méthoxy-phénoxy]cyclobutanecarboxylique ;
l'acide trans-3-[2-[4-bromo-5-(8-chloro-4-oxo-chromén-2-yl)-2-méthoxy-phénoxy]éthoxy]cyclobutanecarboxylique ;
l'acide 3-[3-(8-chloro-4-oxo-chromén-2-yl)-2-méthoxy-phénoxy]cyclobutanecarboxylique ;
l'acide cis-3-[2-[3-(8-chloro-4-oxo-chromén-2-yl)-2-méthoxy-phénoxy]éthoxy]cyclobutanecarboxylique ;
l'acide 3-[3-(8-chloro-4-oxo-chromén-2-yl)-4-méthoxy-phénoxy]cyclobutanecarboxylique ;
l'acide cis-3-[2-[3-(8-chloro-4-oxo-chromén-2-yl)-4-méthoxy-phénoxy]éthoxy]cyclobutanecarboxylique ;
l'acide 2-[3-(8-chloro-4-oxo-chromén-2-yl)-4-méthoxy-phénoxy]acétique ;
l'acide 3-[3-(8-chloro-4-oxo-chromén-2-yl)-4-méthoxy-phénoxy]propanoïque ;
l'acide cis-3-[2-[6-bromo-3-(8-chloro-4-oxo-chromén-2-yl)-2-méthoxy-phénoxy]éthoxy]cyclobutanecarboxylique ;
l'acide 3-[6-bromo-3-(8-chloro-4-oxo-chromén-2-yl)-2-méthoxy-phénoxy]cyclobutanecarboxylique ;
l'acide 3-[3-(8-chloro-4-oxo-chromén-2-yl)-2-méthoxy-6-méthyl-phénoxy]cyclobutanecarboxylique ;
l'acide 2-[6-chloro-3-(8-chloro-4-oxo-chromén-2-yl)-2-méthoxy-phénoxy]acétique ;
l'acide 2-[3-(8-chloro-4-oxo-chromén-2-yl)-2,6-diméthoxy-phénoxy]acétique ;
l'acide 3-[2-bromo-5-(8-chloro-4-oxo-chromén-2-yl)-4-éthoxy-phénoxy]propanoïque ;
l'acide 3-[2-bromo-5-(8-chloro-4-oxo-chromén-2-yl)-4-(difluorométhoxy)phénoxy]propanoïque ;
l'acide 3-[2-bromo-5-(8-chloro-4-oxo-chromén-2-yl)-4-fluoro-phénoxy]propanoïque ;
l'acide 3-[2-bromo-5-(8-chloro-4-oxo-chromén-2-yl)-4-(trifluorométhoxy)phénoxy]propanoïque ;
l'acide 3-[2-bromo-4-chloro-5-(8-chloro-4-oxo-chromén-2-yl)phénoxy]propanoïque ;
l'acide 2-[3-[6-bromo-3-(8-chloro-4-oxo-chromén-2-yl)-2-méthoxy-phénoxy]propylamino]-2-oxo-acétique ;
l'acide 2-[2-[2-bromo-5-(8-chloro-4-oxo-chromén-2-yl)-4-méthoxy-phénoxy]éthylamino]-2-oxo-acétique ; et
l'acide 2-[3-[2-bromo-5-(8-chloro-4-oxo-chromén-2-yl)-4-méthoxy-phénoxy]propylamino]-2-oxo-acétique ;
ou un sel pharmaceutiquement acceptable de ceux-ci.

10. Composé selon l'une quelconque des revendications 1 à 8, choisi parmi l'acide 3-[2-bromo-5-(8-chloro-4-oxo-chromén-2-yl)-4-méthoxy-phénoxy]propanoïque ;
le 3-[2-bromo-5-(8-chloro-4-oxo-chromén-2-yl)-4-méthoxy-phénoxy]-N-cyclopropylsulfonyl-propanamide ;
l'acide 2-[2-bromo-5-(8-chloro-4-oxo-chromén-2-yl)-4-méthoxy-phénoxy]acétique ;
l'acide 2-[3-[2-bromo-5-(8-chloro-4-oxo-chromén-2-yl)-4-méthoxy-phénoxy]propoxy]acétique ;
l'acide cis-3-[2-[2-chloro-5-(8-chloro-4-oxo-chromén-2-yl)-4-méthoxy-phénoxy]éthoxy]cyclobutanecarboxylique ;
l'acide 3-[5-(8-chloro-4-oxo-chromén-2-yl)-2,4-diméthoxy-phénoxy]cyclobutanecarboxylique ;
l'acide 3-[2-[5-(8-chloro-4-oxo-chromén-2-yl)-2,4-diméthoxy-phénoxy]éthoxy]cyclobutanecarboxylique ;
l'acide cis-3-[5-(8-chloro-4-oxo-chromén-2-yl)-2-méthyl-phénoxy]cyclobutanecarboxylique ;
l'acide 2-[5-(8-chloro-4-oxo-chromén-2-yl)-2-(trifluorométhyl)phénoxy]acétique ;
l'acide 3-[3-(8-chloro-4-oxo-chromén-2-yl)-2-méthoxy-phénoxy]cyclobutanecarboxylique ;
l'acide cis-3-[2-[6-bromo-3-(8-chloro-4-oxo-chromén-2-yl)-2-méthoxy-phénoxy]éthoxy]cyclobutanecarboxylique ;
l'acide 3-[6-bromo-3-(8-chloro-4-oxo-chromén-2-yl)-2-méthoxy-phénoxy]cyclobutanecarboxylique ;
l'acide 2-[3-(8-chloro-4-oxo-chromén-2-yl)-2,6-diméthoxy-phénoxy]acétique ;
l'acide 3-[2-bromo-5-(8-chloro-4-oxo-chromén-2-yl)-4-éthoxy-phénoxy]propanoïque ; et
l'acide 3-[2-bromo-5-(8-chloro-4-oxo-chromén-2-yl)-4-fluoro-phénoxy]propanoïque ;
ou un sel pharmaceutiquement acceptable de ceux-ci.

11. Procédé de préparation d'un composé selon l'une quelconque des revendications 1 à 10 comprenant au moins l'une des étapes suivantes :
(a) hydrolyse d'un composé de formule (III-6), 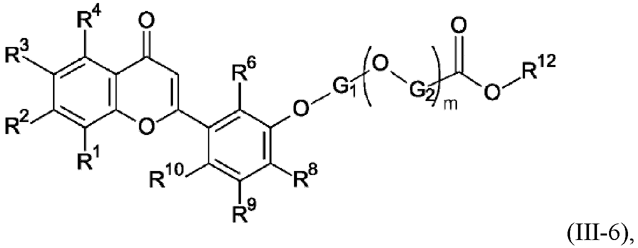 en présence d'une base ou d'un acide ;
(b) hydrolyse d'un composé de formule (IV-1), 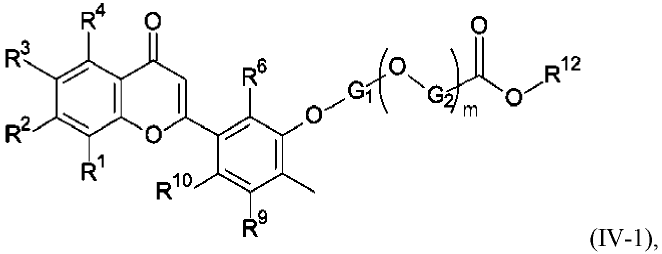 en présence d'une base ou d'un acide ;
(c) réaction d'un composé de formule (III-4), 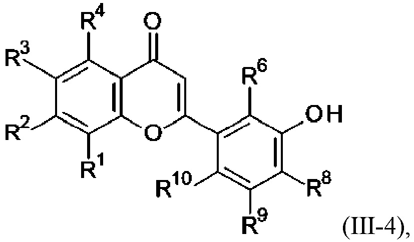 avec de l'oxétan-2-one en présence d'une base ;
(d) oxydation d'un composé de formule (V-1), 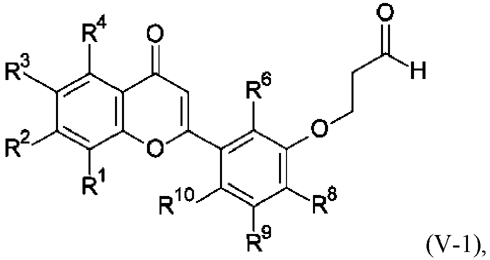 avec du NaClO₂ ;
(e) réaction d'un composé de formule (III-7), 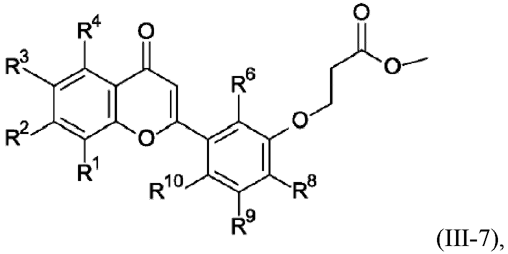 avec du sulfonamide en présence d'un acide de Lewis ;
(f) hydrolyse d'un composé de formule (VI-3), 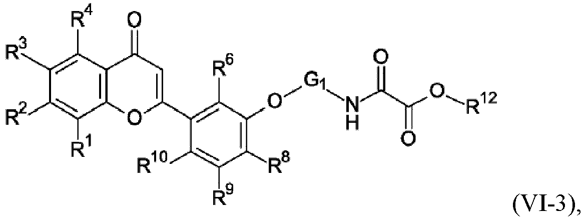 en présence d'une base ; dans lequel R¹ à R⁴, R⁶, R⁸ à R¹⁰, G₁, G₂ et m sont tels que définis dans l'une quelconque des revendications 1 à 3 ; R¹² représente un alkyle en C₁₋₆.

12. Composé selon l'une quelconque des revendications 1 à 10 pour une utilisation comme substance thérapeutiquement active.

13. Composition pharmaceutique comprenant un composé selon l'une quelconque des revendications 1 à 10 et un véhicule thérapeutiquement inerte.

14. Composé selon l'une quelconque des revendications 1 à 10 pour une utilisation dans le traitement ou la prophylaxie d'une infection par le VHB.

15. Composé selon l'une quelconque des revendications 1 à 10, lorsqu'il est fabriqué selon un procédé selon la revendication 11.
